# EUROPEAN PATENT APPLICATION

(11) **EP 1 462 119 A1**
(43) Date of publication of application: **29.09.2004**
(21) Application number: 03006592.4
(22) Date of filing: 24.03.2003
(51) Int. Cl.: A61K 51/10, A61K 49/00, G01N 33/566

(54) **Modulators of the megalin-mediated uptake of radiotherapeutics and/or radiodiagnostics into kidney cells and their use in therapy and diagnostics**

(71) Applicant: Schering AG, 13342 Berlin (DE)
(72) Inventor: Bräutigam, Matthias, 10629 Berlin (DE); Zerhusen, Sandra, 10553 Berlin (DE)
(74) Representative: Krauss, Jan

(57) **Abstract**

The present invention broadly relates to the treatment, diagnosis, and prophylactic prevention of cancer disease. More specifically, the present invention relates to methods and compositions for preventing the endocytosis of radiopharmaceutics into cells of the kidney and the subsequent radioinduced damaging of the kidney catabolism by blocking or interfering with the association or binding of radiotherapeutics and/or radiodiagnostics to the receptor megalin, a member of the LDL-receptor family. In another aspect of the present invention, the expression of megalin is altered, in order to prevent the endocytosis and cellular internalisation of radiopharmaceutics into cells of the kidney.

## Description

### FIELD OF THE INVENTION

The present invention broadly relates to the treatment, diagnosis, and prophylactic prevention of cancer disease. More specifically, the present invention relates to methods and compositions for preventing the endocytosis of radiopharmaceutics into cells of the kidney and the subsequent radioinduced damaging of the kidney catabolism by blocking or interfering with the association or binding of radiotherapeutics and/or radiodiagnostics to the receptor megalin, a member of the LDL-receptor family. In another aspect of the present invention, the expression of megalin is altered, in order to prevent the endocytosis and cellular intemalisation of radio pharmaceutics into cells of the kidney.

### BACKGROUND OF THE INVENTION

Radiopharmaceuticals (in the following also designated "radiotherapeutic" or "radiodiagnostic") are frequently used in order to provide therapy against several, for example, cancerous diseases. Several radiotherapeutics are well known that efficiently bind to cancerous cells and promote their radiation-induced cell death, which kills transformed cells. In the same context, radiodiagnostics (in the following also designated "radiodiagnosticals") are frequently used in the diagnosis of, for example, tumours and cancer. These compounds mainly differ from radiotherapeutics in the use of a different radioactive metal, which, in most cases, has a very short half-life. In addition, radiodiagnostics and/or radiotherapeutics are also used in other diseases, for example Parkinson disease, Alzheimer syndrome, coronary diseases, and arteriosclerosis including restenosis.

Commonly used radiotherapeutics and radiodiagnostics are, amongst others, gallium-67 (67 Ga) citrate injection; iodine-131-MIBG, Y-90 ibritumomab tiuxetan (Zevalin®), F-18-FDG, P-32 sodium phosphate, Co-58 vitamin B12, I-123-sodium iodide (123I), Au-198 colloid, indium-pentreotide (111-In) (Octreoscan®); iodine-125 human serum albumin (125I) injection; sodium iodide (131I) solution; thallium-201 thallous (201TI) chloride injection; indium-111 (Satumomab®); In-111 chloride solution, In-111 oxine solution, 1-131-hippurate (Hipputope ®), P-32 chromic phosphate (Phosphocol ®), technetium Tc-99m sestamibi (Cardiolite®; indium-111 Capromab®; iodine-131 Iobguane®; technetium Tc-99m Depreotide®; technetium Tc-99m Arcitumomab (CEA-Scan®); 1-123-ioflupane (DaTSCAN ®); Tc-99m-apcitide (AcuTect ®); and I-125-iothalamate-sodium-injection).

The use and efficiency of both radiopharmaceuticals and radiodiagnostics is limited by several factors. The major problem currently encountered is the elevated renal uptake and accumulation of radiolabeled molecules within the kidney cells, e.g. in the case of using systemic application of radiolabeled antibody fragments and/or peptides which are filtered by the glomerulus (Behr, 1998). Subsequently due to the maximum tolerated dose of 25 Gy (observed by external beam radiation) the kidney is furthermore the dose limiting organ in most radio-therapies and radioimmunotherapies. As shown by autoradiography, the uptake occurs in the renal cortex and the accumulation of e.g. In-111-DOTA-Fv or In-111-DTPA-glycoproteins takes place in the lysosomes of the proximal tubular cells (Tsai, 2001; Franano, 1994). Little is known about the molecular mechanisms and details of endocytosis of radiolabeled protein-based compounds.

Similar problems like those mentioned above exist in the field of radiodiagnosis in which the uptake of the radiodiagnostics into cells of the kidney leads to a decreased availability of the radiodiagnostics in the tissue to be diagnosed (i.e. low target versus non-target ratio). Radio-diagnostics in principal can behave similar to radiotherapeutics, except for the use of a different radiometal.

The two main functions of the kidneys are the regulation of the composition of the body fluids and the clearance of molecules from the blood. These functions are fulfilled by a number of cellular processes including vector-mediated transport across renal epithelial cells. Specific transport processes are localised along the nephron and the collecting duct system. As vector-mediated transport depends on the integrity of polarised epithelial cells apical and basolateral plasma membranes of these cells differ in the composition of the proteins and lipids (Apocada, 2001 ).

In the last decade several membrane-associated proteins were identified to participate in vector-mediated transport processes essential for the kidney function. These include aquaporins, sodium hydrogen exchangers, urea transporters, amino acid transporters, glucose transporters, anion exchangers, chloride channels etc. In addition other membrane proteins such as receptors, enzymes, adhesion molecules and junctional proteins have been identified and localised in renal epithelial cells. Macromolecules filtered in the glomerulus are reabsorbed by the proximal tubular cells with subsequent transport into lysosomes.

There exist several endocytotic pathways (Mukherjee, 1997) in renal epithelial cells including
- clathrin-coated pit mediated endocytosis
- caveolae-mediated endocytosis (small flask-shaped vesicles rich in cholesterol and signalling molecules)
- clathrin-independent pathways that involve neither clathrin nor caveolaes
- pinocytosis (internalising of small amounts of fluid) and
- phagocytosis (large particles are internalised).

Proteins of the glomerular filtrate are mostly removed from the lumen by absorptive endocytosis (Nielsen, 1994). Only minor amounts are extracted by fluid-phase endocytosis (pinocytosis). Absorptive endocytosis requires a binding of the protein on the apical plasma membrane site. The selectivity and mechanisms for the absorptive endocytosis are still under discussion. Receptor mediated clathrin-coated pit endocytosis occurs for a number of plasma proteins e.g. for albumin (Gekle, 1997).

Different pathways for the uptake of peptides from the lumen have been identified (carrier-mediated pathways mainly related to active H⁺ co-transport) and completed with the finding of basolateral transporters such as Pept1 and Pept2 which mediate the renal uptake of di- and tripeptides (Adibi, 1997). Peptidases located mainly at the proximal tubular brush border are able to cleave a variety of oligopeptides to amino acids or di- and tripeptides. These metabolites are taken up via specific transport mechanisms mainly carrier-mediated Na⁺ -amino acid co-transports. It has also been demonstrated that peptide-like drugs (e.g. β-lactam antibiotics) were efficiently cleared by the kidney mediated by organic anion (OAT1-3) and organic cation (OCT1-3) transporter systems located as well at the luminal as at the basolateral side of the renal epithelium (Inui, 2000).

As one of these transporter systems, megalin, a member of the LDL-receptor family and formerly known as gp 330 or Heymann nephritis antigen, a multiligand receptor often co-localised with cubilin, was identified. Megalin (600 kDa) and cubilin (460 kDa) are highly expressed at the apical side of renal proximal tubular cells where they act as synergistic endocytotic receptors. Both receptors are involved in the normal tubular reabsorption of filtered proteins and bind each other with high affinity. In contrast to cubilin as a peripheral membrane glycoprotein megalin consists of a transmembrane domain and a cytoplasmic tail which is responsible for the clustering in coated pits. Megalin is therefore able to mediate the internalisation of bound (either to cubilin or to megalin itself) ligands into the cells. A variety of ligands that have been identified that bind to megalin (Christensen, 2002) are listed in the following:
a) Vitamin-binding proteins, like transcobalamin-vitamin B12; vitamin-D binding protein; retinol-binding protein
b) Other carrier proteins; like albumin; lactoferrin; aemoglobin; odorant-binding protein; transthyretin
c) Lipoproteins, like apolipoprotein B; apolipoprotein E; apolipoprotein J/ clusterin; apolipoprotein H
d) Hormones and hormone precursors, like parathyroid hormone; insulin; epidermal growth factor; prolactin; thyroglobulin
e) Drugs and toxins, like aminoglycosides; polymyxin B; aprotinin; trichosanthin
f) Enzymes and enzyme inhibitors, like PAI-1, -urokinase, -tPA; pro-urokinase; lipoprotein lipase; plasminogen; β-amylase; β-microglobulin; lysozyme
g) Immune and stress-response related proteins, like immunoglobulin light chains; PAP-1; β 2-microglobulin
h) Others, like receptor associated protein (RAP); Ca²⁺, Ca²⁺-scavengers, lipids, cytochrome C.

WO 99/37757 (EP 1 047 773), entitled "cubilin protein, DNA sequences encoding cubilin and uses thereof" describes cubilin and megalin as representative examples of renal receptors for ligands. Also disclosed are potential uses of these renal receptors for treating toxicity in various tissues and for detecting renal damage.

US 6,428,967 describes methods and compositions for inducing and detecting signal transduction through LDL receptors, including studies of the interaction of the binding polypeptide megalin and the interaction domain, wherein the binding polypeptide is PSD-95.

Hammad et al (2000) describe cubilin as an endocytic receptor for high density lipoproteins (HDL) and explore the possibility that megalin acts in conjunction with cubilin to mediate HDL endocytosis. Cubilin and megalin exhibited coincident patterns of mRNA expression in mouse tissues including the kidney, ileum, thymus, placenta, and yolk sac endoderm. The expression of both receptors in yolk sac endoderm-like cells was inducible by retinoic acid treatment but not by conditions of sterol depletion. Suppression of megalin activity or expression by treatment with either megalin antibodies or megalin antisense oligodeoxynucleotides resulted in inhibition of cubilin-mediated endocytosis of HDL. Furthermore, megalin antisense oligodeoxynucleotide treatment resulted in reduced cell surface expression of cubilin. These data demonstrate that megalin acts together with cubilin to mediate HDL endocytosis and further suggest that megalin may play a role in the intracellular trafficking of cubilin. This publication does not mention that radiotherapeutics and/or radiotherapeutics are taken up by megalin and the use of megalin for the identification of modulators of such uptake by kidney cells.

Several approaches have been pursued in order to limit the unwanted uptake of radiotherapeutics into the kidney. Efforts to reduce the kidney uptake resulted in the use of amino acid infusions and various modifications of the radiodiagnostics and/or radiotherapeutics including cleavable linkers, decreased isoelectrical points, glycosylations, and variations of the charges. Most groups have focused on the use of cationic amino acids such as lysine and arginine not only in animal studies but also in clinical trials (Kobayashi, 1996; DePalatis, 1995; Behr, 1996).

Although the positively charged amino acids reduced the renal uptake especially of radiolabeled peptides in a dose dependent manner (Bernard, 1996), they exhibit the following limitations:
1. Toxic side effects after infusion such as nausea, vomiting etc. are usually observed (Barone, 2000).
2. A reduction of the uptake of the radiopharmaceuticals of more than 50-60% could not be demonstrated.
3. The effect on the tubular cells is non-specific and not long-lasting.
4. A long term infusion or several injections at definite time points are needed.

In order to accelerate the clearance of radioactivity from normal tissues, biological degradable linkers between, for example, the antibody or antibody fragment and the radioactive moiety have been designed for cleavage in the liver or the kidneys. Investigations included for example ester-bonds (Weber, 1990), maleimido-cysteineamido linker (Lewis, 1998), and peptide linkages specific for lysosomal or brush-border enzymes (Novak-Hofer, 2001) but the following major problems of these linkers have not been fully overcome, yet:
1. conjugation of the linker without impairing the functionality of the chelate or the antibody fragment/ peptide (responsible for tumour targeting)
2. in-vivo stability especially in blood and serum
3. degradation under specified conditions

It has been demonstrated that iodohipporyl-maleoyl-lysine (HML) linkers were useful for a rapid kidney clearance of iodinated antibody fragments (Arano, 1999) but compounds radiolabeled with I-125 or I-131 are usually not problematical. The difficulties of renal accumulation exist mainly for radiometal - (e.g. In-111, Y-90, Re-188) labelled antibody fragments where the linker strategy was not successful, yet.

In view of the above, a need in the art exists for selective compounds that can act as modulators of the megalin receptor-mediated uptake of radiodiagnostics and/or radiotherapeutics. These modulators would lead to therapies that would still have some or all of the benefits of a radiopharmaceutical treatment therapy, and would even exhibit better effects because of the fact that the dose as administered can be elevated if a dose-limiting organ, such as the kidney, does not interfere with the treatment, and thus would have fewer undesirable side-effects in view of their protective function for the kidney cells. Furthermore, such modulators would have advantages in the diagnosis employing radiodiagnostics, e.g. by increasing the target versus non-target ratio.

The present invention is based on the surprising finding that radiopharmaceuticals and/or radiodiagnostics are taken up to a great extent by the megalin receptor into cells of the kidney. Therefore, megalin plays an important role in the unwanted side effects of commonly used radiopharmaceutics and the drawbacks encountered during radiodiagnosis and/or radiotherapy.

### SUMMARY OF THE INVENTION

Accordingly, it is an object of the present invention to provide agents which interact with megalin and reduce the interaction and subsequent uptake of radiopharmaceuticals into kidney cells. Other objects, features and advantages of the present invention will be set forth in the detailed description of preferred embodiments that follows, and in part will be apparent from the description or may be learned by practice of the invention. These objects and advantages of the invention will be realised and attained by the methods and compositions particularly pointed out in the written description and claims hereof.

A first embodiment of the present invention therefore relates to a method of modulating the megalin-receptor mediated binding and/or uptake of a radiotherapeutic and/or radiodiagnostic into cells of the kidney in a patient, said method comprising a) administering to said patient an effective amount of a radiotherapeutic and/or radiodiagnostic or a pharmaceutically acceptable salt thereof, and b) administering to said patient an effective amount of a modulator of the megalin-receptor mediated uptake of said radiotherapeutic and/or radiodiagnostic into cells of the kidney, or a pharmaceutical salt thereof; a prodrug thereof; or a metabolite thereof.

The term "modulator" as used in the context of the present invention relates to a compound or group of compounds that interact with the megalin-receptor in a way which, in turn, influences the uptake of radiopharmaceutics and/or radiodiagnostics by said receptor. The modulator can inhibit the uptake completely or partially. The modulator can be competitive with the radiopharmaceutical and/or radiodiagnostic in binding to and uptake by megalin. The compound can either bind directly at the site, at which the radiotherapeutic and/or radiodiagnostic interacts with the receptor and therefore directly prevent the radiotherapeutic and/or radiodiagnostic from binding. In addition, the compound can bind at the megalin receptor at a position that will indirectly influence the uptake of the radiotherapeutic and/or radiodiagnostic, for example, by sterically modifying the structure of the megalin-receptor in such a manner that no or reduced binding and subsequent uptake will occur. The term modulator also includes molecules that influence the expression of the gene encoding for megalin. The compound can modulate the expression either by acting directly on the part of the DNA of the cell that encodes the megalin-receptor (such as a repressor) or by acting indirectly on the protein expression (e.g. by acting on enhancers or by acting on the transcriptional apparatus of the cell). Alternatively, the expression of the receptor can be influenced at the level of regulating the megalin-receptor expression. For example, compounds can be employed that influence the rate of the synthesis of the megalin-receptor in the cell by acting on the control mechanisms of the megalin expression.

Furthermore, "cofactors" can be present, such as certain ions or cubilin, that promote the attachment of the compound to the receptor and therefore support the function of the modulator. Some potential cofactors are mentioned in the present specification. "Binding" can occur via a covalent or non-covalent attachment of the compound or group of compounds to megalin and/or the cofactor.

In one embodiment of the present invention, the above method is employed during the course of a treatment or diagnosis of cancer, and comprises administering to a patient in need thereof a therapeutically effective amount of a composition, comprising, an effective amount of a radiotherapeutic and/or radiodiagnostic or a pharmaceutically acceptable salt thereof, and an effective amount of a modulator of the megalin-receptor (gp330) mediated uptake of said radiotherapeutic and/or radiodiagnostic into cells of the kidney, or a pharmaceutical salt thereof; a prodrug thereof; a metabolite thereof; or mixtures thereof.

In one embodiment of the present invention, the cancer that will be treated or diagnosed can be selected from the group comprising prostate cancer, breast cancer, leukaemia, pancreatic cancer, lung cancer, colon cancer, sarcoma, glioblastoma, melanoma, ovarian cancer, head and neck carcinomas, and lymphoma. In addition, radiodiagnostics and/or radiotherapeutics are also used in other diseases, for example Parkinson disease, Alzheimer syndrome, coronary diseases, and diseases involving restenosis, e.g. arteriosclerosis.

An additional aspect of the present invention relates to a method for the prevention and/or treatment of damages of the kidney of a patient during a radiotherapy and/or radiodiagnosis, comprising a) administering to said patient an effective amount of a radiotherapeutic and/or radiodiagnostic or a pharmaceutically acceptable salt thereof, and b) administering to said patient an effective amount of a modulator of the megalin-receptor mediated uptake of said radiotherapeutic and/or radiodiagnostic into cells of the kidney, or a pharmaceutical salt thereof; a prodrug thereof; a metabolite thereof; or mixtures thereof.

An additional embodiment of the present invention relates to a method according to the invention, wherein said modulator is administered before, simultaneously together with and/or after said radiotherapeutic and/or radiodiagnostic. It might sometimes be desirable to apply the modulator in advance, in order to allow for an initial binding of the modulator to megalin before a subsequent application of the radiotherapeutic and/or radiodiagnostic. Nevertheless, the modulator can also be applied after the radiotherapeutic and/or radiodiagnostic, as long as the modulator interacts and/or interferes with the megalin-mediated uptake. The preferred routes of administration are by infusion or as a bolus or oral dose. The injection can be intravenous or intraperitoneally. Several combinations are possible, for example, modulator intraperitoneally 30 minutes before the intravenous application of the radiotherapeutic and/or radiodiagnostic, or both in admixture together.

The methods of the present invention can be used during the prevention, diagnosis, monitoring and/or treatment of cancers. As mentioned above, in principle the method of the invention can be used in all types of cancers that can be treated with a radiotherapeutic. Said cancer can be selected from the group comprising prostate cancer, breast cancer, leukaemia, pancreatic cancer, lung cancer, colon cancer, sarcoma, glioblastoma, melanoma, ovarian cancer, head and neck carcinomas, and lymphoma. In addition, radiodiagnostics and/or radiotherapeutics are also used in other diseases, for example Parkinson disease, Alzheimer syndrome, coronary diseases, and diseases involving restenosis, e.g. arteriosclerosis.

An additional embodiment of the present invention relates to a method according to the invention, wherein said radiotherapeutic is selected from the group of compounds comprising radiolabeled antibodies, radiolabeled enzymes, radiolabeled peptides, radiolabeled nucleic acids, comprising radiometals selected from the group of Fluorine-18, Chromium-51, Cobalt-58, Gallium-67, Copper-67, Molybdenum-99, Technetium-99, Indium-111, Iodine-123, Iodine-125, Iodine-131, Yttrium-90, Gold Au-198 colloid, Xenon-133, Thallium-201, Rhenium-188, Strontium-189, Actinium-225, Astatine-211, Bismuth-212/213, Rhenium-186, Holmium-166, Samarium-153, and Lutetium-177, and other suitable α-emitters. In addition, a fairly comprehensive listing of suitable radiometals can be found in Illidge et al. (2000), which is herewith incorporated. Several radiotherapeutics are commercially available, e.g. under the names Sautomomab®, Sestamibi®, Capromab®, Iobguane®; Depreotide®; Arctumomab®, Rituximab®, and Zevalin®.

An additional embodiment of the present invention relates to a method according to the invention, wherein said modulator is selected from the group of compounds comprising modified cubilin, vitamin-binding proteins, carrier proteins, lipoproteins, apolipoproteins (e.g. apo E, and apo B, clusterin), LPO-antagonists (e.g., probucol), RAP and derivatives thereof, Ca²⁺, Ca²⁺-scavengers, cytochrome C, antimegalin IgG, anti-cubilin IgG, anti-poly alpha2,8 KDN antibody, aminoglycosides (e.g. gentamicin), hormones and precursors thereof, drugs and toxins, enzymes and inhibitors thereof, and immune- and stress-response related proteins, maleate, dextran-based conjugates, aristolochic acid, cadmium chloride, and L-carnithine.

In another aspect, the present invention relates to a method of screening for modulator compounds of the megalin-receptor mediated binding and/or uptake of radiotherapeutics into cells of the kidney, comprising: a) providing an assay for measuring the megalin-receptor mediated binding and/or uptake of radiotherapeutics, b) adding at least one radiotherapeutic and/or a suitably labelled precursor thereof to the assay, c) adding the candidate modulator compound to be tested to the assay; and d) determining if the amount of radiotherapeutic bound to or taken up by the megalin-receptor-mediated binding and/or uptake is altered as compared to binding or uptake in the absence of the compound to be tested, wherein a difference in binding or uptake mediated by the megalin-receptor identifies a compound which modulates the megalin-receptor mediated binding and/or uptake of the radiotherapeutic into cells of the kidney. According to the present invention, this assay can both be performed in vitro and/or in vivo.

In yet another aspect, the present invention relates to screening methods which can be used to identify agents which modulate the megalin-receptor mediated binding and/or uptake of radiotherapeutics into cells of the kidney. Such agents are potentially useful in the treatment of cancer diseases. Preferred agents are inhibitors of the megalin-receptor mediated binding and/or uptake of radiotherapeutics into cells of the kidney. Treatment of cancers with radiotherapeutics together with the modulators will generally be more efficient and associated with less undesirable side-effects than treatment with a radiotherapeutic alone.

The methods of the invention permit the identification of agents which directly modulate the megalin-receptor mediated binding and/or uptake of radiotherapeutics into cells of the kidney by determining if the amount of radiotherapeutics is bound to or taken up by a kidney cell or kidney cell line.

The methods of the invention are screening assays in which candidate agents are examined to identify suitable modulators of the megalin-receptor mediated binding and/or uptake of radiodiagnostics and/or radiotherapeutics into cells of the kidney. One type of screening assay involves determining if the amount of radiodiagnostics and/or radiotherapeutics bound to or taken up by the megalin-receptor-mediated binding and/or uptake is altered as compared to binding or uptake in the absence of the compound to be tested, wherein a difference in binding or uptake mediated by the megalin-receptor identifies a compound which modulates the megalin-receptor mediated binding and/or uptake of radiodiagnostics and/or radiotherapeutics into cells of the kidney. During the course of the assay of the method according to the invention, the compound will initially bind or attach to the receptor. The compound can either bind directly to megalin at the same site, at which the radiotherapeutic or radiodiagnostic interacts with the receptor and therefore directly prevent the radiotherapeutic from binding. In addition, the compound can bind at the megalin receptor at a position that will indirectly influence the uptake of the radiodiagnostic and/or radiotherapeutic, for example, by sterically modifying the structure of the megalin-receptor in such a manner, that no binding and subsequent uptake will occur. Furthermore, cofactors can be present, such as certain ions or cubilin, that promote the attachment of the compound to the receptor and therefore support the function of the modulator. "Binding" can occur via a covalent or non-covalent attachment of the compound or group of compounds to megalin. Based on the binding properties of the screened compounds, a first pre-selection of compounds can be performed, in which a non-binding compound is screened in a second "round" of screening using a set of co-factors. If still no binding occurs, the compound will be classified as "non-binding" and disregarded in further screenings. Such pre-selection will be encompassed by the terms "screening" and/or "determining" in the general context of this invention. A compound that shows an in-vitro modulating (e.g. inhibiting) action should in vivo preferably not further interact with components of the patients' or test (model) organisms' body, e.g. within the bloodstream, lung and/or heart of the patient or test organism.

In general, assays to determine a binding and biological effect of a compound to a specific target (in this case megalin) are well known to the person skilled in the art and can be found, for example, in US patents 4,980,281, 5,266,464 and 5,688,655 to Housey (which are herein incorporated by reference) for phenotypic changes of cells after incubation with a screening compound. Furthermore, US 5,925,333 to Krieger at al. (which is herein incorporated by reference) describes methods for modulation of lipid uptake and related screening methods.

US 6,475,733 to Lemer (which is herein incorporated by reference) describes a method exploits the evolutionary principles responsible for the development of the broad spectrum general odorant detector system, to create a cell-surface receptor based system capable of detecting and discriminating between thousands of chemicals that could be modified in order to be used for the present screenings.

In a preferred embodiment of the present invention, these compounds are initially screened using an assay such as one of the assays described herein and then tested in, for example, transgenic, megalin expressing and/or over-expressing, animals made using standard transgenic animal technology. A technique such as embryonic stem cell technology using rats, mice or hamsters is preferred.

The screening assays of the present invention preferably involve the use of kidney cells and non-kidney cells, as long as these cells express the megalin-receptor. Furthermore, recombinant cells can be used that express the megalin-receptor, optionally in addition with the protein cubilin. How to produce such recombinant cells is well known to the skilled artisan and is further described below.

The methods of the invention can be used to identify agents which exert their effect directly on the megalin-receptor, cubilin, or through an as yet unidentified megalin-receptor cofactor. Moreover, the methods of the invention can be used to identify agents which exert their effect through a receptor other than the megalin receptor.

In selecting a modulator, among the undesirable side-effects to be avoided or minimised, which have been found during clinical trials, are: toxic side effects after infusion such as nausea, vomiting etc.; a reduction of the uptake of less than 50-60%, no long-lasting and non-specific effect on the kidney tubular cells, and the need for several injections at definite time points. Of course, it may not be possible to completely avoid all of these undesirable side-effects in selecting a modulator. A modulator can be therapeutically beneficial even if its use is associated to some extent with some or all of these side effects. If already known clinical side effects occur at certain dosages which are commonly used for modulating the megalin interaction, this compound will not be selected further.

The method of screening according to the present invention can be performed in several different formats. One embodiment is a method, wherein the assay is performed in vitro.

Some of the methods which can be used to identify modulators involve measuring cellular uptake and/or proliferation. Proliferation can be measured by measuring cell number, DNA content, or ³H-thymidine uptake.

In all assays entailing the measurement and comparison of the megalin-receptor mediated binding and/or uptake of radiotherapeutics into cells of the kidney the measured rate of uptake of a given cell type in the presence of an agent is always corrected for the rate of uptake of a control cell. The control cell is an otherwise identical cell grown in the absence of the agent being tested.

An additional embodiment of the present invention relates to a method, wherein the assay is performed in a mammalian cell culture.

An additional embodiment of the present invention relates to a method wherein said mammalian cell is transformed with a plasmid containing a DNA sequence encoding the megalin-receptor. In an additional embodiment of the present invention, said cell mammalian cell is further transformed with a plasmid containing a DNA sequence encoding cubilin.

In various preferred embodiments the kidney or non-kidney cells are stable transfected with a non-endogenous megalin-expressing construct; the kidney or non-kidney cells are co-transfected with a cubilin expression construct. Most preferred are mammalian cells in the form of human cells.

An additional embodiment of the present invention relates to a method wherein the assay is performed in vivo. Preferably, the assay is performed in a mouse or rat. In general, the in vivo assay will not be substantially different from the above-mentioned in vitro assay. In a general screening assay for modulator compounds of the megalin-receptor mediated binding and/or uptake of radiotherapeutics into cells of the kidney, the assay for measuring binding and/or uptake of the megalin-receptor mediated binding and/or uptake of radiotherapeutics will be provided in that the compound to be tested is/are administered to a mouse or a rat. Then, at least one radiodiagnostic and/or radiotherapeutic and/or a suitably labelled precursor thereof is added to the assay, i.e. given to the animal, and adding the candidate modulator compound to be tested to the assay will be performed in a similar fashion, and it will be determined, if the amount of radiodiagnostics and/or radiotherapeutics bound to or taken up into cells of the kidney by the megalin-receptor-mediated binding and/or uptake is altered as compared to binding or uptake in the absence of the compound to be tested, wherein a difference in binding or uptake mediated by the megalin-receptor identifies a compound which modulates the megalin-receptor mediated binding and/or uptake of radiotherapeutics into cells of the kidney of said mouse or rat. Of course, these assays can be performed in other non-human mammals as well.

As one advantage of the in vivo model, it can be determined, if the modulator (compound) will have an effect on the amount of radioactivity that is excreted via the urine. From a suitable modulator, it will expected to have such an effect.

An additional embodiment of the present invention relates to a method according to the invention, wherein an assay includes a cell expressing the megalin-receptor protein and the compound is a nucleic acid sequence which potentially alters expression of the megalin-receptor protein and/or expression of cubilin. This assay can be performed in vitro and/or in vivo.

An additional embodiment of the present invention relates to a method according to the invention, wherein said radiodiagnostic and/or radiotherapeutic is selected from the group of compounds comprising radiolabeled antibodies, radiolabeled peptides, radiolabeled enzymes, radiolabeled nucleic acids, comprising radiometals selected from the group of chromium-51; gallium-67 (67 Ga) citrate injection; iodine-131-MIBG, Y-90 ibritumomab tiuxetan (Zevalin®), F-18-FDG, P-32 sodium phosphate, Co-58 vitamin B12, I-123-sodium iodide (123I), Au-198 colloid, indium-pentreotide (111-In) (Octreoscan®); iodine-125 human serum albumin (125I) injection; sodium iodide (131I) solution; thallium-201 thallous (201T1) chloride injection; indium-111 (Satumomab®); In-111 chloride solution, In-111 oxine solution, I-131-hippurate (Hipputope ®), P-32 chromic phosphate (Phosphocol ®), technetium Tc-99m sestamibi (Cardiolite®; indium-111 Capromab®; iodine-131 Iobguane®; technetium Tc-99m Depreotide®; technetium Tc-99m Arcitumomab (CEA-Scan®); I-123-ioflupane (DaTSCAN ®); Tc-99m-apcitide (AcuTect ®); and I-125-iothalamate-sodium-injection).

An additional embodiment of the present invention relates to a method according to the invention, wherein said modulator is selected from a library of naturally occurring or synthetic compounds which are randomly tested for alteration of megalin binding and/or uptake. Such libraries and the methods how to build up such a library, as well as methods for using these libraries for the screening of candidate compounds are well known to the person skilled in the art and further described below. Furthermore, some of these libraries are commercially available.

An additional embodiment of the present invention relates to a method according to the invention, wherein said modulator is selected from the group of compounds comprising modified cubilin, vitamin-binding proteins, carrier proteins, lipoproteins, apolipoproteins (e.g. apo E, and apo B, clusterin), LPO-antagonists (e.g., probucol), RAP and derivatives thereof, Ca²⁺, Ca²⁺-scavengers, lipids, cytochrome C, antimegalin IgG, anti-cubilin IgG, anti-poly alpha2,8 KDN antibody, aminoglycosides (e.g. gentamicin), hormones and precursors thereof, drugs and toxins, enzymes and inhibitors thereof, and immune- and stress-response related proteins, maleate, dextran-based conjugates, aristolochic acid, cadmium chloride, and L-camithine. These compounds can also be used as potential cofactors for yet unindentified modulators.

During the course of the assay of the method according to the invention, the candidate compound will initially bind or attach to the receptor. The compound can either bind directly to megalin at the same site, at which the radiodiagnostic and/or radiotherapeutic interacts with the receptor and therefore directly prevent the radiodiagnostic and/or radiotherapeutic from binding. In addition, the compound can bind at the megalin receptor at a position that will indirectly influence the uptake of the radiodiagnostic and/or radiotherapeutic, for example, by sterically modifying the structure of the megalin-receptor in such a manner, that no binding and subsequent uptake will occur. Furthermore, cofactors can be present, such as certain ions or cubilin, that promote the attachment of the compound to the receptor and therefore support the function of the modulator. "Binding" can occur via a covalent or non-covalent attachment of the compound or group of compounds to megalin, if several compounds are screened. Based on the binding properties of the screened compounds, a first pre-selection of compounds can be performed, in which an initially "non-binding" candidate compound is screened in a second "round" of screening using a set of co-factors. If still no binding occurs, the compound will be finally classified as "non-binding" and not used in further screenings. Such pre-selection will be encompassed in the terms "screening" and/or "determining" in the general context of this invention. Assays to determine a binding of a compound to a specific target (in this case megalin) are well known to the person skilled in the art and can be found, for example, in US patents 4,980,281, 5,266,464 and 5,688,655 to Housey for phenotypic changes of cells after incubation with a screening compound. Furthermore, US 5,925,333 to Krieger at al. (which is herein incorporated by reference) describes methods for modulation of lipid uptake and related screening methods.

Several assay formats can be employed in the methods according to the present invention. These include, for example, preferred methods like a two-hybrid assay, a biochemical pull-down assay, a high-throughput screening, in vitro fluorescent polarisation assay, an in vitro fluorescent binding assay, a conformational sensor-solid-phase chemiluminescence assay, a surface plasmon resonance assay, a filter binding assay, a sepharose chromatography, and a scintillation proximity assay. Examples of these assays are known to the person skilled in the art and are, without being limited thereto, described further below.

An additional embodiment of the present invention relates to a method, wherein the assay is a cell expressing both the megalin receptor and a cofactor. In an alternative embodiment, the assay is a an in vitro, cell-free mixture comprising a determined amount of the megalin receptor and, optionally, the cofactor.

An additional embodiment of the present invention relates to the modulating compound of the megalin-receptor mediated binding and/or uptake of radiotherapeutics into cells of the kidney that is identified using a method according to the invention.

The modulator according to the present invention should preferably fulfil certain requirements in order to be functional and effective. First, the modulator of the of the megalin-receptor mediated binding and/or uptake of radiotherapeutics into cells of the kidney is preferably an inhibitor. Second, the modulator of the of the megalin-receptor mediated binding and/or uptake of radiotherapeutics into cells of the kidney according to the invention has an affinity to megalin and/or cubilin of 50-500 nM, preferably of less than 50 nM. Similar affinities should be present in the case of a compound that modulates the expression of the megalin-receptor in the cells of the kidney. There, of course, the affinity must be present between the modulator and the respective cellular element that regulates the expression/display/turnover of the megalin receptor on/in said cells of the kidney. Third, the modulator of the megalin-receptor mediated binding and/or uptake of radiotherapeutics into cells of the kidney according to the invention must be able to pass through the filtration barrier of the glomerulus. Normally, proteins or other compounds which are small than approximately 60 kDa will be able to pass said filtration barrier. Molecules between 60-80 kDa are able to pass the barrier to a certain extent, depending mostly from the charge of the molecule.

A preferred modulator of the of the megalin-receptor mediated binding and/or uptake of radiotherapeutics into cells of the kidney according to the present invention will reduce the uptake of said radiotherapeutic into cells of the kidney by more than about 60%. Even more preferred is a modulator of the of the megalin-receptor mediated binding and/or uptake of radiotherapeutics into cells of the kidney according to the present invention that does not significantly (p < 0.05) reduce the uptake (%ID/g) of a radiotherapeutics and/or radiodiagnostics into tumour cells.

In one embodiment of the present invention, the modulator of the of the megalin-receptor mediated binding and/or uptake of radiotherapeutics into cells of the kidney is competitively inhibiting the binding of the radiotherapeutic and/or radiodiagnostic to the megalin-receptor protein.

In another embodiment of the present invention, the modulator of the of the megalin-receptor mediated binding and/or uptake of radiotherapeutics into cells of the kidney is sterically inhibiting the binding of the radiotherapeutic and/or radiodiagnostic to the megalin-receptor protein.

Preferred is a modulator of the of the megalin-receptor mediated binding and/or uptake of radiotherapeutics into cells of the kidney according to the present invention, that is selected from the group of megalin-binding compounds comprising modified cubilin, vitamin-binding proteins, carrier proteins, lipoproteins, apolipoproteins (e.g. apo E, and apo B, clusterin), LPO-antagonists (e.g., probucol), RAP and derivatives thereof, Ca²⁺, Ca²⁺-scavengers, lipids, cytochrome C, lipids, antimegalin IgG, anti-cubilin IgG, anti-poly alpha2,8 KDN antibody, aminoglycosides (e.g. gentamicin), hormones and precursors thereof, drugs and toxins, enzymes and inhibitors thereof, and immune- and stress-response related proteins, maleate, dextran-based conjugates, aristolochic acid, cadmium chloride, and L-carnithine. Furthermore the modulator can be a fragment or peptide of these compounds that is optionally further modified in order to function as a modulator. Suitable modifications include introducing changes into the amino acids sequence of these compounds or a kind of modification that will allow binding to the receptor without a subsequent initiation of the uptake into the cells of the kidney.

The present invention contemplates high throughput screening of agents for the modulation of the megalin-mediated uptake of radiotherapeutics and/or radiodiagnostics into cells of the kidney. The agents as described below, and modifications of said agents, including analogues, derivatives, fragments, active moieties, and the like, may be screened using methods and systems of the present invention.

Another aspect of the present invention relates to a method for producing a pharmaceutical composition for the treatment and/or diagnosis of cancer, comprising screening for a modulator compound of the megalin-receptor mediated binding and/or uptake of radiodiagnostics and/or radiotherapeutics into cells of the kidney as descried herein, and mixing of said modulator compound thus identified with a pharmaceutically suitable carrier and/or diluent. Consequently, the present invention is further directed to a pharmaceutical composition that is produced according to the above-mentioned method. An additional embodiment of the present invention relates to such a pharmaceutical composition that further comprises an additional safe and effective amount of a chemotherapeutic agent. Such chemotherapeutic agent is preferably selected from the group consisting of a DNA-interactive agent, alkylating agent, antimetabolite, tubulin-interactive agent, and a hormonal agent, and is, for example, selected from the group consisting of Methotrexate, Fluorouracil, Fluorodeoxyuridin, CB3717, Azacitidine, Floxuridine, Mercapyopurine, 6-Thioguanine, Pentostatin, Cytarabine, and Fludarabine. Additional chemotherapeutic agents are well-known to the skilled artisan in the field of cancer therapy and are described below.

As mentioned above, the modulators of the of the megalin-receptor mediated binding and/or uptake of radiotherapeutics into cells of the kidney according to the present invention shall be used for the prevention, diagnosis, monitoring and/or treatment of cancer. Another aspect of the present invention is therefore directed to such uses.

It is to be understood that both the foregoing general description and the following detailed description are exemplary and explanatory only and are intended to provide further explanation of the invention as claimed.

### DEFINITIONS

The scientific literature that has evolved around megalin and related receptors has adopted a number of terms to refer to modulators having various effects on receptors. For clarity and consistency, the following definitions will be used throughout this patent document. To the extent that these definitions conflict with other definitions for these terms, the following definitions shall control.

AGONISTS shall mean moieties that activate the intracellular response, i.e. the intemalisation of radiotherapeutics and/or radiodiagnostics, when they bind to the megalin receptor or the part of the DNA that is responsible for the expression of the megalin receptor or to cubilin or the part of the DNA that is responsible for the expression of cubilin.

PARTIAL AGONISTS shall mean moieties which activate the intracellular response when they bind to the megalin receptor or the part of the DNA that is responsible for the expression of the megalin receptor or to cubilin or the part of the DNA that is responsible for the expression of cubilin to a lesser degree/extent than do agonists.

ANTAGONIST shall mean moieties that competitively bind to the megalin receptor at the same or a remote site as the agonists but which do not activate the intracellular response initiated by the active form of the receptor or the part of the DNA that is responsible for the expression of the megalin receptor or to cubilin or the part of the DNA that is responsible for the expression of cubilin, and can thereby inhibit the intracellular responses by agonists or partial agonists.

CANDIDATE COMPOUND or CADIDATE AGENT or AGENT shall mean a molecule (for example, and not limiting, a chemical compound) which is amenable to a screening technique. One preferred candidate compound is a modulator in the form of an antagonist.

A COFACTOR shall mean a compound or set of compounds that will promote the binding of a modulator and/or candidate compound to the megalin-receptor. Potential examples of such cofactors are derivatives of already known binding partners of megalin, like modified cubilin, vitamin-binding proteins, carrier proteins, lipoproteins, apolipoproteins (such as apo E and apo B), clusterin, LPO-antagonists (e.g., probucol), RAP and derivatives thereof, Ca²⁺, Ca²⁺- scavengers, lipids, cytochrome C, antimegalin IgG, anti-cubilin IgG, anti-poly alpha2,8 KDN antibody, aminoglycosides (e.g. gentamicin), hormones and precursors thereof, drugs and toxins, enzymes and inhibitors thereof, and immune- and stress-response related proteins, maleate, dextran-based conjugates, aristolochic acid, cadmium chloride, and L-carnithine. Already known cofactors are, for example, Ca²⁺ for clusterin-binding. When applying these cofactors in the extracellular environment, the derivatives must be able to pass the filtration barrier of the glomerulus.

A MODULATOR will be a compound that modifies the activity of the megalin receptor when applied extracellularly and/or intracellularly. The term "modulator" as used in the context of the present invention thus relates to a compound or group of compounds that interact with the megalin-receptor in a way which, in turn, influences the uptake of radiopharmaceutics and/or radiodiagnostics by said receptor. The modulator can inhibit the uptake completely or partially. The modulator can be competitive with the radiopharmaceutical in binding to and uptake by megalin. The compound can either bind directly at the site, at which the radiotherapeutic and/or radiodiagnostic interacts with the receptor and therefore directly prevent the radiotherapeutic and/or radiodiagnostic from binding. In addition, the compound can bind at the megalin receptor at a position that will indirectly influence the uptake of the radiotherapeutic and/or radiodiagnostic, for example, by sterically modifying the structure of the megalin-receptor in such a manner that no or reduced binding and subsequent uptake will occur. The term modulator also includes molecules that influence the expression of the gene encoding for megalin. The compound can modulate the expression either by acting directly on the part of the DNA of the cell that encodes the megalin-receptor (such as a repressor) or by acting indirectly on the protein expression (e.g. by acting on enhancers or by acting on the transcriptional apparatus of the cell). Alternatively, the expression of the receptor can be influenced at the level of regulating the megalin-receptor expression. For example, compounds can be employed that influence the rate of the synthesis of the megalin-receptor in the cell by acting on the control mechanisms of the megalin expression. Examples of modulators are candidate compounds, agents, agonists, antagonists and/or inhibitors of the megalin mediated uptake of radiotherapeutics and/or radiodiagnostics into cells of the kidney.

COMPOUND EFFICACY shall mean a measurement, e.g. screening, of the ability of a compound to inhibit or stimulate megalin receptor functionality, as opposed to megalin receptor binding affinity.

CONTACT or CONTACTING shall mean bringing at least two moieties together, whether in an in vitro system or an in vivo system.

INHIBIT or INHIBITING, in relationship to the term "response" shall mean that a response is decreased or prevented in the presence of a compound as opposed to in the absence of the compound.

INTRACELLULAR INHIBITOR shall mean a moiety that influences the intracellular cycle of the megalin protein, e.g. by influencing the expression and/or turnover, and thereby reduces or prevents the functionality of megalin and cubilin or the expression levels on the cell surface.

LIGAND shall mean an endogenous, naturally occurring molecule specific for an endogenous, naturally occurring receptor.

The MEGALIN MEDIATED UPTAKE OF RADIOPHARMACEUTICALS AND/OR RADIODIAGNOSTICS and/or MEGALIN UPTAKE and/or MEGALIN RECEPTOR ACTIVITY and/or RESPONSE shall mean any kind of uptake of radiotherapeutics and/or radiodiagnostics in which the megalin receptor is functionally directly or indirectly involved. This includes both the function of the protein itself and/or its availability in the uptake process based on its expression and/or turnover in the cells of the kidney. The above mentioned terms encompass also an indirect involvement of the megalin receptor in the uptake process, for example, as a cofactor, regulatory protein and/or part of a protein complex that is formed for the uptake process (e.g. including cubilin).

A PROPHYLACTIC PREVENTION in the context of the present invention shall mean that the treatment using the modulator of the present invention is provided (either exclusively and/or additionally) in order to prevent the cells of the kidney from damages as introduced by the radiopharmaceutical. Such damages could lead to necrosis and/or cancer of the kidney.

PHARMACEUTICAL COMPOSITION shall mean a composition comprising at least one active ingredient, whereby the composition is amenable to investigation for a specified, efficacious outcome in a mammal (for example, and not limitation, a human). Those of ordinary skill in the art will understand and appreciate the techniques appropriate for determining whether an active ingredient has a desired efficacious outcome based upon the needs of the artisan, in this case the modulation megalin-mediated uptake of radiotherapeutics and/or radiodiagnostics into cells of the kidney.

STIMULATE or STIMULATING, in relationship to the term "response" shall mean that a response is increased in the presence of a compound as opposed to in the absence of the compound.

TREATMENT or THERAPY shall mean a process of the reversing or ameliorating or reducing a condition of a certain type or certain types of cells or tissues with respect to an abnormal behaviour, such as a proliferative disease. Preferably, a treatment is applied to a patient.

REPORTER GENE is a sequence which includes a mammalian promoter, a sequence encoding a detectable protein, e.g., luciferase, and, in most cases, a poly(A) signal sequence. Reporter genes are routinely used to investigate upstream regulatory regions. Generally, all or a portion of an upstream regulatory region of interest is position upstream (5') of the reporter gene promoter and the entire construct is introduced into a selected cell type to study the effect of the selected upstream region on expression of the protein encoded by the reporter gene. Reporter genes are often inserted into vectors. For example, the pGL3-Promoter Vector (Promega, Madison, Wis.) is a vector which contains an SV40 promoter upstream of a firefly luciferase gene (including a polyA signal sequence). The vector includes sequences required for replication of the vector in E. coli and mammalian cells. A sequence element is OPERABLY LINKED to a reporter gene when it is inserted upstream of the reporter gene promoter such that can influence expression of the protein encoded by the gene. For example, an estrogen responsive element inserted into the polylinker found upstream of the SV40 promoter in pGL3-Promoter Vector is operably linked to the luciferase reporter gene.

### DETAILED DESCRIPTION

According to a first aspect of the present invention, a method of modulating the megalin-receptor mediated binding and/or uptake of a radiotherapeutic into cells of the kidney in a patient in need thereof is provided. In general, said inventive method comprises the steps of a) administering to said patient an effective amount of a radiotherapeutic or a pharmaceutically acceptable salt thereof, and b) administering to said patient an effective amount of a modulator of the megalin-receptor mediated uptake of said radiotherapeutic into cells of the kidney, or a pharmaceutical salt thereof; a prodrug thereof; or a metabolite thereof. According to another aspect of the present invention, the modulator can be administered before, simultaneously together with and/or after said radiotherapeutic.

According to yet another aspect of the method according to the present invention, said modulator is a modulator of the expression of the megalin receptor in kidney cells. Most preferred is a method according to the present invention, wherein said modulator is an inhibitor of the megalin-receptor mediated uptake of said radiotherapeutic into cells of the kidney.

In the course of a preferred method according to the present invention, an effective amount of a radiotherapeutic and/radiodiagnostic is administered to a patient or subject to be diagnosed. Usually, the radiotherapeutic and/or radiodiagnostic is selected from the group of compounds comprising radiolabeled antibodies, radiolabeled enzymes, radiolabeled nucleic acids, comprising radiometals selected from the group of Flourine-18, Chromium-51, Cobalt-58, Gallium-67, Molybdenum-99, Technetium-99, Indium-111, Iodine-123, Iodine-125, Iodine-131, Yttrium-90, gold Au-198 colloid, Xenon-133, Thallium-201, Rhenium-188, Strontium-189, Actinium-225, Astatine-211, Bismuth-212/213, Rhenium-186, Holmium-166, Samarium-153, and Lutetium-177. The use and application of radiopharmaceutics and/or radiotherapeutics as well as treatment regimen and dosage forms are well known in the art and described, for example, in Wiseman et al. (2001); Förster G.J. et al. (2001); Behr et al. (2002); and Kwekkeboom D.J., et al. (2001).

The method of treatment and/or the diagnosis according to the present invention is usually performed during the diagnosis, treatment and/or monitoring of a proliferative disease or proliferative diseases. Thus, the method according to the present invention can be employed in case of a cancer, which can be selected from the group comprising prostate cancer, breast cancer, leukaemia, pancreatic cancer, lung cancer, colon cancer, sarcoma, glioblastoma, melanoma, ovarian cancer, head and neck carcinomas, and lymphoma. In addition, radiodiagnostics and/or radiotherapeutics are also used in other diseases, for example Parkinson disease, Alzheimer syndrome, coronary diseases, and restenosis. Preferably, the administration is performed during the prevention of damages of the kidney of a patient during a radiotherapy.

In the further course of the preferred method according to the present invention, an effective amount of a modulator of the megalin mediated uptake of radiopharmaceutics and/or radiotherapeutics into cells of the kidney is administered either together with before or after the radiodiagnostic and/or radiotherapeutic. Suitable modulators and their nature are described below in the context of the screening methods of the present invention. In brief, said modulator exhibits an affinity to megalin and/or cubilin of 50-500 nM, preferably of less than 50 nM. Preferably, said modulator is able to pass through the filtration barrier of the glomerulus. Furthermore, said modulator reduces the uptake of said radiodiagnostics and/or radiotherapeutics into cells of the kidney by more than about 60% and finally, said modulator preferably does not significantly (p < 0.05) reduce the uptake (%ID/g) of said radiotherapeutic and/or radiodiagnostic into tumour cells.

The modulator of the present invention can be applied to said patient together with a suitable cofactor selected from the group of compounds comprising modified cubilin, vitamin-binding proteins, carrier proteins, lipoproteins, apolipoproteins (apo E, and apo B), LPO-antagonists (e.g., probucol), RAP and derivatives thereof, Ca²⁺, Ca²⁺-scavengers, lipids, cytochrome C, antimegalin IgG, anti-cubilin IgG, anti-poly alpha2,8 KDN antibody, aminoglycosides (e.g. gentamicin), hormones and precursors thereof, drugs and toxins, enzymes and inhibitors thereof, and immune- and stress-response related proteins, maleate, dextran-based conjugates, aristolochic acid, cadmium chloride, and L-camithine. The cofactor will further modify the interaction of the megalin receptor and the modulator, either by increasing the efficiency of the modulator towards the modulation of radiopharmaceutics and/or radiotherapeutics into cells of the kidney or will enable the modulator to bind to a complex comprising megalin, the cofactor and the modulator in order to exhibit a function.

For in vivo use, the agents/modulators of the present invention are provided to a patient as a means of reducing the amount or rate of binding to and/or uptake of radiotherapeutics and/or radiodiagnostics by megalin. Furthermore, the compounds can be used in order to reduce the amount of megalin expression or turnover inside cells of the kidney.

The present invention therefore provides pharmaceutical compositions comprising a modulator of the invention. These pharmaceutical compositions may be administered orally, rectally, parenterally, intracistemally, intravaginally, intraperitoneally, topically (as by powders, ointments, drops or transdermal patch), bucally, or as an oral or nasal spray.

As used herein, "pharmaceutically acceptable carrier" is intended to mean a non-toxic solid, semisolid or liquid filler, diluent, encapsulating material or formulation auxiliary of any type. The term "parenteral" as used herein refers to modes of administration which include intravenous, intramuscular, intraperitoneal, intrastemal, subcutaneous and intraarticular injection and infusion. One of ordinary skill will recognise that the choice of a particular mode of administration can be made empirically based upon considerations such as the particular disease state being treated; the type and degree of the response to be achieved; the specific agent or composition employed; the age, body weight, general health, sex and diet of the patient; the time of administration and rate of excretion of the agent or composition; the duration of the treatment; drugs (such as a chemotherapeutic agent) used in combination or coincidental with the specific composition; and like factors well known in the medical arts.

Pharmaceutical compositions of the present invention for parenteral injection may comprise pharmaceutically acceptable sterile aqueous or nonaqueous solutions, dispersions, suspensions or emulsions as well as sterile powders for reconstitution into sterile injectable solutions or dispersions just prior to use. Illustrative examples of suitable aqueous and nonaqueous carriers, diluents, solvents or vehicles include, but are not limited to, water, ethanol, polyols (such as glycerol, propylene glycol, polyethylene glycol, and the like), carboxymethylcelulose and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

The compositions of the present invention may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption such as aluminium monostearate and gelatine.

In some cases, in order to prolong the effect of the modulator or inhibitor, it is desirable to slow the absorption from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilised, for example, by filtration through a bacterial-retaining filter, or by incorporating sterilising agents in the form of sterile solid compositions which can be dissolved or dispersed in sterile water or other sterile injectable medium just prior to use.

Solid dosage forms for oral administration include, but are not limited to, capsules, tablets, pills, powders, and granules. In such solid dosage forms, the active compounds are preferably mixed with at least one pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatine, polyvinylpyrrolidone, sucrose, and acacia, c) humectants such as glycerol, d) disintegrating agents such as agar agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and I) lubricants such as talc, calcium stearate, magnesium stearate, solid polyethylene glycols, sodium lauryl sulfate, and mixtures thereof. In the case of capsules, tablets and pills, the dosage form may also comprise buffering agents as appropriate.

Solid compositions of a similar type may also be employed as fillers in soft and hard filled gelatine capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Illustrative examples of embedding compositions which can be used include polymeric substances and waxes.

The active agents of the invention can also be in microencapsulated form, if appropriate, with one or more of the above-mentioned excipients. Liquid dosage forms for oral administration include, but are not limited to, pharmaceutically acceptable emulsions, solutions, suspensions, syrups and elixirs. In addition to the active compounds, the liquid dosage forms may contain inert diluents commonly used in the art such as, for example, water or other solvents, solubilising agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfiryl alcohol, polyethylene glycols and fatty acid esters of sorbitan, and mixtures thereof.

Besides inert diluents, the oral compositions may also contain adjuvants such as wetting agents, emulsifying and suspending agents, sweetening, flavouring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminium metahydroxide, bentonite, agar--agar, and tragacanth, and mixtures thereof.

The agent or inhibitor can also be administered in the form of liposomes. As is known to those skilled in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolisable lipid capable of forming liposomes can be used. The present compositions in liposome form can contain, in addition to the agent or inhibitor, stabilisers, preservatives, excipients, and the like. Preferred lipids are phospholipids and phosphatidyl cholines (lecithins), both natural and synthetic. Methods to form liposomes are known in the art. See, e.g., Prescott, ed., (1976).

The agents of the present invention can be formulated according to known methods to prepare pharmaceutically acceptable compositions, whereby these materials, or their functional derivatives, are combined in a mixture with a pharmaceutically acceptable carrier vehicle. Suitable vehicles and their formulation, inclusive of other human proteins, e.g., human serum albumin, are well known in the art. In order to form a pharmaceutically acceptable composition suitable for effective administration, such compositions will contain an effective amount of one or more agents of the present invention.

Additional pharmaceutical methods may be employed to control the duration of action. Control release preparations may be achieved through the use of polymers to complex or absorb the therapeutic agents of the invention. The controlled delivery may be exercised by selecting appropriate macromolecules (such as polyesters, polyamino acids, polyvinyl, pyrrolidone, ethylenevinylacetate, methylcellulose, carboxymethylcellulose, or protamine sulfate) and methods of incorporation in order to control release. Another possible method to control the duration of action by controlled release preparations is to incorporate antibodies into particles of a polymeric material such as polyesters, polyamino acids, hydrogels, poly(lactic acid) or ethylene vinyl acetate copolymers. Alternatively, instead of incorporating these agents into polymeric particles, it is possible to entrap these materials in microcapsules prepared, for example, by coacervation techniques or by interfacial polymerization, for example, hydroxymethylcellulose or gelatine-microcapsules and poly(methylmethacylate) microcapsules, respectively, or in colloidal drug delivery systems, for example, liposomes, albumin microspheres, microemulsions, nanoparticles, and nanocapsules or in macroemulsions.

The pharmaceutical formulations of the present invention are prepared, for example, by admixing the active agent with solvents and/or carriers, optionally using emulsifiers and/or dispersants, whilst if water is used as the diluent, organic solvents may be used as solubilising agents or auxiliary solvents. As described above, the excipients used include, for example, water, pharmaceutically acceptable organic solvents such as paraffins, vegetable oils, mono- or polyfunctional alcohols, carriers such as natural mineral powders, synthetic mineral powders, sugars, emulsifiers and lubricants.

One of ordinary skill will appreciate that effective amounts of the inventive modulating agents can be determined empirically and may be employed in pure form or, where such forms exist, in pharmaceutically acceptable salt, ester or prodrug form. The agonist or antagonist my be administered in compositions in combination with one or more pharmaceutically acceptable excipients. It will be understood that, when administered to a human patient, the total daily usage of the agents and compositions of the present invention will be decided by the attending physician within the scope of sound medical judgement. The specific therapeutically effective dose level for any particular patient will depend upon a variety of factors including the type and degree of the response to be achieved; the specific agent or composition employed; the age, body weight, general health, sex and diet of the patient; the time of administration, route of administration, and rate of excretion of the agent or composition; the duration of the treatment; drugs (such as a chemotherapeutic agent) used in combination or coincidental with the specific composition; and like factors well known in the medical arts.

Techniques of dosage determination are well known in the art for e. g. antibody and peptide agents. In general, it is desirable to provide a patient with a dosage of antibody or peptide agent in the range of from about 1 pg/kg to 10 mg/kg (body weight of patient). The therapeutically effective dose can be lowered if the agent of the present invention is additionally administered with another compound. As used herein, one compound is said to be additionally administered with a second compound when the administration of the two compounds is in such proximity of time that both compounds can be detected at the same time in the patient's serum.

For example, satisfactory results are obtained by oral administration of therapeutic dosages on the order of from 0.05 to 10 mg/kg/day, preferably 0.1 to 7.5 mg/kg/day, more preferably 0.1 to 2 mg/kg/day, administered once or, in divided doses, 2 to 4 times per day. On administration parenterally, for example by i.v. drip or infusion, dosages on the order of from 0.01 to 5 mg/kg/day, preferably 0.05 to 1.0 mg/kg/day and more preferably 0.1 to 1.0 mg/kg/day can be used. Suitable daily dosages for patients are thus on the order of from 2.5 to 500 mg p.o., preferably 5 to 250 mg p.o., more preferably 5 to 100 mg p.o., or on the order of from 0.5 to 250 mg i.v., preferably 2.5 to 125 mg i.v. and more preferably 2.5 to 50 mg i.v.

Dosaging may also be arranged in a patient specific manner to provide a predetermined concentration of an agent in the blood, as determined by the RIA technique. Thus patient dosaging may be adjusted to achieve regular on-going trough blood levels, as measured by RIA, on the order of from 50 to 1000 ng/ml, preferably 150 to 500 ng/ml.

As mentioned above, the modulator is preferably administered as an injection (intraperitoneally and/or intravenously), an infusion, a bolus dose and/or as an oral dosage, either alone or in combination with a cofactor which can be a compound selected from the group of compounds comprising modified cubilin, vitamin-binding proteins, carrier proteins, lipoproteins, apolipoproteins (apo E and apo B), clusterin, LPO-antagonists (e.g., probucol), RAP and derivatives thereof, Ca²⁺, Ca²⁺-scavengers, lipids, cytochrome C, antimegalin IgG, anti-cubilin IgG, anti-poly alpha2,8 KDN antibody, aminoglycosides (e.g. gentamicin), hormones and precursors thereof, drugs and toxins, enzymes and inhibitors thereof, and immune- and stress-response related proteins, maleate, dextran-based conjugates, aristolochic acid, cadmium chloride, and L-camithine. Said cofactor itself preferably exhibits an affinity to megalin and/or cubilin of 50-500 nM, preferably of less than 50 nM. Preferably, said cofactor is able to pass through the filtration barrier of the glomerulus and can help for the modulator of the present invention to reduce the uptake of the radiopharmaceutics and/or radiotherapeutics into cells of the kidney by more than about 60%. Logically, the cofactor itself should also not significantly (p < 0.05) reduce the uptake (%ID/g) of said radiotherapeutic into tumour cells.

Similar to the modulator of the invention, the cofactor is administered during the prevention, diagnosis, monitoring and/or treatment of cancer, e.g. selected from the group comprising prostate cancer, breast cancer, leukaemia, pancreatic cancer, lung cancer, colon cancer, sarcoma, glioblastoma, melanoma, ovarian cancer, head and neck carcinomas, and lymphoma. In addition, radiodiagnostics and/or radiotherapeutics are also used in other diseases, for example Parkinson disease, Alzheimer syndrome, coronary diseases, and restenosis.

Another important aspect of the present invention is directed to a method of screening for modulator compounds of the megalin-receptor mediated binding and/or uptake of radiotherapeutics and/or radiodiagnostics into cells of the kidney. Said method comprises the steps of : a) providing an assay for measuring the megalin-receptor mediated binding and/or uptake of radiotherapeutics and/or radiodiagnostics, b) adding at least one radiotherapeutic and/or radiodiagnostic and/or a suitably labelled precursor thereof to the assay; c) adding a candidate compound to be tested to the assay; and d) determining if the amount of radiotherapeutics and/or radiodiagnostics bound to or taken up by the megalin-receptor-mediated binding and/or uptake is altered as compared to binding or uptake in the absence of the candidate compound to be tested, wherein a difference in binding or uptake mediated by the megalin-receptor identifies a compound which is a modulator of the megalin-receptor mediated binding and/or uptake of radiotherapeutics and/or radiodiagnostics into cells of the kidney. Specific methods for screening are detailed below. Preferably, said candidate compound is added before, simultaneously together with and/or after said radiotherapeutic and/or radiodiagnostic during the screening process.

According to another aspect of the present invention, said determining of the amount of the radiotherapeutic and/or radiodiagnostics bound to or taken up by the megalin-receptor-mediated binding and/or uptake is altered as compared to binding or uptake in the absence of the candidate compound to be tested comprises measuring the expression of the megalin receptor in kidney cells.

According to yet another aspect of the present invention, the method further comprises measuring the expression of cubilin in the cells of the kidney.

According to yet another aspect of the present invention and as detailed below, the invention provides a method wherein the candidate compound is added to the assay together with a cofactor selected from the group of compounds comprising modified cubilin, vitamin-binding proteins, carrier proteins, lipoproteins, apolipoproteins (apo E and apo B), clusterin, LPO-antagonists (e.g., probucol), RAP and derivatives thereof, Ca²⁺, Ca²⁺-scavengers, lipids, cytochrome C, antimegalin IgG, anti-cubilin IgG, anti-poly alpha2,8 KDN antibody, aminoglycosides (e.g. gentamicin), hormones and precursors thereof, drugs and toxins, enzymes and inhibitors thereof, and immune- and stress-response related proteins, maleate, dextran-based conjugates, aristolochic acid, cadmium chloride, and L-camithine. The cofactor will in some cases further modify the interaction of the megalin receptor and the modulator, either by increasing the efficiency of the modulator towards the modulation of radiopharmaceutics and/or radiotherapeutics into cells of the kidney or will enable the modulator to bind to a complex comprising megalin, the cofactor and the modulator in order to exhibit a function. Therefore, in these cases the cofactor will modify the result of the screening procedure and allow the detection of agents that could not be identified in screenings that do not employ cofactors.

The screening methods of and assays of the invention can be performed in vitro. Preferably, a mammalian cell culture can be used, which more, preferably, comprises kidney cells. The use of cells, tissues, and cell cultures is further detailed below. In yet another aspect of the present invention, a mammalian cell that is used for the screening method is transformed with a plasmid containing a DNA sequence encoding the megalin-receptor. Optionally, said cell mammalian cell is further transformed with a plasmid containing a DNA sequence encoding cubilin. Preferably, the mammalian cell is a human cell or a mouse cell.

The screening methods of and assays of the invention can also be performed in vivo. Preferably, the method is an vivo assay that is performed in a mouse.

In yet another aspect of the method according to the present invention, the assay includes a cell expressing the megalin-receptor protein and the modulator is a nucleic acid sequence which alters the expression of the megalin-receptor protein. One embodiment of this method will be an assay involving a recombinant megalin-producing cell and an antisense oligonucleotide, as detailed below.

The radiopharmaceutics and/or radiotherapeutics used in the screening methods according to the present invention can be identical to the radiopharmaceutics and/or radiotherapeutics as used in the in vivo diagnoses and/or treatments. Thus, the radiopharmaceutics and/or radiotherapeutics can be selected from the group of compounds comprising radiolabeled antibodies, radiolabeled enzymes, radiolabeled nucleic acids, comprising radiometals selected from the group of Flourine-18, Chromium-51, Cobalt-58, Gallium-67, Molybdenum-99, Technetium-99, Indium-111, Iodine-123, Iodine-125, Iodine-131, Yttrium-90, Gold Au-198 colloid, Xenon-133, Thallium-201, Rhenium-188, Strontium-189, Actinium-225, Astatine-211, Bismuth-212/213, Rhenium-186, Holmium-166, Samarium-153, and Lutetium-177.

Most preferred is a method of the present invention, wherein the candidate compound is selected from a library of naturally occurring or synthetic compounds which are randomly tested for the alteration of binding and/or modulation of the megalin uptake. As an example, the candidate compound can be selected from the group of megalin-binding compounds comprising modified cubilin, vitamin-binding proteins, carrier proteins, lipoproteins, apolipoproteins (e.g. apo E, and apo B, clusterin), LPO-antagonists (e.g., probucol), RAP and derivatives thereof, Ca²⁺, Ca²⁺-scavengers, lipids, cytochrome C, antimegalin IgG, anti-cubilin IgG, anti-poly alpha2,8 KDN antibody, aminoglycosides (e.g. gentamicin), hormones and precursors thereof, drugs and toxins, enzymes and inhibitors thereof, and immune- and stress-response related proteins, maleate, dextran-based conjugates, aristolochic acid, cadmium chloride, and L-camithine.

As described and detailed below, various methods for screening candidate agents to identify modulators of the uptake of radiotherapeutics and/or radiodiagnostics by the megalin receptor can be employed.

The methods of the invention rely on examining the effect of various agents based on either their effect on the megalin receptor mediated uptake of radiotherapeutics and/or radiodiagnostics or the level of expression of the megalin receptor in kidney cells. Thus, a modulator can generally be identified based on either its effect on the amount of radioactivity that is taken up or bound by the megalin receptor or its effect on the expression of a megalin gene in a particular cell type, e.g. kidney cells.

In one aspect, the present invention relates to screening methods which can be used to identify agents which modulate the megalin-receptor mediated binding and/or uptake of radiotherapeutics into cells of the kidney. Such agents are potentially useful in the treatment of cancer diseases. Preferred agents are inhibitors of the megalin-receptor mediated binding and/or uptake of radiotherapeutics into cells of the kidney. Treatment of cancers with radiotherapeutics together with the modulators will generally be associated with less undesirable side-effects than treatment with a radiotherapeutic alone. Furthermore, the radiotherapeutics could also be used at elevated doses and in a prolonged treatment regimen.

The methods of the invention permit the identification of agents which modulate the megalin-receptor mediated binding and/or uptake of radiotherapeutics into cells of the kidney directly by determining the amount of radiotherapeutics that is bound to or taken up by a kidney cell or kidney cell lines or renal tissue.

The methods of the invention are screening assays in which candidate agents are examined to identify suitable modulators of the megalin-receptor mediated binding and/or uptake of radiotherapeutics into cells of the kidney. One type of screening assay involves determining if the amount of radiotherapeutics bound to or taken up by the megalin-receptor-mediated binding and/or uptake is altered as compared to binding or uptake in the absence of the compound to be tested, wherein a difference in binding or uptake mediated by the megalin-receptor identifies a compound which modulates the megalin-receptor mediated binding and/or uptake of radiotherapeutics into cells of the kidney. During the course of the assay of the method according to the invention, the compound will initially bind or attach to the receptor. The compound can either bind directly to megalin at the same site, at which the radiotherapeutic interacts with the receptor and therefore directly prevent the radiotherapeutic from binding. In addition, the compound can bind at the megalin receptor at a position that will indirectly influence the uptake of the radiotherapeutic, for example, by sterically modifying the structure of the megalin-receptor in such a manner, that no binding and subsequent uptake will occur. Furthermore, cofactors can be present, like certain ions or cubilin, that promote the attachment of the compound to the receptor and therefore support the function of the modulator. "Binding" can occur via a covalent or non-covalent attachment of the compound or group of compounds to megalin. Based on the binding properties of the screened compounds, first pre-selection of compounds can be performed, in which a non-binding compound is screened in a second "round" of screening using a set of co-factors. If still no binding occurs, the compound will be classified as "non-binding" and not used in further screenings. Such pre-selection will be encompassed in the terms "screening" and/or "determining" in the general context of this invention. In general, assays to determine a binding and biological effect of a compound to a specific target (in this case megalin) are well known to the person skilled in the art and can be found, for example, in US patents 4,980,281, 5,266,464 and 5,688,655 to Housey for phenotypic changes of cells after incubation with a screening compound. Furthermore, US 5,925,333 to Krieger at al. (which is herein incorporated by reference) describes methods for modulation of lipid uptake and related screening methods. US 2002-055092 (incorporated herein by reference) contemplates high throughput screening of agents using optical detection techniques.

One may use the screening method to identify such substances which activate or repress the uptake function of the megalin receptor protein, and thus lead to elevated or decreased uptake of radiotherapeutics and/or radiodiagnostics into the cells. In turn, this will improve the responsiveness of a person to radiotherapeutic and/or radiodiagnostic therapy when treated in combination with the substance, and therefore the substance might be used to treat medical indications such as cancer.

In another embodiment, the invention concerns the isolation of substance inhibiting the interaction of a cofactor protein and megalin. Such substances are useful for the development of drugs against diseases as listed above. Substances disrupting the interactions may be isolated by a variety of screening methods including the two hybrid system or the reverse two hybrid system (Lenna C.A. and Hannink, 1996), or any variation of cellular or cell free assays as described in this invention, as is obvious to anyone skilled in the art.

The present invention provides cell based assays. In order to identify a candidate substance capable of influencing the megalin uptake activity, one might first obtain a recombinant cell line. One designs the cell line in such a way that the activity of the megalin receptor leads to the expression of a protein which has an easily detectable phenotype (a reporter), such as luciferase, fluorescent proteins such as green or red fluorescent protein, beta-galactosidase, alpha-galactosidase, beta-lactamase, chloramphenicol-acetyl-transferase, beta-glucuronidase, or any protein which can be detected by a secondary reagent such as an antibody. Methods for detecting such proteins using antibodies, such as ELISA assays, are well known to those skilled in the art.

The amount of reporter protein as present reflects the activity of the megalin receptor. In one embodiment, this recombinant cell line is then screened for the effect of substances on the expression of megalin, thus measuring the effect of these substances on the activity of the promoter. These substances can be derived from natural sources, such as fungal extracts, plant extracts, bacterial extracts, higher eukaryotic cell extracts, or even extracts from animal sources, or marine, forest or soil samples, and may be assayed for the presence of potentially useful pharmaceutical agents.

In a second embodiment, the above recombinant cell line is screened for the effect of substances on the megalin mediated uptake, thus measuring the effect of substances on the activity of the megalin itself. These substances can also be derived from natural sources, such as fungal extracts, plant extracts, bacterial extracts, higher eukaryotic cell extracts, or even extracts from animal sources, or marine, forest or soil samples, and may also be assayed for the presence of potentially useful pharmaceutical agents.

In general one of the assays can be performed by firstly bringing a suitable cell containing a reporter gene which transcription is influenced by megalin promoter activity in contact with a compound and secondly monitoring the expression of the reporter gene to evaluate the effect of the compound on the activity of the promoter.

Recombinant forms of the megalin receptor and/or cubilin can be used in cell-free screening assays aiming at the isolation of compounds affecting the activity of the megalin uptake. In such an assay, the megalin and/or cubilin polypeptides are brought into contact with a substance to test if the substance has an effect on the megalin uptake.

The detection of an interaction between an agent and megalin may be accomplished through techniques well-known in the art. These techniques include but are not limited to centrifugation, chromatography, electrophoresis and spectroscopy. The use of isotopically labelled reagents in conjunction with these techniques or alone is also contemplated. Commonly used radioactive isotopes include ³H, ²²Na, ³³P, ³⁵S, ⁴⁵Ca, ⁶⁰Co, and ¹³¹I. Also, but less commonly used stable isotopes include ¹⁴C, ³²P, and ¹²⁵I,.

For example, if an agent binds to megalin, the binding may be detected by using a radiolabeled agent. Briefly, if radiolabeled agent is utilised, the agent-megalin or cubilin complex may be detected by liquid scintillation or by exposure to x-ray film or phospho-imaging devices.

One way to screen for substances affecting megalin uptake activity is to measure the effect of the substance on the binding affinity of megalin to radiopharmaceutics and/or radiotherapeutics. Assays measuring the binding of a protein to a ligand are well known in the art and include ELISA assays, FRET assays, bandshift assays, plasmon-resonance based assays, scintilllation proximity assays, fluorescence polarisation assays, and alpha screen assays.

In one example, a mixture containing a megalin polypeptide, optionally a cofactor and candidate substance is allowed to incubate. The unbound cofactor is separable from any megalin/cofactor complex so formed. One then simply measures the amount of each (*e.g.*, versus a control to which no candidate substance has been added). This measurement may be made at various time points where such data is desired. From this, one determines the ability of the candidate substance to alter or modify the function of the megalin uptake.

A screening assay in which candidate agents that modulated binding and/or uptake of radiopharmaceutics and/or radiodiagnostics by megalin is analysed can include a number of conditions. These conditions include but are not limited to pH, temperature, tonicity, the presence of relevant other proteins, and relevant modifications to the polypeptide such as glycosylation or lipidation. It is contemplated that the megalin can be expressed and utilised in a prokaryotic or eukaryotic cell. The host cell expressing the megalin can be used whole or the cofactor can be isolated from the host cell. The megalin can be membrane bound in the membrane of the host cell or it can be free in the cytosol of the host cell. The host cell can also be fractionated into sub-cellular fractions where the megalin can be found. For example, cells expressing the megalin and/or cubilin can be fractionated into the nuclei, the endoplasmic reticulum, vesicles, or the membrane surfaces of the cell.

In preferred embodiments, the pH is preferably from about a value of 6.0 to a value of about 8.0, more preferably from about a value of about 6.8 to a value of about 7.8, and most preferably, about 7.4. In a preferred embodiment, temperature is from about 20°C degrees to about 50°C degrees, more preferably, from about 30°C degrees to about 40°C degrees and even more preferably about 37°C degrees. Osmolality is preferably from about 5 milliosmols per liter (mosm/L) to about 400 mosm/l, and more preferably, from about 200 milliosmols per liter to about 400 mosm/l and, even more preferably from about 290 milliosmols/L to about 310 mosm/L. The presence of further cofactors, like cubilin or other proteins can be required for the proper functioning of the megalin for screening according to the invention. Typical chemical cofactors include sodium, potassium, calcium, magnesium, and chloride. In addition, small, non-peptide molecules, known as prosthetic groups may also be required. Other biological conditions needed for megalin and/or cubilin function are well-known in the art.

It is well-known in the art that proteins can be reconstituted in artificial membranes, vesicles or liposomes. (Danboldt et al.,1990). The present invention contemplates that the megalin can be incorporated into artificial membranes, vesicles or liposomes. The reconstituted megalin can be utilised in screening assays of the invention.

It is further contemplated that megalin or a cofactor, such as cubilin, can be coupled to a solid support, e.g., to agarose beads, polyacrylamide beads, polyacrylic, sepharose beads or other solid matrices capable of being coupled to polypeptides. Well-known coupling agents include cyanogen bromide (CNBr), carbonyldiimidazole, tosyl chloride, diaminopimelimidate, and glutaraldehyde.

In a typical screening assay for identifying candidate substances, one employs the same recombinant expression host as the starting source for obtaining the megalin polypeptide, generally prepared in the form of a crude homogenate. Recombinant cells expressing the megalin are washed and homogenised to prepare a crude polypeptide homogenate in a desirable buffer such as disclosed herein. In a typical assay, an amount of polypeptide from the cell homogenate, is placed into a small volume of an appropriate assay buffer at an appropriate pH. Candidate substances, such as agonists and antagonists, are added to the admixture in convenient concentrations and the interaction between the candidate substance and the megalin and/or megalin uptake of radiopharmaceutics and/or radiotherapeutics is monitored.

Where one uses an appropriate known substrate for megalin, like radiopharmaceutics and/or radiotherapeutics, one can, in the foregoing manner, obtain a baseline activity for the recombinantly produced megalin, optionally in the presence or absence of cubilin. Then, to test for inhibitors or modifiers of the megalin uptake, one can incorporate into the admixture a candidate substance whose effect on the megalin uptake of radiopharmaceutics and/or radiotherapeutics is unknown. By comparing reactions which are carried out in the presence or absence of the candidate substance, one can then obtain information regarding the effect of the candidate substance on the uptake of megalin into the cells of the kidney.

Accordingly, this aspect of the present invention will provide those of skill in the art with methodology that allows for the identification of candidate substances having the ability to modify the megalin uptake in one or more manners.

Additionally, screening assays for the testing of candidate substances are designed to allow the determination of structure-activity relationships of agonists or antagonists with megalin, e.g., comparisons of binding between naturally-occurring substances that are known to bind to megalin or comparison of the activity caused by the binding of such molecules to megalin.

In certain aspects, the polypeptides, like megalin and/or cubilin of the invention are crystallised or co-crystallised in order to carry out x-ray crystallographic studies as a means of evaluating interactions with candidate substances or other molecules with the polypeptide(s). For instance, the purified recombinant polypeptides of the invention, i.e. of megalin, when crystallised in a suitable form, are amenable to detection of intra-molecular interactions by x-ray crystallography. In another aspect, the structure of the polypeptides can be determined using nuclear magnetic resonance.

Libraries of molecularly diverse molecules for screening purposes of the present invention can be prepared using chemical and/or recombinant technology. Such libraries for screening include libraries of so-called "small molecules" having a molecular weight of less than approximately 1000, and recombinantly-produced libraries of proteins and/or fusion proteins.

An exemplary recombinantly-produced library is prepared by ligating fragments of cDNA into, for example, the pGEX-2T vector (Pharmacia, Piscataway, NJ). This vector contains the carboxy terminus of glutathione S-transferase (GST) from *Schistosoma japonicum.* Use of the GST-containing vector facilitates purification of GST-candidate fusion proteins from bacterial lysates by affinity chromatography on glutathione sepharose. After elution from the affinity column, the fusion proteins are tested for activity by, for example, contacting at least one fusion protein with an megalin-expressing cell. Fusion proteins which inhibit the megalin-receptor mediated binding and/or uptake of radiotherapeutics into cells of the kidney are selected as pharmaceutical lead compounds and/or to facilitate further characterisation of the portion of megalin to which the protein binds. See, for example, Koivunen E. et al. (1993) which describes the selection of peptides which bind to the alpha₅beta₁ integrin from a phage display library.

According to yet another aspect of the invention, a functional assay is provided for screening a molecular library to identify a pharmaceutical lead compound. A particularly preferred functional screening method involves performing an adhesion assay between a radiotherapeutic and/or a radiodiagnostic and a megalin expressing cell in the presence and absence of at least one member of the molecular library to determine, whether the library member modulates the adhesion between the radiotherapeutic and/or radiodiagnostic and the megalin expressing cell in vitro. This embodiment involves: (1) performing a first adhesion assay between a radiotherapeutic and/or radiodiagnostic and a megalin expressing cell to obtain a first adhesion assay result; (2) performing a second adhesion assay between the radiotherapeutic and/or radiodiagnostic and the megalin-expressing cell in the presence of the library member to obtain a second adhesion assay result; and (3) comparing the first and the second adhesion assay results to determine whether the library member modulates megalin-mediated uptake of radiotherapeutics and/or radiodiagnostics into the megalin expressing cell. Thus, for example, an adhesion assay result which shows reduced binding between the radiotherapeutic and/or radiodiagnostic and the megalin expressing cell when the assay is conducted in the presence of the library member, compared to the assay result obtained when the assay is performed in the absence of the library member indicates that the library member inhibits binding of a radiotherapeutic and/or radiodiagnostic to megalin expressing cells. An exemplary adhesion assay is provided in the Examples. Other such adhesion assays are well known in the art and can be developed and performed using no more than routine experimentation. Thus, for example, the adhesion assay can be performed by substituting the above-described alternative binding partners, e.g., an isolated ligand and its isolated receptor, for the above-mentioned radiotherapeutic and/or radiodiagnostic and megalin expressing cell.

In one embodiment of the present invention, methods for screening a candidate compound as a modulator of the megalin-mediated uptake of radiotherapeutic and/or radiodiagnostic into biological material are provided. Biological materials which may be examined using such techniques include cells, tissues, organs, in particular derived from the kidney of mammals. The inventive techniques may be employed in high-throughput screening of potential diagnostic and/or therapeutic agents.

In another aspect of the present invention, a method for screening a candidate compound for activity as a diagnostic or therapeutic agent is provided, comprising: maintaining at least one sample population of biological material; introducing a candidate compound and a radiotherapeutic and/radiodiagnostic to the sample population; acquiring and recording at least one test data set corresponding to one or more properties of the sample population following introduction of the candidate compound; comparing the test data set to a comparison data set, wherein the comparison data set corresponds to one or more properties of a predetermined sample population having a predetermined uptake state; and assessing the activity of the candidate compound as a diagnostic or therapeutic agent based on the comparison between the test data set and the comparison data set.

The methods and systems of the present invention employ radiation detection techniques for assessing the modulation of the megalin-mediated uptake of radiotherapeutics and/or radiodiagnostics into biological materials such as tissues, and cells, and may be used in both in vitro and in vivo systems. One important application of the methods of the present invention is high-throughput screening of candidate agents and conditions to evaluate their suitability as diagnostic or therapeutic agents.

Methods for assessing the effects of various agents or physiological activities on biological materials, in both in vitro and in vivo systems, generally comprise also the assessment of the effect of a physiological agent, such as a drug, on a population of cells or tissue grown in culture. Additionally, current assessment techniques generally provide information relating to a single or a small number of parameters. Candidate agents are systematically tested for cytotoxicity, which may be determined as a function of concentration. A population of cells is treated and, at one or several time points following treatment, cell survival is measured. A similar scenario is present for measuring the uptake of substances into cells. Similarly, agents are frequently evaluated based on their physiological effects, for example, on a particular metabolic function or metabolite. An agent is administered to a population of cells or a tissue sample, and the metabolic function or metabolite of interest is assayed to assess the effect of the agent. These assessments can be further employed, when measuring the effect of the modulator(s) of the megalin uptake.

U.S. Pat. Nos. 5,902,732 and 5,976,825, (incorporated herein by reference) disclose methods for screening drug candidate compounds for anti-epileptic activity using glial cells in culture by osomotically shocking glial cells, introducing a drug candidate, and assessing whether the drug candidate is capable of abating changes in glial cell swelling. These patents also disclose a method for screening drug candidate compounds for activity to prevent or treat symptoms of Alzheimer's disease, or to prevent CNS damage resulting from ischemia, by adding a sensitization agent capable of inducing apoptosis and an osmotic stressing agent to CNS cells, adding the drug candidate, and assessing whether the drug candidate is capable of abating cell swelling. U.S. Pat. Nos. 6,096,510 discloses additional methods and systems for assessing biological materials using optical detection techniques. A method for determining the viability and health of living cells inside polymeric tissue implants is also disclosed, involving measuring dimensions of living cells inside the polymeric matrix, osmotically shocking the cells, and then assessing changes in cell swelling. Assessment of cell swelling activity is achieved by measuring intrinsic optical signals using an optical imaging screening apparatus.

A cell line where the activity of a megalin receptor determines the expression of a reporter can be obtained by generating an artificial promoter upstream of a reporter gene. Furthermore, transgenic animals as described in the invention can be used to derive cell lines useful for cellular screening assays.

Cell lines useful for such cellular screening assays include many different kinds of cells, including prokaryotic, animal, fungal, plant and human cells. Yeast cells can be used in this assay, including *Saccharomyces cerevisiae* and *Schizosaccharomyces pombe* cells.

One way of building cellular assays is by measuring the effect of compounds is the use of the two hybrid system (see, for example, U.S. Pat. No. 5,283,317; Zervos et al. (1993) Cell 72:223-232; Madura et al. (1993); Bartel et al. (1993); Iwabuchi et al. (1993); WO 94/10300, and U.S. Pat. No. 5,667,973), or possible variants of the basic two hybrid system as discussed e.g in Vidal M et al. (1999). Briefly, the two hybrid assay relies on reconstituting in vivo a functional transcriptional activator protein from two separate fusion proteins. In particular, the method makes use of chimeric genes which express hybrid proteins. To illustrate, a first hybrid gene comprises the coding sequence for a DNA-binding domain of a transcriptional activator fused in frame to the coding sequence for a cofactor. The second hybrid protein encodes a transcriptional activation domain fused in frame to another gene, for example megalin. If the cofactor and megalin proteins are able to interact, they bring into close proximity the two domains of the transcriptional activator. This proximity is sufficient to cause transcription of a reporter gene which is operably linked to a transcriptional regulatory site responsive to the transcriptional activator, and expression of the reporter gene can be detected and used to score for the interaction of the cofactor and megalin. Suitable host cells for such assays include yeast cells, but also mammalian cells or bacterial cells.

In such assays, one primarily measures the effect of a compound on a given interaction involving the megalin receptor, and optionally additional cofactors, like cubilin, and a binding protein to be screened. In a preferred embodiment of the invention systems using other hosts such as prokaryotes as *E. coli,* or eukaryotic mammalian cells are described.

Two hybrid systems using hybrid protein fusions with other proteins than transcription factors, including enzymes such as beta-galactosidase or dihydrofolate reductase may also be applied. These assays are useful both to monitor the effect of a compound, including peptides, proteins or nucleic acids on an interaction of megalin with radiodiagnostics and/or radiotherapeutics, as well as to identify novel proteins or nucleic acids interacting with megalin.

Cells from nearly every organ and tissue, of both plant and animal origin, can be dissociated into single cells, grown and propagated using cell culture techniques. Pathological cells from diseased or dysfunctional tissue can also be isolated and maintained in tissue culture. Various types of tissues, including normal, dysfunctional and malignant tissues, may be maintained under culture conditions for prolonged periods of time and assessed according to methods of the present invention. Short-term experiments may also be conducted on living acute tissue samples that are prepared and maintained under physiological conditions. Many multicellular systems and tissues may also be maintained as functioning systems in cell culture. Healthy, pathogenic and dysfunctional cells and tissue may also be tested and observed in situ in living animal models, including humans, using methods and compounds of the present invention.

Another aspect of the methods of the present invention involves the development and use of databases cataloguing the effects of the test agents on the megalin-mediated uptake of radiotherapeutics and/or radiodiagnostics of the megalin-expressing biological materials including cells, tissues, organs, subcellular components, portions of intact organisms, and the like. Changes of the uptake induced (or not) by administration of a test agent are detected, stored, compared to changes induced in different biological systems and/or by administration of different test agents, and patterns are identified. The changes induced by different test agents on specific biological sample materials have unique profiles that are predictive of various types of responses and are useful for the development and screening of candidate therapeutic and diagnostic compositions.

The database may archive, for example, the response patterns of megalin-expressing-cells, tissues and whole animal sample populations to existing, approved therapeutic agents. The response patterns of candidate agents and combinations may then be compared to and screened against the response patterns of approved and presumably safe and efficacious known agents to predict the response of the candidate agent and/or combination on the megalin-mediated uptake of radiotherapeutics and/or radiodiagnostics into cells of the kidney.

Comparison of screening data against response patterns stored in the database provides valuable assessment of target validation, lead selection and optimisation, and detection of side effects. Detection and analysis of the properties of individual cells or sample cell populations provides information permitting the classification of the uptake state of individual cells or sample cell populations. Based on analysis of the uptake properties of a sample cell population, the sample may be classified as "inhibition". The use of probes and labelled markers and assaying of extrinsic optical properties of sample populations to assess various uptake conditions at resolved locations in time and in space provides yet further information relating to cellular and biological systems responses to various test agents.

Screening techniques may be adapted for use with various types of cell sample populations maintained in vitro under appropriate cell culture conditions to provide a high throughput, automated screening system. Alternatively, screening techniques may be adapted to examine cell and tissue populations using various animal models to assess the effect of the administration of a test agent on the megalin mediated uptake of a radiotherapeutic and/or radiodiagnostic in animal models in situ. Screening techniques of the present invention may also be implemented to examine cell and tissue populations, using animal models, to assess the effect(s) of genetic modifications of such animal models in situ.

Various data processing techniques may be advantageously used to assess the data collected in accordance with the present invention. Comparison data may be assessed or presented in a variety of formats. Processing may include averaging or otherwise combining a plurality of data sets to produce control, subsequent and various comparison data sets. Data may be converted from an analogue to a digital form for processing, and back to an analogue form for display as an image. Alternatively, data may be acquired, processed, analysed, and output in a digital form Data processing may also include amplification of certain signals or portions of a data set (e.g., areas of an image) to enhance the contrast seen in data set comparisons, and to thereby identify kidney cells or kidney cell populations undergoing changes.

Data processing techniques may also be used to manipulate data sets to provide more accurate combined and comparison data. It is important that corresponding data points in data sets are spatially resolved and precisely aligned to provide accurate combined and comparison data. Suitable markers may be fixed at an area of interest and detected as the data is collected to aid in manual alignment or mathematical manipulation of data sets. Various processing techniques are described below and in the patents incorporated herein by reference.

The data storage processing and analysis function is generally performed and controlled by a host computer. The host computer may comprise any general computer (such as an IBM PC type with an Intel 386, 486, Pentium or similar microprocessor or Sun SPARC) that is interfaced with the emr source and/or optical detector and controls data acquisition and flow, comparison computations, analysis, output, and the like. The host computer thus controls acquisition and analysis of data and provides a user interface. The host computer may comprise a single-board embedded computer with a VME64 interface, or a standard (IEEE 1014-1987) VME interface, depending upon bus band width considerations. Host computer boards which may be employed in the present invention include, for example, Force SPARC/CPU-2E and HP9000 Model 7471. The user interface can be, for example, a Unix/X-Window environment.

Thus, for example, a candidate for the modulation of the megalin-mediated uptake of radiotherapeutics and/or radiodiagnostics into cells of the kidney may be screened using various types of kidney cell and tissue populations to determine safety and efficacy in the kidney, as well as other types of cell populations, such as hepatic cell populations, to determine safety and efficacy in other portions of the biological system. Similarly, a candidate for treating a renal cancer may be screened using various types of cancer and normal cell populations to assess the specificity and toxicity of the candidate agent or combination on the megalin-mediated uptake of radiotherapeutics and/or radiodiagnostics into cells of various cell populations. Additionally, the effect of genetic modifications on the numerous physiological states and conditions described herein may be assessed with respect to various cell and tissue sample populations and compared to unaltered, wild-type sample populations, or to differently genetically altered sample populations.

Antibody-based screening assays are performed by, for example, contacting an radioactively labelled therapeutic and/or megalin-specific antibody with a megalin receptor in the presence and absence of at least one member of the molecular library and determining whether the library member modulates binding between the antibody and the receptor megalin. In a particularly preferred embodiment, the cell is a megalin-expressing cell, an isolated megalin or an isolated peptide related to, or derived from, the extracellular domain of megalin. Such antibodies and their production are known to the person skilled in the art, and further described below.

In a particularly preferred embodiment, the antibody screening method involves: (1) performing a first antibody assay in the absence of the library molecule to obtain a first antibody assay result; (2) performing a second antibody assay in the presence of the library molecule to obtain a second antibody assay result; and (3) comparing the first and the second antibody assay results to determine whether the molecular library member modulates binding between the antibody and the receptor megalin. According to this embodiment, a second antibody assay result which shows reduced binding between the antibody and receptor megalin indicates that the library member has inhibited binding of antibody to the receptor megalin. Antibody binding assays also can be used to assess the relative affinity of a molecular library member for a heterotypic cognate of megalin, using no more than routine experimentation.

It will be understood that that the agents to be screened can also be derived from chemical compositions or man-made compounds. Accordingly, in screening assays for identifying modulators and/or pharmaceutical agents which affect megalin uptake, it is proposed that compounds isolated from natural sources, such as fungal extracts, plant extracts, bacterial extracts, higher eukaryotic cell extracts, or even extracts from animal sources, or marine, forest or soil samples, may be assayed for the presence of potentially useful modulators or pharmaceutical agents. The candidate substances could also include monoclonal or polyclonal antibodies, peptides or proteins, such as those derived from recombinant DNA technology or by other means, including chemical peptide synthesis. The active compounds may include fragments or parts or derivatives of naturally-occurring compounds or may be only found as active combinations of known compounds which are otherwise inactive. We anticipate that such screens will in some cases lead to the isolation of agonists of megalin, either alone or in combination with cofactors, in other cases to the isolation of antagonists. In other instances, substances will be identified that have mixed agonistic and antagonistic effects, or affect the megalin mediated uptake in any other way.

Agents for use in the screening assays of the invention can be obtained from any source. Libraries of synthetic and/or natural compounds are particularly useful. Numerous means are currently used for random and directed synthesis of saccharide, peptide, nucleic acid, and small molecule compounds. Synthetic compound libraries are commercially available from Maybridge Chemical Co. (Trevillet, Cornwall, UK), Comgenex (Princeton, N.J.), Brandon Associates (Merrimack, N.H.), and Microsource (New Milford, Conn.). A rare chemical library is available from Aldrich (Milwaukee, Wis.). Libraries of natural compounds in the form of bacterial, fungal, plant and animal extracts are available from Pan Laboratories (Bothell, Wash.) and MycoSearch (N.C.). Phytoestrogens (Maleka et al., 1994; Knight et al., 1995) and the components of Premarin (Stem, 1982; Jayalilaka et al., 1993) can also be screened using the methods of the invention.

The candidate substances can also include monoclonal or polyclonal antibodies, peptides or proteins, such as those derived from recombinant DNA technology or by other means, including chemical peptide synthesis. The active compounds may include fragments or parts or derivatives of naturally-occurring compounds or may be only found as active combinations of known compounds which are otherwise inactive.

Numerous agents are known for treatment of various conditions, such as cancers and other proliferative disorders. In one approach, such agents may be screened using techniques of the present invention. Modifications to such agents may then be made, for example, using combinatorial chemistry and molecular modelling techniques, to produce modified candidate agents, which may be screened for activity as a modulator of the megalin mediated uptake of radiotherapeutics and/or radiodiagnostics into cells of the kidney using methods of the present invention. Similarly, combinations of both known agents and modified agents may be screened using techniques of the present invention.

In one exemplary embodiment, candidate agent combinations for treating cancer are tested using screening methods of the present invention. Candidate compounds may be screened for megalin radiotherapeutic and/or radiodiagnostic uptake, binding and/or transport agonist and/or antagonist activity using screening methods of the present invention with various types of cells in culture such as renal cells, and the like, or in situ in animal models. Following administration of the candidate agents, the corresponding uptake properties of the cell or tissue sample are monitored to determine whether the cells responded by altering the radioactivity in the extracellular and intracellular space.

The following agents, and modifications of the following agents, including analogues, derivatives, fragments, active moieties, and the like, may be screened using methods and systems of the present invention: AA (ara-C, Adriamycin); AAF (2-acetylaminofluorene); AAFC (flurocitabine); ABC (Adriamycin, BCNU, cyclophosphamide); ABCD (Adriamycin, bleomycin, CCNU and dacarbazine); ABCM (Adriamycin, bleomycin, cyclophosphamide, mitomycin-C); ABD (Adriamycin, bleomycin, DTIC); ABDIC (Adriamycin, bleomycin, dacarbazine, CCNU and prednisone); ABDV (Adriamycin, bleomycin, DTIC, vinblastine); ABLC (amphotericin B lipid complex); ABOS (doxorubicin, bleomycin sulfate, vincristine, streptozocin); ABP (Adriamycin, bleomycin, prednisone); ABPP (bropirimine); ABV (actinomycin-D, bleomycin, vincristine); ABV (Adriamycin, bleomycin, vinblastine); ABVD (Adriamycin, bleomycin, vincristine, dacarbazine); ABVE (Adriamycin, bleomycin, vincristine, etoposide); ABVP (Adriamycin, bleomycin sulfate, teniposide, prednisone); ABVP (Adriamycin, bleomycin sulfate, vinblastine, prednisone); ABVP (Adriamycin, bleomycin sulfate, vincristine, prednisone); AC (Adriamycin, carmustine); AC (Adriamycin, CCNU); AC (Adriamycin, cisplatin); AccuSite; Ac-D-Ac (Adriamycin, daunorubicin, Adriamycin); ACe (Adriamycin, cyclophosphamide); ACE (Adriamycin, cyclophosphamide, etoposide); ACFUCY (actinomycin D, 5-fluorouracil, cyclophosphamide); ACID (Adriamycin, cyclophosphamide, imidazole, dactinomycin); Acivicin; aclarubicin; aclarubicin HCl; ACM (Adriamycin, cyclophosphamide, methotrexate); ACNU (nimustine); ACOAP (Adriamycin, cyclophosphamide, Oncovin, cytosine arabinoside, prednisone); ACOP (Adriamycin, cyclophosphamide, Oncovin, prednisone); ACOPP (Adriamycin, cyclophosphamide, Oncovin, prednisone, procarbazine); ACR (aclarubicin); ACT (actinomycin); ACT-C (actinomycin-C); ACT-D (actinomycin-D); Actinex (masoprocol); actinomycin-D (ACT-D); ACT-FU-Cy (actinomycin-D, 5-FU, cyclophosphamide); ADBC (Adriamycin, DTIC, bleomycin, CCNU); ADE (ara-C, daunorubicin, etoposide); adenine arabinoside (Ara-A); ADIC (Adriamycin, DTIC); ADOC (Adriamycin, cisplatin, vincristine, cyclophosphamide); AdOAP (Adriamycin, Oncovin, ara-C, prednisone); AdOP (Adriamycin, Oncovin, prednisone); adozelesin; ADR-529; Adria+BCNU (Adriamycin+BCNU); Adriamycin PFS; Adriamycin RDF; Adria-L-PAM (Adriamycin, L-phenylalanine mustard); Adrucil (fluorouracil); AF 1890 (lonidamine); AFM (Adriamycin, 5-fluorouracil, methotrexate); AGT (aminoglutethimide); Agrelin (anagrelide); AID (Adriamycin, ifosfamide, dacarbazine, mesna); AIM (L-asparaginase, ifosfamide, methotrexate); AL-721; aldesleukin (Proleukin); alfa-2 or 26 interferon; alfacalcidol (One-Alpha); Alferon LDO; interferon alfa-n3); Alferon-N (interferon alfa-n3); Alkaban-AQ (vinblastine); Alkeran (melphalan); all trans-retinoic acid (Tretinoin); Allergan 211 (idoxuridine); ALOMAD (Adriamycin, Leukeran, Oncovin, methotrexate, actinomycin D, dacarbazine); Alpha-Beta (alpha tocopherol and beta carotene); Alpha Chymar (alpha-chymotrypsin); alpha interferon (IFN-A); alphal-antitrypsin; alpha-2 interferon(IFN-alpha-2); 5-alpha reductase inhibitors; alpha-TGI (teroxirone); AlphaNine; ALTO (afterloading tandem and ovoids); ALT-RCC (autolymphocyte-based treatment for renal cell carcinoma); altretamine (Hexalen, Hexastat); AmBisome; amethopterin (methotrexate); amiloride (Midamor); 9-aminocamptothecin; aminoglutethimide (Cytadren, AGT, Elipten); aminopterin; aminothiadiazole; AML-2-23 monoclonal antibody; AMMEN-OE5 monoclonal antibody; amonafide (nafidimide); amphotericin B colloidal dispersion (ABCD); amphotericin B lipid complex (ABLC); Ampligen (polyribonucleotide); AMSA (amsacrine); amsacrine (m-AMSA, AMSA, Amsidyl); Amsidyl (amsacrine); anakinra (Antril); anagrelide (Agrelin); ANAN (anandron); anandron (nilutamide); anastrozole (Arimidex); Andro-Cyp; Android-F (**Note: Discontinued in 1991); Andronaq (**Note: Discontinued in 1991); Andronate (testosterone cypionate); Andropository (testosterone enanthate); Andryl (testosterone); annamycinLF (liposomal annamycin); anthramycin (also antramycin); anthrapyrazole; anti-B4-blocked ricin; anti-EGFR (RG 838520); anti-MY9-blocked ricin; anti-T12 allogeneic BMT; anti-TAP-72 immunotoxin; Antril (anakinra); AOPA (ara-C, Oncovin, prednisone, asparaginase); AOPE (Adriamycin, Oncovin, prednisone, etoposide); APC (AMSA, prednisone, chlorambucil); APE (Adriamycin, Platinol, etoposide); APE (ara-C, Platinol, etoposide); APO (Adriamycin, prednisone, Oncovin); AR-623 (liposomal Tretinoin); ara-A (adenine arabinoside); ara-AC (azacytosine arabinoside) (fazarabine); arabinofurano-sylcytosine (cytarabine); arabinosyl cytosine (cytarabine); ara-C (cytosine arabinoside) (cytarabine); ara-C+ADR (cytarabine, Adriamycin); ara-C+DNR+PRED+MP (cytarabine, daunorubicin, prednisolone, mercaptopurine); ara-C+6TG (cytarabine, thioguanine); ara-C-HU (ara-C, liydroxyurea); Ara-Cytidine (cytarabine); ara-G; Aredia (pamidronate disodium); Arensin (fadrozole); Arimidex (anastrozole); Arotinoid; ASHAP (Adriamycin, Solu-Medrol, high-dose ara-C, Platinol); ASHAP/BACOS; ASN (L-asparaginase); ASP (L-asparaginase); asparaginase (Elspar); ATG (antithymocyte globulin); Atzpodien regimen for renal cell carcinoma; autolymphocyte therapy; AV (Adriamycin, vincristine); AVAD (doxorubicin, vincristine, cytarabine, dexamethasone); Avicidin; AVM (Adriamycin, vinblastine, methotrexate); AVM (Adriamycin, vincristine, mitomycin-C); AVP (actinomycin D, vincristine, Platinol); AVP (Adriamycin, vincristine, procarbazine); azacitidine; 5-azacitidine (Mylosar); azathioprine (Imuran); AZC (azacitidine); aziridinylbenzoquinone (diaziquone); AZQ (aziridinylbenzoquinone); BAC (BCNU, cytarabine, cyclophosphamide); Bacatin III; BACO (bleomycin, Adriamycin, CCNU, Oncovin); BACOD (bleomycin, Adriamycin, cyclophosphamide, Oncovin, dexamethasone); BACON (bleomycin, Adriamycin, CCNU, Oncovin, nitrogen mustard); BACOP (bleomycin, Adriamycin, cyclophosphamide, Oncovin, prednisone); BACT (BCNU, ara-C, cyclophosphamide, 6-thioguanine); BACT (bleomycin, Adriamycin, cyclophosphamide, tamoxifen); BAM/BLITZ (B4 blocked ricin); BAMON (bleomycin, Adriamycin, methotrexate, Oncovin, nitrogen mustard); BAP (bleomycin, Adriamycin, prednisone); BAPP (bleomycin, doxorubicin, cisplatin, prednisone); BAVIP (bleomycin, Adriamycin, vinblastine, imidazole carboxanide, prednisone); BBVP-M (BCNU, bleomycin, VePesid, prednisone, methotrexate); BCAP (BCNU, cyclophosphamide, Adriamycin, prednisone); BCAVe (bleomycin, CCNU, Adriamycin, Velban); BCD (bleomycin, cyclophosphamide, dactinomycin); BCG (bacillus Calmette-Guerin vaccine); B-CHOP (bleomycin Cytoxan, hydroxydaunomycin, Oncovin, prednisone); BCMF (bleomycin, cyclophosphamide, methotrexate, fluorouracil); BCNU (bis-chloronitrosourea) (carmustine); BCOP (BCNU, cyclophosphamide, Oncovin, prednisone); BCP (BCNU, cyclophosphamide, prednisone); BCVP (BCNU, cyclophosphamide, vincristine, prednisone); BCVPP (BCNU, cyclophosphamaide, vinblastine, procarbazine, prednisone); BCVPP-bleo (BCNU, cyclophosphamide, vinblastine, procarbazine, bleomycin); BCX-34; B-DOPA (bleomycin, dacarbazine, Oncovin, prednisone, Adriamycin); BEAC (BCNU, etoposide, ara-C, cyclophosphamide); BEAM (BCNU, etoposide, cytarabine, melphalan); BEMP (bleomycin, Eldisine, mitomycin, Platinol); BEP (bleomycin, etoposide, Platinol); Betaseron (interferon beta-16); BHD (BCNU, hydroxyurea, dacarbazine); BHDV (BCNU, hydroxyurea, dacarbazine, vincristine); bicalutamide; BiCNU (carmustine); B-IFN (beta-interferon); Biodel Implant/BCNU; BioTropin; BIP (bleomycin, ifosfamide, Platinol); bisacetamide (heramethylene); bisantrene hydrochloride (Bisantrene); Bisantrene (bisantrene hydrochloride); bischlorethylnitrosurea (BCNU); bispecific antibody 520C9x22; bizelesin; Blenoxane (bleomycin); BLEO (bleomycin); BLEO-COMF (bleomycin, cyclophosphamide, Oncovin, methotrexate, fluorouracil); BLEO-MOPP (bleomycin, nitrogen mustard, Oncovin, procarbazine, prednisone); bleomycin (Blenoxane) (BLEO) (BLM); bleomycin HCl; bleomycin sulfate; BLITZ (monoclonal antibodies); BLM (bleomycin sulfate); BM-92103; B-MOPP (bleomycin, mechlorethamine, Oncovin, procarbazine, prednisone); BMP (BCNU, methotrexate, procarbazine); BMS-182248 (BR96-Dox); BMY-28090; BMY-45622; BOAP (bleomycin, Oncovin, Adriamycin, prednisone); BOLD (bleomycin, Oncovin, lomustine, dacarbazine); BOMP (bleomycin, Oncovin, Matulane, prednisone); BONP (bleomycin, Oncovin, Natulan, prednisolone); BOP (BCNU, Oncovin, prednisone); BOP (bleomycin, Oncovin, Platinol); BOPAM (bleomycin, Oncovin, prednisone, Adriamycin, mechlorethamine, methotrexate); BOPP (BCNU, Oncovin, procarbazine, prednisone); Borocell; BR-96 (doxorubicin monoclonal antibody immunoconjugate); brachytherapy; BRCA genetic testing; brequinar; brequinar sodium; BRL39123A; bromodeoxyuridine (BUdR); 5-bromodeoxyuridine (broxuridine or BUdR); 5-bromo-2-deoxyuridine; 5-bromouracil; bromfenac; bropirimine; broxuridine (BUdR); BSO (buthionine sulfoximine); BSRL (buserelin) (Suprefact); BT (BCNU, triazinate); BUdR (bromodeoxyuridine or broxuridine); BU (busulfan); BUS (busulfan, Myleran); buserelin (BSRL) (Suprefact); busulfan (BU, Myleran); buthionine sulfoxirnine (BSO); BVAP (BCNU, vincristine, Adriamycin, prednisone); BV-ara-U; BVCPP (BCNU, vinblastine, cyclophosphamide, procarbazine, prednisone); BVD (BCNU, vincristine, dacarbazine); BVDS (bleomycin, Velban, doxorubicin, streptozocin); BVPP (BCNU, vincristine, procarbazine, prednisone); BW 301U (piritrexim); BW A770U (crisnatol mesylate); CA 15-3; CA 72-4; CA (cyclophosphamide, Adriamycin); CABOP, CA-BOP (cyclophosphamide, Adriamycin, bleomycin, Oncovin, prednisone); CABS (CCNU, Adriamycin, bleomycin, streptozocin); CAC (cisplatin, ara-C, caffeine); CACP (cisplatin); cactinomycin (actinomycin C); CAD (cyclophosphamide, Adriamycin, dacarbazine); CAD (cytosine arabinoside, daunorubicin); CADIC (cyclophosphamide, Adriamycin, DTIC); CAE (cyclophosphamide, Adriamycin, etoposide); CAF (cyclophosphamide, Adriamycin, 5-fluorouracil); CAFFI (cyclophosphamide, Adriamycin, 5-fluorouracil by continuous infusion); CAFP (cyclophosphamide, Adriamycin, 5-fluorouracil, prednisone); CAFTH (cyclophosphamide, Adriamycin, 5-fluorouracil, tamoxifen, Halotestin); CAFVP (cyclophosphamide, Adriamycin, 5-fluorouracil, vincristine, prednisone); CALF (cyclophosphamide, Adriamycin, leucovorin calcium, 5-fluorouracil); CALF-E; (cyclophosphamide, Adriamycin, leucovorin calcium, 5-fluorouracil, ethinyl estradiol); calusterone; CAM (cyclophosphamide, Adriamycin, methotrexate); CAMB (cyclophosphamide, Adriamycin, methotrexate, bleomycin); CAMELEON (cytosine arabinoside, high-dose methotrexate, leucovorin, Oncovin); CAMEO (cyclophosphamide, Adriamycin, methotrexate, etoposide, Oncovin); CAMF (cyclophosphamide, Adriamycin, methotrexate, 5-fluorouracil); CAMF (cyclophosphamide, Adriamycin methotrexate, folic acid); CAMLO (cytosine arabinoside, methotrexate, leucovorin, Oncovin); CAMP (cyclophosphamide, Adriamycin, methotrexate, procarbazine); Campath 1H; camptothecin-11 (CPT-11); CAO (cyclophosphamide, Adriamycin, Oncovin); CAP (cyclophosphamide, Adriamycin, Platinol); CAP (cyclophosphamide, Adriamycin, prednisone); CAP-I (cyclophosphamide, Adriamycin, Platinol); CAP-II (cyclophosphamide, Adriamycin, high-dose Platinol); CAP-BOP (cyclophosphamide, Adriamycin, procarbazine, bleomycin, Oncovin, prednisone); capromab pendetide; CAPPr (cyclophosphamide, Adriamycin, Platinol, prednisone); caracemide; carbetimer, carboplatin (Paraplatin); carboxamide; Cardioxane; carmustine (BCNU) (BiCNU); carubicin; carubicin HCl; carzelesin; Casodex; CAT (cytosine arabinoside, Adriamycin, 6-thioguanine); CAT (cytosine arabinoside, thioguanine); Catrix capsules and amp; injections; CAV (cyclophosphamide, Adriamycin, Velban); CAV (cyclophosphamide, Adriamycin, vincristine); CAVe, CA-Ve (CCNU, Adriamycin, Velban); CAVP (cyclophosphamide, Adriamycin, VM-26, prednisone); CAVP-I (cyclophosphamide, Adriamycin, vincristine, prednisone); CAVP-16 (cyclophosphamide, Adriamycin, VP-16); CAVPM (cyclophosphamide, Adriamycin, VP-16, prednisone, methotrexate), CB10-277, CPBBA (cyclophosphamide, bleomycin, procarbazine, prednisone, Adriamycin); CBV (cyclophosphamide, BCNU, VePesid); CBV (cyclophosphamide, BCNU, VP-16); CBVD (CCNU, bleomycin, vinblastine, dexamethasone); CC (carboplatin, cyclophosphamide); CC49 monoclonal antibody; CCAVV (CCNU, cyclophosphamide, Adriamycin, vincristine, VP-16); CCFE (cyclophosphamide, cisplatin, 5-fluorouracil, estramustine); CCM (cyclophosphamide, CCNU, methotrexate); CCMA (CCNU, cyclophosphamide, methotrexate, Adriamycin); CCNU (lomustine); CCNU-OP (CCNU, Oncovin, prednisone); CCOB (CCNU, cyclophosphamide, Oncovin, bleomycin); CCV (CCNU, cyclophosphamide, vincristine); CCV-AV (CCNU, cyclophosphamide, vincristine/Adriamycin, vincristine); CCVB (CCNU, cyclophosphamide, vincristine, bleomycin); CCVPP (CCNU, cyclophosphamide, Velban, procarbazine, prednisone); CCVV (cyclophosphamide, CCNU, VP-16, vincristine); CCVVP (cyclophosphamide, CCNU, VP-16, vincristine, Platinol); CD (cytarabine, daunorubicin); CdA (chlorodeoxyadenosine) (cladribine); 2-CdA (2-chlorodeoxyadenosine); CDC (carboplatin, doxorubicin, cyclophosphamide); CDDP, C-DDP (cis-diamminedichloroplatinum- ); CDDP (cisplatin); CDE (cyclophosphamide, doxorubicin, etoposide); CEAker; CEB (carboplatin, etoposide, bleomycin); CECA (cisplatin, etoposide, cyclophosphamide, Adriamycin), CeeNu (CCNU or lomustine); CEF (cyclophosphamide, epirubicin, 5-fluorouracil); celogovab (OncoScint OV103); CEM (cytosine arabinoside, etoposide, methotrexate); Centixsin; centrifugation; CEP (CCNU, etoposide, prednimustine); CEP (cyclophosphamide, etoposide, Platinol); Ceptrate SC (monoclonal antibodies); Cerubidine (daunorubicin); CEV (cyclophosphamide, etoposide, vincristine); CF (cisplatin, 5-fluorouracil); CF (citrovorum factor); CF (leucovorin); CFL (cisplatin, 5-fluorouracil, leucovorin calcium); CFM (cyclophosphamide, 5-fluorouracil, mitoxantrone); CFP (cyclophosphamide, 5-fluorouracil, prednisone); CFPT (cyclophosphamide, 5-fluorouracil, prednisone, tamoxifen); CGS 16949A; CHAD (cyclophosphamide, hexamethylmelamine, Adriamycin, DDP); CHAMOCA (Cytoxan, hydroxyurea, actinomycin D, methotrexate, Oncovin, calcium, folinate, Adriamycin); CH1VPP, Ch1VPP (chlorambucil, vinblastine, procarbazine, prednisone); CHAP (cyclophosphamide, Hexalen, Adriamycin, Platinol); CHD (cyclophosphamide, hexamethylmelamine, DDP); CHD-R (cyclophosphamide, hexamethylmelamine, DDP, radiotherapy); CHEX-UP (cyclophosphamide, hexamethylmelamine, 5-fluorouracil, Platinol); CHF (cyclophosphamide, hexamethylmelamine, 5-fluorouracil); chimeric 17-1A (C17-1A); chimeric L6 monoclonal antibodies; CHIP (cis-dichlorotranshydroxy-bis-isopropylamine platinum IV); CHIP (iproplatin); chlorambucil (CLB or Leukeran); Chlorbutin (chlorambucil); chlormethine; chlormethine HCl; 8-chlorocamp; 2-chlorodeoxyadenosine (2-CdA); chlorodeoxyadenosine; chlorotrianisene (Tace); chlorozotocin (DCNU); CHL+PRED (chlorambucil , prednisone); Chl-VPP (chlorambucil, vinblastine, procarbazine, prednisone); CHO (cyclophosphamide, hydroxydaunomycin, Oncovin); CHOB (cyclophosphamide, hydroxydaunomycin, Oncovin, bleomycin); CHOD (cyclophosphamide, hydroxydaunomycin, Oncovin, dexamethasone); CHOP (cyclophosphamide, Halotestin, Oncovin, prednisone); CHOP (cyclophosphamide, hydroxydaunomycin, Oncovin, prednisone); CHOP-BLEO (cyclophosphamide, hydroxydaunomycin, Oncovin, prednisone, bleomycin); CHOPE (cyclophosphamide, Halotestin, Oncovin, prednisone, etoposide); CHOR (cyclophosphamide, hydroxydaunomycin, Oncovin, radiotherapy); chromic phosphate P 32; CHVP (cyclophosphamide, hydroxydaunomycin, VM-26, prednisone); CI-958, 973, 980; CIA (CCNU, ifosfamide, Adriamycin); cinchona bark; CIS 39300; cis-retinoic acid (CRA); 13-cis-retinoic acid (13-CRA); CISCA, CisCA (cisplatin, cyclophosphamide, Adriamycin); CISCAii/BViv (cisplatin, cyclophosphamide, Adriamycin, vinblastine, bleomycin); cis-DDP (cisplatin); cis-diamminedichloroplatinum (cisplatin); cisplatin (CDDP or cis-platinum or DDP or Platinol); cisplatin/collagen matrix; citrovorum factor (CF); citrovorum rescue; CIVPP (chlorambucil, vinblastine, procarbazine, prednisone); cladribine (Leustatin); clofarabine; CLB (chlorambucil); CMC (cyclophosphamide, methotrexate, CCNU); CMC-VAP (cyclophosphamide, methotrexate, CCNU, vincristine, Adriamycin,; procarbazine); CMF (cyclophosphamide, methotrexate, 5-fluorouracil); CMF-AV (cyclophosphamide methotrexate, 5-fluorouracil, Adriamycin, vincristine); CMFAVP (cyclophosphamide, methotrexate, 5-fluorouracil, Adriamycin, vincristine,; prednisone); CMF-BLEO (cyclophosphamide, methotrexate, 5-fluorouracil, bleomycin); CMF-FLU (cyclophosphamide, methotrexate, 5-fluorouracil, fluoxymesterone); CMFH (cyclophosphamide, methotrexate, 5-fluorouracil, hydroxyurea); CMFP (cyclophosphamide, methotrexate, 5-fluorouracil, prednisone); CMFPT (cyclophosphamide, methotrexate, 5-fluorouracil, prednisone, tamoxifen); CMFPTH (cyclophosphamide, methotrexate, 5-fluorouracil, prednisone, tamoxifen,; Halotestin); CMFP-VA (cyclophosphamide, methotrexate, 5-fluorouracil, prednisone, vincristine,; Adriamycin); CMFT (cyclophosphamide, methotrexate, 5-fluorouracil, tamoxifen); CMF-TAM (cyclophosphamide, methotrexate, 5-fluorouracil, tamoxifen); CM-5-FU (cyclophosphamide, methotrexate, 5-fluorouracil); CMFV (cyclophosphamide, methotrexate, 5-fluorouracil, vincristine); CMFVAT (cyclophosphamide, methotrexate, 5- fluorouracil, vincristine, Adriamycin,; testosterone); CMFVP (cyclophosphamide, methotrexate, 5-fluorouracil, vincristine, prednisone); CMH (cyclophosphamide, m-AMSA, hydroxyurea); CMOPP, C-MOPP (cyclophosphamide, mechlorethamine, Oncovin, procarbazine; prednisone); CMP (CCNU, methotrexate, procarbazine); CMPF (cyclophosphamide, methotrexate, prednisone, 5-fluorouracil); CMV (cisplatin, methotrexate, vinblastine); CNF (cyclophosphamide, Novantrone, 5-fluorouracil); CNOP, C-NOP (cyclophosphamide, Novantrone, Oncovin, prednisone); COAP (cyclophosphamide, Oncovin, ara-C, prednisone); COAP-BLEO (cyclophosphamide, Oncovin, ara-C, prednisone, bleomycin); COB (cisplatin, Oncovin, bleomycin); COBMAM (cyclophosphamide, Oncovin, bleomycin, methotrexate, Adriamycin,; MeCCNU); COF/COM (cyclophosphamide, Oncovin, 5-fluorouracil/cyclophosphamide, Oncovin,; methotrexate); colaspase; COM (cyclophosphamide, Oncovin, methotrexate); COM (cyclophosphamide, Oncovin, MeCCNU); COMA-A (cyclophosphamide, Oncovin, methotrexate, Adriamycin, ara-C); COMB (cyclophosphamide, Oncovin, methotrexate, bleomycin); COMB (cyclophosphamide, Oncovin, MeCCNU, bleomycin); COMBAP (cyclophosphamide, Oncovin, methotrexate, bleomycin, Adriamycin,; prednisone); COMe (cyclophosphamide, Oncovin, methotrexate); COMET-A (cyclophosphamide, Oncovin, methotrexate, leucovorin, etoposide, ara-c); COMF (cyclophosphamide, Oncovin, methotrexate, 5-fluorouracil); COMLA (cyclophosphamide, Oncovin, methotrexate, leucovorin, ara-C); COMP (Cyclophosphamide, Oncovin, methotrexate, prednisone); conjugated estrogens; CONPADRI, COPADRI-I (cyclophosphamide, Oncovin, L-phenylalanine mustard,; Adriamycin); Consensus interferon; Cooper CMFVP (cyclophosphamide, methotrexate, 5-fluorouracil, vincristine, prednisone); Cooper Regimen (cyclophosphamide, methotrexate, 5-fluorouracil, vincristine, prednisone); COP (cyclophosphamide, Oncovin, prednisone); COP (cyclophosphamide, Oncovin, prednisolone); COPA (cyclophosphamide, Oncovin, prednisone, Adriamycin); COPA-BLEO (cyclophosphamide, Oncovin, prednisone, Adriamycin, bleomycin); COPAC (CCNU, Oncovin, prednisone, Adriamycin, cyclophosphamide); COP-B, COPB (cyclophosphamide, Oncovin, prednigone, bleomycin); COP-BLAM (cyclophosphamide, Oncovin, prednisone, bleomycin, Adriamycin, Matulane); COP-BLEO (cyclophosphamide, Oncovin, prednisone, bleomycin); COPE (cyclophosphamide, Oncovin, Platinol, etoposide); COPP (CCNU, Oncovin, procarbazine, prednisone); COPP (cyclophosphamide, Oncovin, procarbazine, prednisone); Cosmegen (dactinomycin); CP (Cytoxan, Platinol); CP (cyclophosphamide, prednisone); CPA (cyclophosphamide); CPB (cyclophosphamide, Platinol, BCNU); CPC (cyclophosphamide, Platinol, carboplatin); CPDD (cis-platinum diamminedichloride); CPM (CCNU, procarbazine, methotrexate); CPOB (cyclophosphamide, prednisone, Oncovin, bleomycin); CPT-11 (camptothecin-11); CPTR (cyproterone); CRA (cis-retinoic acid); 13-CRA (13-cis-retinoic acid); Crasnitin (C-asparaginase); CRF-187; crisnatol mesylate; CROP (cyclophosphamide, rubidazone, Oncovin, prednisone); CROPAM (cyclophosphamide, rubidazone, Oncovin, prednisone, L-asparaginase,; methotrexate); CT (cytarabine, thioguanine); CTCb (cyclophosphamide, thiotepa, carboplatin); CTX (cyclophosphamide); CTX-Plat (cyclophosphamide, Platinol); CV 205-502; CV (cisplatin, VP-16); CVA (cyclophosphamide, vincristine, Adriamycin); CVA-BMP (cyclophosphamide, vincristine, Adriamycin, BCNU, methotrexate; procarbazine); C-VAD, CVAD (cyclophosphamide, vincristine, Adriamycin, dexamethasone); CVB (CCNU, vinblastine, bleomycin); CVBD (CCNU, vinblastine, bleomycin, dexamethasone); CVD (cisplatin, vinblastine, dacarbazine); CVEB (cisplatin, Velban, etoposide, bleomycin); CVI (carboplatin, VePesid, ifosfamide, Mesnex uroprotection); CVM (cyclophosphamide, vincristine, methotrexate); CVP (cyclophosphamide, vincristine, prednisone); CVP-BLEO (cyclophosphamide, vincristine, prednisone, bleomycin); CVPP (CCNU, vinblastine, prednisone, procarbazine); CVPP (cyclophosphamide, vinblastine, procarbazine, prednisone); CVPP-CCNU (cyclophosphamide, vinblastine, procarbazine, prednisone, CCNU); CY (cyclophosphamide or Cytoxan); CyADIC (cyclophosphamide, Adriamycin, DTIC); CYC (cyclophosphamide); cyclocreatine; cyclohexylnitrosourea (CCNU); cyclophosphamide (CPA or CPM or CYC); Cycoblastin (cyclophosphamide); CyHOP (cyclophosphamide, Halotestin, Oncovin, prednisone); cyproterone (CPTR); cysteamine dichloroplatinum-2; CYT-103-Y-90 (OncoRad); CytaBOM, CYTABOM (cytarabine, bleomycin, Oncovin, mechlorethamine); Cytadren (aminoglutethimide); cytarabine (ara-C or Cytosar-U or cytosine arabinoside); Cytosar-U (cytarabine); cytosine arabinoside (ara-C); Cytoxan (CTX); Cytoxan Lyophilized; CyVADACT, CY-VA-DACT (cyclophosphamide, vincristine, Adriamycin, dactinomycin); CyVADIC (cyclophosphamide, vincristine, methotrexate, Adriamycin, DTIC); CyVMAD (cyclophosphamide, vincristine, methotrexate, Adriamycin, DTIC); DA (daunorubicin, ara-C); dacarbazine (DTIC); DACT (dactinomycin); dactinomycin (actinomycin D); DAP (dianhydrogalactitol, Adriamycin, Platinol); DAP/TMP (dapsone, trimethoprim); DAT (daunomycin, ara-C, 6-thioguanine); DATVP (daunomycin, ara-C, 6-thioguanine, vincristine, prednisone); Datelliptium (ellipticine); daunomycin (DNM); daunorubicin hydrochloride (Cerubidine or DNR); DaunoXome (liposomal daunorubicin); DAV (dibromodulcitol, Adriamycin, vincristine); DAVA (desacetyl vinblastine amide); DAVH (dibromodulcitol, Adriamycin, vincristine, Halotestin); DBD (dibromodulcitol); DBV (dacarbazine, BCNU, vincristine); DC (daunorubicin, cytarabine); DCCMP (daunorubicin, cyclocytidine, 6-metacaptopurine, prednisone); DCF (2-deoxycoformycin or pentostatin); DCM (dichloromethotrexate); DCMP (daunorubicin, cytarabine, 6-metacaptopurine, prednisone); DCT (daunorubicin, cytarabine, thioguanine); DCV (dacarbazine, CCNU, vincristine); DDP (cis-diaminodichloroplatinum or cisplatin); 3-deazaguanine; DECAL (dexamethasone, etoposide, cisplatin, ara-C, L-asparaginase); Decapeptyl (triptorelin); decitabine; deferoxamine; Deladiol-40 (estradiol); Delatest (testosterone); Delatestryl (testosterone); Delestrogen (estradiol); 2-deoxy-5-azacitidine (DAC); deoxycoformycin (DCF or pentostatin); deoxyspergualin (DSG); depAndro, depAndro 200 (testosterone); Depofoam encapsulated cytarabine (DTC 101); Depo-Provera; Depotest (testosterone); Depo-Testosterone; dexormaplatin; dexverapamil; dezaguanine; dezaguanine mesylate; DFDC (gemcitabine); DFMO (eflomithine); DFMO-MGBG; DFV (DDP, 5-fluorouracil, VePesid); DHAC (azacitidine); DHAD (mitoxantrone); DHAP (dexamethasone, high-dose ara-C, Platinol); DI 694; diaziquone (aziridinylbenzoquinone); dibromodulcitol (Mitolactol); Dicorvin; didemnin B; didox; diethyldithiocarbamate (DTC); diethylstilbestrol diphosphate; dihematoporphyrin ethers; dihydro-5-azacitidine (azacitidine or DHAC); DIMOPP (dose-intensified MOPP); Dioval XX, Dioval 40 (estradiol); DL (doxorubicin, lomustine); DM (dexamethasone); DMC (dactinomycin, methotrexate, cyclophosphamide); DMP 840; DNM (daunomycin); DNR (daunorubicin); DOAP (daunorubicin, Oncovin, ara-C, prednisone); docetaxel; dolasestron; DOX (doxorubicin); Doxil (liposome formulation of doxorubicin); doxorubicin (Adriamycin or DOX); Drolban; droloxifene; dromostanolone propionate (Drostanolone); dronabinol; Drostanolone; DTC (diethyldithiocarbamate); DTIC (dacarbazine); DTIC-ACTD (DTIC, actinomycin D); DTIC-Dome (dacarbazine); duazomycin; Durabolin (nandrolone phenpropionate); Duragen (estradiol); Duralutin (hydroxyprogesterone); Duratest (testosterone); Durathate (testosterone); DVB (DDP, vindesine, bleomycin); DVP (daunorubicin, vincristine, prednisone); DVPL-ASP (daunorubicin, vincristine, prednisone, L-asparaginase); DZAPO (daunorubicin, azacitidine, ara-C, prednisone, Oncovin); EAP (etoposide, Adriamycin, Platinol); Eastern Cooperative Oncology Group (ECOG); EBAP (Eldisine, BCNU, Adriamycin, prednisone); echinomycin; EBRT (external beam radiation therapy); ECHO (etoposide, cyclophosphamide, hydroxydaunomycin, Oncovin); 10-EDAM (10-ethyl-10-deaza-aminopterin) (Edatrexate); ECMV (etoposide, Cytoxan, methotrexate, vincristine); ECOG (Eastern Cooperative Oncology Group); EDAM (10-ethyl-deazaaminopterin or 10-EdAM); EDAP (etoposide, dexamethasone, ara-C, Platinol); Edatrexate (10-EDAM) (10-ethyl-10-deaza-aminopterin); eflomithine (DFMO); Efudex (fluorouracil); EFP (etoposide, fluorouracil, Platinol); Einhom regimen; Eldisine (vindesine sulfate); ELF (etoposide, leucovorin, 5-fluorouracil); Elipten (aminoglutethimide); Elliott's B solution; ellipticine; elsamitrucin; Elspar (asparaginase); EMACO (etoposide, methotrexate, actinomycin D, cyclophosphamide, Oncovin); Emcyt (estramustine); E-MVAC (escalated methotrexate, vinblastine, Adriamycin, cyclophosphamide); Endoxan-Asta (cyclophosphamide); enloplatin; enpromate; EP (etoposide, Platinol); EPI (epirubicin); epi-ADR (epinephrine-Adriamycin); epipropidine; epirubicin; epirubicin HCl; EPO (erythropoietin); EPOCH (etoposide, prednisone, Oncovin, cyclophosphamide, Halotestin); Epodyl; Epogen; Eprex (erythropoietin); erbulozole; Ergamisol (levamisole); Erwinase; erwinia asparaginase; erwinia L-asparaginase; ESHAP (etoposide, Solu-Medrol, ara-C, Platinol); esorubicin; esorubicin HCl; esperamycin; Esterified estrogens; Estinyl; Estra-L (estradiol); Estrace; estradiol; Estradurin; estramustine (Emcyt); Estratab; Estraval; Estrone 5; etanidazole; ethacrynic acid; ethinyl estradiol; Ethiodol; Ethiofos (WR2721); 10-ethyl-10-deaza-aminopt- erin (10-EDAM) (Edatrexate); Ethyol (amifostine); etoposide (VP-16, VePesid); etoposide phosphate; etoprine; Eulexin; EVA (etoposide, vinblastine, Adriamycin); EVAP; Everone; EVMAC (escalated methotrexate, vinblastine, Adriamycin, cisplatin); FAC (5-fluorouracil, Adriamycin, cyclophosphamide); FAC-BCG (Ftorafur, Adriamycin, cyclophosphamide, BCG); FAC-LEV (5-fluorouracil, Adriamycin, cyclophosphamide, levamisole); FAC-M (5-fluorouracil, Adriamycin, cyclophosphamide, methotrexate); FACP (Ftorafur, Adriamycin, cyclophosphamide, Platinol); FACS (5-fluorouracil, Adriamycin, cyclophosphamide, streptozocin); FACVP (5-fluorouracil, Adriamycin, cyclophosphamide, VP-16); fadrozole; fadrozole HCl (Arensin); FAM (5-fluorouracil, Adriamycin, mitomycin-C); FAM-C (5-fluorouracil, Adriamycin, methyl-CCNU); FAM-CF (5-fluorouracil, Adriamycin, mitomycin, citrovorum factor); FAME, FAMe (5-fluorouracil, Adriamycin, MeCCNU); FAMMe (5-fluorouracil, Adriamycin, mitomycin-C, MeCCNU); FAMP (fludarabine monophosphate); FAM-S (5-fluorouracil, Adriamycin, mitomycin-C, streptozocin); FAMTX (5-fluorouracil, Adriamycin, high-dose methotrexate); FAP (5-fluorouracil, Adriamycin, Platinol); Farmorubicin (epirubicin HCl); fazarabine (ara-AC); FCAP (5-fluorouracil, cyclophosphamide, Adriamycin, Platinol); FCE (5-fluorouracil, epirubicin, cyclophosphamide); F-CL (5-fluorouracil, leucovorin calcium); FCP (5-fluorouracil, cyclophosphamide, prednisone); FEC (5-fluorouracil, epirubicin, cyclophosphamide); FED (5-fluorouracil, etoposide, DDP); Feminone (discontinued in 1993) (ethyl estradiol); fenretinide (4-HPR); filgrastim (Neupogen) (G-CSF); FIMe (5-fluorouracil, ICRF-159, MeCCNU); finasteride (MK-906); 5+2 protocol (cytarabine, daunorubicin); FK 506; FL (flutamide, leuprolide acetate); FL (flutamide, Lupron depot); FLAC (5-fluorouracil, leucovorin calcium, Adriamycin, cyclophosphamide); FLAG-Ida; FLAP (5-fluorouracil, leucovorin, Adriamycin, Platinol); FLe (5-fluorouracil, levamisole); floxuridine (FUdR); flucytosine (Ancobon or 5-FC); Fludara (fludarabine); fludarabine (FAMP or Fludara); fludarabine phosphate; fluorodeoxyuridine (FUdR); Fluoroplex cream, topical (fluorouracil); fluorouracil (Adrucil or Efudex or 5-fluorouracil or 5-FU or Fluoroplex); Fluosol/BCNU (Fluosol-DA20, BCNU); fluoxymesterone (FMX or Halotestin); fluorodeoxyuridine (FUDR); Fluoroplex (5-fluorouracil); flurocitabine; flutamide (Eulexin or FLUT); FMS (5-fluorouracil, mitomycin-C, streptozocin); FMV (5-fluorouracil, methyl CCNU, vincristine); FNM (5-fluorouracil, Novantrone, methotrexate); FOAM (5-fluorouracil, Oncovin, Adriamycin, mitomycin-C); Folex (methotrexate) (discontinued in 1992); Folex PFS (methotrexate); folinic acid (leucovorin); FOM (5-fluorouracil, Oncovin, mitomycin-C); FOMi (5-fluorouracil, Oncovin, mitomycin-C); Fosfestrol (diethylstilbestrol diphosphate); fosquidone; fostriecin; fostriecin sodium; fotemustine (S 10036); FRACON (framycetin, colistin, nystatin); Ftorafur (1, 2-tetrahydrofuranyl-5-fluorouracil); 5-FU (5-fluorouracil); FUDR (floxuridine); FUdR (5-fluorouracil deoxyribonucleoside); FUM (5-fluorouracil, methotrexate); Fungizone (amphotericin-B); FUra (fluorouracil); FURAM (Ftorafur, Adriamycin, mitomycin-C); Furhman nuclear grade; FUVAC (5-fluorouracil, vinblastine, Adriamycin, cyclophosphamide); FXM (fluoxymesterone or Halotestin); FZ (flutamide, Zoladex); galamustine (G-6-M); gallium nitrate (GAN); Gamimune Normal (immune globulin); gemcitabine (DFDC) (Gemzar); gemcitabine HCl; Gemzar (gemcitabine); Gesterol (progesterone); GL331; Gliadel; glycyrrhetinic acid; GM-CFU (granulocyte-macrophage colony-forming unit); GM-CSF (granulocyte-macrophage colony-stimulating factor); GOD-26MM; gold Au 198; goserelin acetate (ZDX or Zoladex); GVAX; Gynogen L.A. "10", Gynogen L.A. "20", Gynogen L.A. "40" (estradiol); H-447; H (Halotestin); HAD (hexamethylenamine, Adriamycin, DDP); Halodrin; Halotestin; HAM (hexamethylenamine, Adriamycin, melphalan); HAM (hexamethylenamine, Adriamycin, methotrexate); HAMP (hexamethylenamine, Adriamycin, methotrexate, Platinol); HC (hydrocortisone); HCAO (hexamethylenamine, cyclophosphamide, Adriamycin, Platinol); H-CAP (hexamethylenamine, cyclophosphamide, Adriamycin, Platinol); 4-HC (4-hydroperoxycyclophospham- ide or Pergamid); HDARA-C (high-dose ara-C); HDC (pentostatin); HDMTX (high-dose methotrexate); HDMTX-CF (high-dose methotrexate, citrovorum factor); HDMTX/LV (high-dose methotrexate, leucovorin); HDPEB (high-dose PEB or Platinol, etoposide, bleomycin); HD-VAC (high-dose methotrexate plus vinblastine, Adriamycin, cisplatin); hepsulfam; HER2 (monoclonal antibody); Hexa-CAF (Hexalen, cyclophosphamide, Adrucil, Folex); Hexalen (altretamine); hexamethylene; hexamethylene bisacetamide; hexamethyhmelamine; Hexastat (altretamine); HiC-COM (ara-C, citrovorum factor, allopurinol, Elliot B solution, cyclophosphamide,; Oncovin, methotrexate); HIDAC, HiDAC (high-dose ara-C); high-risk ATAC (L-asparaginase, ara-C, VP-16, anti-J2 26 monoclonal antibody,; anti-CALLA hybridoma antibody); Histerone; HMBA (hexamethylene bisacetamide); HIMM (Hexalen); HMTX (high-dose methotrexate); HN2 (nitrogen mustard); HOAP-BLEO (hydroxydaunomycin, Oncovin, ara-C, prednisone, bleomycin); homoharringtonine; HOP (hydroxydaunomycin, Oncovin, prednisone); HPR (fenretinide); 4-HPR (fenretinide); HU (hydroxyurea); human IgM monoclonal antibody to cytomegalovirus; HXM (hexamethylmelamine); Hybolin Improved (nandrolone phenpropionate); Hybri-CEAker; Hy-Gestrone; hydrazine sulfate; 4-hydroxyperoxycyclophospha- mide; Hydrea (hydroxyurea); 4-hydroperoxycyclophosphamide (4-HC); hydroxycarbamide; hydroxyurea (Hydrea); Hylutin; Hyprogest; Hyproval; Hyproxon; ICE (ifosfamide, carboplatin, etoposide); ICI D1694 (Tomudex); ICRF-187; IDA (idarubicin); Idamycin (idarubicin HCl); idarubicin; idarubicin HCl; IDEC-C2B8; IDMTX (intermediate-dose methotrexate); IDX (4'-iodo-4'deoxydoxorubicin); Ifex (ifosfamide); IFF (ifosfamide); IFLrA (recombinant interferon alpha); IFM (ifosfamide); IFN-A (interferon alpha); IFN-alpha 2a (interferon alpha 2a); IFN-G (gamma interferon); IFOS (ifosfamide); ifosfamide; IFX (ifosfamide); IL-2 (interleukin-2); ilmofosine; IMF (Ifex, mesna, Folex, 5-fluorouracil); imidazole carboxamide (dacarbazine); ImmTher; ImmuRAID-AFP; ImmuRAID-CEA; ImmuRAID-hCG; ImmuRAID-LL2; ImmuRAIT-LL2; Imuran (azathioprine); Imuvert (Serratia marcescens extract); IMVP-16 (ifosfamide, methotrexate, VP-16); interferon alfa; interferon alfa-2a; interferon alfa-2b; interferon beta (Serono, R-Frone); interferon gamma-1b; interleukin-1, 1a, 2, 3, 4, 6, 11; intoplicine; Intron-A; iododeoxyuridine; isotretinoin; iododeoxyuridine; IPP (isopropyl pyrrolizine); iproplatin; iridium; irinotecan; Isorvorin (L-leucovorin); IT MTX (intrathecal methotrexate); IUdR (idoxuridine); JT 1001; Kestrone 5; KGC (Keflin, gentamicin, carbenicillin); Kynacyte; ladakamycin; L.A.E.20 (discontinued in 1992); LAM (L-asparaginase, methotrexate); lanreotide acetate; LAPOCA (L-asparaginase, prednisone, Oncovin, cytarabine, Adriamycin); L-ASP (L-asparaginase); L-asparaginase; L-buthionine sulfoximine; L-CF (leucovorin-citrovorum factor); LCR (vincristine); LDI-200; letrozole; Leucomax (molgramostim); L-leucovorin; leucovorin calcium; Leukeran (chlorambucil); Leukine (sargramostim); Leukoglobulin; leuprolide acetate (Leuprorelin); Leuprorelin; leurocristine; Leustatin (2-CdA or cladribine); LEV (levamisole hydrochloride); levamisole (Ergamisol); LGD-1069; liarozole fumarate; liarozole HCl; linker protocol; Linomide (roquinimex); Lipidox (liposomal doxorubicin); liposomal doxorubicin (TLC D-99); liposomal MTP-PE; liposomal nystatin; L-leucovorin (Isovorin); LMF (Leukeran, methotrexate, 5-fluorouracil); L-PAM (L-phenylaline mustard or melphalan); lobaplatin; LOMAC (leucovorin, Oncovin, methotrexate, Adriamycin, cyclophosphamide); lometrexol; lomustine (CCNU); lonidamine; losoxantrone; losoxantrone HCI; LP 2307; L-PAM (L-phenylalanine mustard) (melphalan); Lupron; Lupron Depot; LuVax (monoclonal antibodies); LV (leucovorin); L-VAM (Lupron, Velban, Adriamycin, Mutamycin); LVVP (Leukeran, vinblastine, vincristine, prednisone); Lysodren; M2 (vincristine, carmustine, cyclophosphamide, melphalan, prednisone); MAb, MAB (monoclonal antibody); MAb B72,3; MAb-L6; MABOP (Mustargen, Adriamycin, bleomycin, Oncovin, prednisone); MAC (methotrexate, actinomycin D, cyclophosphamide); MAC (methotrexate, Adriamycin, cyclophosphamide); MAC (niitomycin-C, Adriamycin, cyclophosphamide); MACC (methotrexate, Adriamycin, cyclophosphamide, CCNU); MACHO (methotrexate, asparaginase, cyclophosphamide, hydroxydaunomycin, Oncovin); MACOP-B (methotrexate, Adriamycin, cyclophosphamide, Oncovin, prednisone,; bleomycin); Macrolin (macrophage colony-stimulating factor); Macstim (macrophage colony-stimulating factor); MAD (MeCCNU, Adriamycin); MADDOC (mechlorethamine, Adriamycin, dacarbazine, DDP, Oncovin,; cyclophosphamide); MAID (mesna, Adriamycin, interleukin-3, dacarbazine); MAID (Mesnex, Adriamycin, Ifex, dacarbazine); maitansine; MAK 195 F (monoclonal antibodies); m-AMSA (amsacrine); MAP (melphalan, Adriamycin, prednisone); MAP (mitomycin-C, Adriamycin, Platinol); masoprocol; MAT (multiple agent therapy); Matulane (procarbazine); maytansine; MAZE (m-AMSA, azacitidine, etoposide); M-BACOD (high-dose methotrexate, bleomycin, Adriamycin, cyclophosphamide, Oncovin,; dexamethasone); M-BACOS (methotrexate, bleomycin, Adriamycin, cyclophosphamide, Oncovin,; Solu-Medrol); M-BAM (cyclophosphamide, total body irradiation, monoclonal antibodies); MBC (methotrexate, bleomycin, cisplatin); MBD (methotrexate, bleomycin, cisplatin); MC (mitoxantrone, cytarabine); MCBP (melphalan, cyclophosphamide, BCNU, prednisone); MCP (melphalan, cyclophosphamide, prednisone); M-CSF (macrophage colony-stimulating factor); MCV (methotrexate, cisplatin, vinblastine); MD 28314QA; MDAM; MDL18, 962; MDL 73,147EF; MDLO (metoclopramide, dexamethasone, lorazepam, ondansetron); MDX210 (humanized anti-cancer bispecific antibody); MeCCNU (methyl-CCNU or semustine); mechlorethamine; MECY (methotrexate, cyclophosphamide); medroxyprogesterone; MeFA (methyl-CCNU, 5-fluorouracil, Adriamycin); Megace (megestrol acetate); megestrol acetate (Megace); Melacine (melanoma vaccine); melanoma vaccine (Melacine); melanoma cell lysate vaccine; melengestrol acetate; MeliBu (busulfan); Melimmune-1 (monoclonal antibodies); Melimmune-2 (monoclonal antibodies); melphalan; melphalan IV; MelVax (monoclonal antibodies); Memorial Sloan-Kettering protocol; Menest (esterified estrogens); menogaril; MePRDL (methylprednisolone); merbarone; mercaptopurine; 6-mercaptopurine (6-MP or Purinethol); mesna (Mesnex); Mesnex (mesna); methionyl granulocyte CSF, recombinant; methionyl human granulocyte CSF, recombinant; Methosarb; methotrexate (MTX); methotrexate LFP sodium; methotrexate sodium; methotrexate/Azone (methotrexate, laurocapram); methyl-CCNU (semustine or MeCCNU); methylmercaptopurine; Methylprednisolone; Meticorten; metoprine; Mexate; MF (methotrexate, 5-fluorouracil); MF (mitomycin, 5-fluorouracil); MFP (melphalan, 5-fluorouracil, Provera); MGDF (megakaryocyte growth and development factor); MICE (mesna rescue, ifosfamide, carboplatin, etoposide); MIFA (mitomycin, fluorouracil, Adriamycin); mifepristone (RU486); MIH (procarbazine); MINE (mesna, ifosfamide, Novantrone, etoposide); MINE/ESHAP; mini-COAP (cyclophosphamide, Oncovin, ara-C, prednisone); MINT; MIP-1 alpha; misonidazole; Mithracin (plicamycin); mithramycin (now plicamycin); mitindomide; MITO (mitomycin-C); mitocarcin;mitocromin; mitogillin; mitoguazone; mitolactol (dibromodulcitol); mitomalcin; mitomycin; mitomycin-C (MMC or Mutamycin); mitosper; mitotane; mitoxantrone (Novantrone); MM (mercaptopurine, methotrexate); MMC (mitomycin-C); MMOPP (methotrexate, mechlorethamine, Oncovin, procarbazine, prednisone); MMPR (methylmercaptopurine); MMPT (methylprednisolone pulse therapy); MNA (N-monomethyl-L-arginine); MOAB, MoAb, MAB (monoclonal antibody); MOAD (methotrexate, Oncovin, L-asparaginase, dexamethasone); MOAP (PEG-asparaginase, Oncovin, methotrexate, prednisone); MOB (Mustargen, Oncovin, bleomycin); MOB-III (mitomycin-C, Oncovin, bleomycin, cisplatin); MOCA (methotrexate, Oncovin, cyclophosphamide, Adriamycin); Modrastane; MOF (MeCCNU, Oncovin, 5-fluorouracil); MOF (methotrexate, Oncovin, 5-fluorouracil); MOF-STREP (MeCCNU, Oncovin, 5-fluorouracil); molgramostim; MOMP (mechlorethamine, Oncovin, methotrexate, prednisone); monoclonal antibodies; monoclonal antibodies recognizing B-cell lymphoma idiotypes; monoclonal antibody 17-1A; monoclonal antibody PM-81; monoclonal antibody PM-81 & AML-2-23; Monogen; monomercaptoundecahydro-closo-DQ decaborate sodium; Monox-IX; MOP (mechlorethamine, Oncovin, prednisone); MOP (mechlorethamine, Oncovin, procarbazine); MOP (melphalan, Oncovin, methylprednisolone); MOP-BAP (mechlorethamine, Oncovin, procarbazine, bleomycin, Adriamycin, prednisone); MOPP (mechlorethamine, Oncovin, procarbazine, prednisone); MOPP (methotrexate, Oncovin, procarbazine, prednisone); MOPP-ABV (mechlorethamine, Oncovin, procarbazine, prednisone, Adriamycin,; bleomycin, vinblastine); MOPP-ABVD (mechlorethamine, Oncovin, procarbazine, prednisone, Adriamycin; bleomycin, vinblastine, dacarbazine); MOPP-LO BLEO (mechlorethamine, Oncovin, procarbazine, prednisone, bleomycin); MOPPHDB (mechlorethamine, Oncovin, procarbazine, prednisone, high-dose bleomycin); MOPPLDB (mechlorethamine, Oncovin, procarbazine, prednisone, low-dose bleomycin); MOPr (mechlorethamine, Oncovin, procarbazine); MP (melphalan, prednisone); 6-MP (6-mercaptopurine); MPFL (methotrexate, Platinol, 5-fluorouracil, leucovorin, calcium); MPL (melphalan); MPL+PRED (melphalan, prednisone); MTX (methotrexate); MTX+MP (methotrexate, mercaptopurine); MTX+MP+CTX (methotrexate, mercaptopurine, Cytoxan); Murine L6 monoclonal antibody; Mustargen (mechlorethamine HCl); mustine HCl; Mutamycin (mitomycin); MV (mitoxantrone, VP-16); MVAC (methotrexate, vinblastine, Adriamycin, cisplatin); MVF (mitoxantrone, vincristine, 5-fluorouracil); MVP (mitomycin-C, vinblastine, Platinol); MVPP (mechlorethamine, vinblastine, procarbazine, prednisone); MVT (mitoxantrone, VP-16, thiotepa); MVVPP (mechlorethamine, vincristine, vinblastine, procarbazine, prednisone); Myleran; Mylosar (5-azacytidine); N-901-blocked ricin (Oncolysin S); NAC (nitrogen mustard, Adriamycin, CCNU); Nadrobolic (discontinued in 1994); nafidimide (amonafide); Navelbine (vinorelbine); neon particle; Neosar (cyclophospharnide); neptamustine; NEU differentiation factor; Neupogen (filgrastim); Neu-Sensamide; N-methylhydrazine; nimustine; Nipent (pentostatin); nitrogen mustard; NM (mechlorethamine); N-methylhydrazine (procarbazine); N-monomethyl-L-arginine (MNA); nocodazole; nogalamycin; Nolvadex (tamoxifen); Novantrone (mitoxantrone); NovoSeven; Numega (interleukin-11); CAP (Oncovin, ara-C, prednisone); OAP-BLEO (Oncovin, ara-C, prednisone, bleomycin); OctreoScan 111 (Indium In 111 pentetreotide); octreotide (Sandostatin); O-DAP (Oncovin, dianhydrogalactitol, Adriamycin, Platinol); OLX-102; OMAD (Oncovin, methotrexate, Adriamycin, dactinomycin); onapristone; Oncaspar (pegaspargase); Oncoject; Oncol; Oncolym; Oncolysin B, Oncolysin S; Onco Non-Small Cell; OncoPurge; OncoRad OV103 (CYT-103-Y-90); OncoRad Bladder; OncoRad Ovarian; OncoRad Prostate; OncoScint CR/OV; OncoScint OV103 (celogobab); OncoScint PR; OncoTrac; Oncovin (vincristine); Oncozole (3-deazaguanine); ondansetron; OPAL (Oncovin, prednisone, L-asparaginase); OPEN (Oncovin, prednisone, etoposide, Novantrone); OPP (Oncovin, procarbazine, prednisone); OPPA (Oncovin, procarbazine, prednisone, Adriamycin); Ora-Testryl; ormaplatin; omithine; Orthozyme-CD5; Ossirene; ovarian rhenium-186 MAb; oxisuran; P32; PAB-Esc-C (Platinol, Adriamycin, bleomycin, escalating doses of cyclophosphamide); PAC (Platinol, Adriamycin, cyclophosphamide); PACE (Platinol, Adriamycin, cyclophosphamide, etoposide); paclitaxel (Taxol); PALA (N-phosphonoacetyl-L-aspartate); PAM, L-PAM (phenylalanine mustard); Panel Quest; Panorex (monoclonal antibody 17-1A); Paraplatin (carboplatin); PATCO (prednisone, ara-C, thioguanine, cyclophosphamide, Oncovin); PAVE (procarbazine, Alkeran, Velban); PBV (Platinol, bleomycin, vinblastine); PCB (procarbazine); PCE (Platinol, cyclophosphamide, Eldisine); PCNU; PCV (procarbazine, CCNU, vincristine); PEB (Platinol, etoposide, bleomycin); PEC (Platinol, etoposide, cyclophosphamide); PEG-ADA (Adagen, pegademase bovine); PEG-ASP (PEG-asparaginase); PEG-L-asparaginase; pegaspargase; peliomycin; Penclomedine; pentamustine; pentostatin; PEP (Procytox, epipodophyllotoxinderivative, prednisolone); peplomycin; perfosfamide; Pergamid; PF+E (Platinol, 5-fluorouracil plus etoposide, methotrexate, leucovorin); PFL (Platinum, 5-fluorouracil, leucovorin calcium); PFM (Platinol, 5-fluorouracil, methotrexate); PFT (phenylalanine mustard, 5-fluorouracil, tamoxifen); phenylalanine mustard; phenylbutyrate; Phosphocol P32; Photofrin; PHRT (procarbazine, hydroxyurea, radiotherapy); PIA (Platinol, ifosfamide, Adriamycin); pipobroman; piposulfan; pirarubicin; pirazofurin; piritrexim; piroxantrone; PIXY; PIXY 321; Pixykine; Platinol (cisplatin); Platinol-AQ; platinum diamminodichloride; plicamycin; plomestane; PM (prednimustine); PMB (Platinol, methotrexate, bleomycin); PMF (phenylalanine mustard, methotrexate, 5-fluorouracil); PMFAC (prednisone, methotrexate, 5-fluorouracil, Adriamycin, cyclophosphamide); P-MVAC (Platinol, methotrexate, vinblastine, Adriamycin, carboplatin); POC (procarbazine, Oncovin, CCNU); POCA (prednisone, Oncovin, cytarabine, Adriamycin); POCC (procarbazine, Oncovin, cyclophosphamide, CCNU); POG protocol (Pediatric Oncology Group); polyestradiol; POMP (prednisone, Oncovin, methotrexate, Purinethol); porfimer sodium, porfiromycin; porton asparaginase; PPI-002; PRDL (prednisolone); PRED (prednisone); prednimustine; PRH-E (Platinol, etanidazole); PRIME (procarbazine, ifosfamide, methotrexate); Pro-Depo; procarbazine; Procrit; Progens; Progynon pellets; Prokine (sargramostim); Proleukin (aldesleukin); Proloid (discontinued in 1992); ProMACE (prednisone, methotrexate, Adriamycin, cyclophosphamide, etoposide); ProMACE-CytaBOM (prednisone, methotrexate, Adriamycin, cyclophosphamide,; etoposide, cytarabine, bleomycin, Oncovin, methotrexate); ProMACE-MOPP (procarbazine, methotrexate, Adriamycin, cyclophosphamide, etoposide; Mustargen, Oncovin, procarbazine, prednisone); properomine protocol; Proscar; pulse VAC (vincristine, actinomycin D, cyclophosphamide); Purinethol (mercaptopurine), puromycin; PUVA (psoralens, ultraviolet A); PVA (prednisone, vincristine, asparaginase); PVB (Platinol, vinblastine, bleomycin); PVDA (prednisone, vincristine, daunorubicin, asparaginase); PVP (Platinol, VP-16); pyrazine diazohydroxide; pyrazoloacridine; pyridoglutethimide (Rogletimide); Quadramet; R-837; Radinyl (etanidazole); Ralox; recombinant interleukin-2; Recombinate; Renova (tretinoin emollient cream); Retin-A; retinoic acid; R-Frone (interferon beta, Serono); Rheumatrex (methotrexate sodium); RIDD (recombinant interleukin-2, dacarbazine, DDP); rIFN-A; rIFN-gamma; RIGS/ACT (adoptive cellular therapy); riboprine; ricin (blocked) conjugated murine monoclonal antibody; RIDD (recombinant interleukin-2, dacarbazine, DDP); RMP-7; ROAP (rubidazone, Oncovin, ara-C, prednisone); Roferon-A; Rogletimide (pyridoglutethimide); Rubrex (doxorubicin); rufocromomycin; St. Jude Research Children's Hospital protocol; Salagen; SAM (streptozocin, Adriamycin, methyl-CCNU); Sandimmune (cyclosporine); Sandostatin (octreotide); sargramostim (Prokine); SCAB (streptozocin, CCNU, Adriamycin, bleomycin); semustine (methyl-CCNU); Serono (interferon beta, R-Frone); Serratia marcescens extract; 7+3 protocol (cytarabine, daunorubicin); 7U85; Sialyl Tn-KLH; SIMAL-pilot (ara-C, hydrocortisone, mesna, prednisone, VP-16, leucovorin); SIMAL-second induction/maintenance (prednisone, L-asparaginase, daunomycin, VM-26,; methotrexate, ara-C, VP-16, leucovorin); SIMAL-bone marrow transplant (ara-C, methotrexate, prednisone); Simon capsule (cervical); simtrazene; single-chain antigen-binding proteins; SK (Sloan-Kettering) protocol; SMF (streptozocin, mitomycin-C, 5-fluorouracil); sparfosic acid; sparsomycin; Specifid (discontinued in 1994); Spherex; spirogermanium; spiromustine; spiroplatin; S-P-T "liquid" capsules; SR-2508; SSTN (octreotide or Sandostatin); ST1-RTA immunotoxin; STAMP protocol (Solid Tumor Autologous Bone Marrow Program); STEAM (streptonigrin, thioguanine, cyclophosphamide, actinomycin, mitomycin); STEPA (thiotepa); Sterecyt; Stilphostrol; streptonigrin; streptozocin; streptozotocin; SU10; sulofenur; super-CM regimen (cyclophosphamide, methotrexate, 5-fluorouracil); Suprefact (buserelin); suramin; suramin sodium; SWOG CMFVP (cyclophosphamide, methotrexate, 5-fluorouracil, vincristine, prednisone); synercid; T-2 protocol (dactinomycin, Adriamycin, vincristine, cyclophosphamide, radiation); T-10 protocol (methotrexate, calcium leucovorin rescue, Adriamycin, cisplatin, bleomycin,; cyclophosphamide, dactinomycin); T4N5 (T4 endolase V, liposome encapsulated); Tace (chlorotrianisene); TAD (6-thioguanine, ara-C, daunomycin); talisomycin; tallimustine; TAM (tamoxifen); tamoxifen; Tarabine PFS (cytarabine); Tasmar; tauromustine; Taxol (paclitaxel); Taxotere (docetaxel); TBC-CEA; TBI (total body irradiation); TC (6-thioguanine, cytarabine); T-CAP (Baker Antifol, cyclophosphamide, Adriamycin, Platinol); T-CAP III (triazinate, cyclophosphamide, Adriamycin, Platinol); TCN-P (triciribine phosphate); TEC (thiotepa, etoposide, carboplatin); teceleukin; teleleukin; teloxantrone; temozolomide; TEMP (tamoxifen, etoposide, mitoxantrone, Platinol); teniposide (VeeM-26); terephthalamidine; teroxirone; Teslac (testolactone); TESPA (thiotepa); Testamone (testosterone); Testaqua; Testex (testosterone); testolactone; Testone (testosterone); Testred (testosterone); Testrin (testosterone); Testroject (testosterone); TFN tumor factor necrosis; Theelin Aqueous (estrone); TheraCys (BCG vaccine); Theradex (AD-32); TheraSeed; thiamiprine; thioguanine (Lanvis or 6-TG or 6-thioguanine); 6-thioguanine (6-TG or thioguanine); 6-TG (6-thioguanine); thiosulfate; thiotepa (STEPA or TESPA or TTPA); thymidine; Thymosin Alpha-I; thymidine; Thyroid Strong; tiamiprine; tiazofurin; Tiazole; Tice BGC; T-inzole (tizofurin); tirapazamine; Tiratricol; tizofurine; TLC D-99 (liposome-encapsulated doxorubicin); TMCA; T-MOP (6-thioguanine, methotrexate, Oncovin, prednisone); TMP (trimethoprim); TMQ (trimetrexate); TMTX (trimetrexate); TMX (tamoxifen); TNF (tumor necrosis factor); TNP 470; TOAP (thioguanine, Oncovin, cytosine arabinoside, prednisone); Tomosar (menogaril); Tomudex (ICI D1694); topotecan (hycamptamine); toremifene; TPCH (thioguanine, procarbazine, CCNU, hydroxyurea); TPDC-FUHU; TPDCV (thioguanine, procarbazine, DBC, CCNU, vincristine); TRA (trans-retinoic acid or tretinoin); trans-retinoic acid (TRA); TRAP (thioguanine, rubidomycin, ara-C, prednisone); trestolone; tretinoin (all trans-retinoic acid); triacana (tiratricol); triciribine phosphate; triethylene melamine; trifluoperazine; trilostane; trimetrexate; triptorelin; TSPA (thiotepa); TTPA (thiotepa); tubulozole; tumor necrosis factor (TNF); UFT (Uracil+Ftorafur), uracil mustard; uramustine; uredepa; VA (vincristine, Adriamycin); VAAP (vincristine, asparaginase, Adriamycin, prednisone); VAB 1 (vinblastine, actinomycin D, bleomycin); VAB 2 (vinblastine, actinomycin D, bleomycin, cisplatin); VAB 3 (vinblastine, actinomycin D, bleomycin, cisplatin, chlorambucil, cyclophosphamide); VAB 4 (vinblastine, actinomycin D, bleomycin, cisplatin, cyclophosphamide); VAB 5 (vinblastine, actinomycin D, bleomycin, cisplatin, cyclophosphamide); VAB 6 (cyclophosphamide, dactinomycin, vinblastine, bleomycin, cisplatin); VABCD (vinblastine, Adriamycin, bleomycin, CCNU, DTIC); VAB-V (vinblastine, actinomycin D, bleomycin, cyclophosphamide, cisplatin); VABCD (vinblastine, Adriamycin, bleomycin, CCNU, DTIC); VAC (vincristine, actinomycin D, cyclophosphamide); VAC (vincristine, Adriamycin, cyclophosphamide), VACA (vincristine, actinomycin D, cyclophosphamide, Adriamycin); VACAD (vincristine, Adriamycin, cyclophosphamide, actinomycin D, dacarbazine); VACP (VePesid, Adriamycin, cyclophosphamide, Platinol); VAD (vincristine, Adriamycin, dexamethasone); VAD/V (vincristine, Adriamycin, dexamethasone, verapamil); VAFAC (vincristine, amethopterin, 5-fluorouracil, Adriamycin cyclophosphamide); VAI (vincristine, actinomycin D, ifosfamide); Valergen; VAM (VP-26-213, Adriamycin, methotrexate); VAMP (vincristine, Adriamycin, methotrexate, prednisone); VAMP (vincristine, amethopterin, 6-mercaptopurine, prednisone); VAP (vincristine, Adriamycin, procarbazine); VAP (vincristine, asparaginase, prednisone); VAP-II (vinblastine, actinomycin D, Platinol); vapreotide; VAT (vinblastine, Adriamycin, thiotepa); VAT (vincristine, ara-A, 6-thioguanine); VATD (vincristine, ara-C, 6-thioguanine, daunorubicin); VATH (vinblastine, Adriamycin, thiotepa, Halotestin); VAV (VP-26-213, Adriamycin, vincristine); VB (vinblastine, bleomycin); VBA (vincristine, BCNU, Adriamycin); VBAP (vincristine, BCNIJ, Adriamycin, prednisone); VBC (VePesid, BCNU, cyclophosphamide); VBC (vinblastine, bleomycin, cisplatin); VBD (vinblastine, bleomycin, DDP); VBL (vinblastine); VBM (vincristine, bleomycin, DDP); VBMCP (vincristine, BCNU, melphalan, cyclophosphamide, prednisone); VBMF (vincristine, bleomycin, methotrexate, 5-fluorouracil); VBP (vinblastine, bleomycin, Platinol); VC (VP-16, carboplatin); VCAP (vincristine, cyclophosphamide, Adriamycin, prednisone); VCAP-I (VP-16, cyclophosphamide, Adriamycin, Platinol); VCF (vincristine, cyclophosphamide, 5-fluorouracil); VCMP (vincristine, cyclophosphamide, melphalan, prednisone); VCP (vincristine, cyclophosphamide, prednisone); VCP-I (VP-16, cyclophosphamide, Platinol); VCR (vincristine); VDP (vincristine, daunorubicin, prednisone); VDS (vindesine); VeeM-26 (teniposide); VeIP (Velban, ifosfamide, Platinol); Velban (vinblastine sulfate); Velsar (vinblastine sulfate); VEMP (vincristine, Endoxan, 6-mercaptopurine, prednisone); VePesid (etoposide or VP-16); Vercyte (pipobroman); verteprofin; Vesanoid (all trans-retinoic acid); VIC (vinblastine, ifosfamide, CCNU); VIC (VP-16, ifosfamide, carboplatin); VIE (vincristine, ifosfamide, etoposide); vinblastine; vincaleukoblastine; Vincasar; vincristine; vindesine; vinepidine; vinglycinate; vinleurosine; vinorelbine (Navelbine); vinrosidine; vinzolidine; VIP (VePesid, ifosfamide, Platinol); VIP-B (VP-16, ifosfamide, Platinol, bleomycin); VLB (vinblastine); VLP (vincristine, L-asparaginase, prednisone); VM-26 (teniposide); VM-26PP (teniposide, procarbazine, prednisone); VMAD (vincristine, methotrexate, Adriamycin, actinomycin D); VMC (VP-16, methotrexate, citrovorum factor); VMCP (vincristine, melphalan, cyclophosphamide, prednisone); VMP (VePesid, mitoxantrone, prednimustine); VOCA (VP-16, Oncovin, cyclophosphamide, Adriamycin); VOCAP (VP-16-213, Oncovin, cyclophosphamide, Adriamycin, Platinol); vorozole; VP (vincristine, prednisone); VP-16; VP-16-213; VP-16+DDP (etoposide, cisplatin); VP-16-P (VP-16, Platinol); VP+A (vincristine, prednisone, asparaginase); VPAM (verapamil); VPB (vinblastine, Platinol, bleomycin); VPBCPr (vincristine, prednisone, vinblastine, chlorambucil, procarbazine); VPCA (vincristine, prednisone, cyclophosphamide, ara-C); VPCMF (vincristine, prednisone, cyclophosphamide, methotrexate, 5-fluorouracil); VP+L-asparaginase (vincristine, prednisone, L-asparaginase); VPP (VePesid, Platinol); VPVCP (vincristine, prednisone, vinblastine, chlorambucil, procarbazine); Vumon (teniposide); Wayne State protocol (cisplatin, 5-fluorouracil); Wehgen (estrone); Wellcovorin (leucovorin); Xomazyme-791 (anti-TAP-72 immunotoxin); Xomazyme-H65 (CD5-T-lymphocyte immunotoxin); Zadaxin (thymosin alpha 1); Zanosar (streptozocin); zanoterone; ZD 0490; ZD 1694; ZDV (zidovudine); ZDX (goserelin); Zeneca 182,780; zidovudine; zinostatin; Zofran (ondansetron HCl); Zoladex (goserelin acetate), and implant; zolimomab aritox (anti-T lymphocyte monoclonal antibody); zorubicin- zorubicin HCl; Zyloprim.

In addition, the following agents, and modifications of the following agents, including, for example, analogs, derivatives, fragments, active moieties, and the like, may be screened using methods and systems of the present invention: Acetaminophen; Acyclovir; Albuterol (oral); Albuterol, inhaled; Allopurinol; Alprazolam; Amantadine; Amikacin; Amitriptyline; Amlodipine; Amoxicillin; Amoxicillin/clavulanate; Ampicillin; Aspirin; Atenolol; Azithromycin; Aztreonam; Beclomethasone; Benzonatate; Benztropine; Bethanechol; Bumetanide; Captopril; Carbamazepine; Cefaclor; Cefadroxil; Cefamandole; Cefazolin; Cefxime; Cefotaxime; Cefoxitin; Ceftazidime; Ceftizoxime; Ceftriaxone; Cephalexin; Chlorpromazine; Cimetidine; Ciprofloxacin; Cisapride; Clarithromycin; Clindamycin; Clonidine; Codeine; Colchicine; Desipramine; Dexamethasone; Dextromethorphan; Diazepam; Dicloxacillin; Digoxin; Digoxin Fab; Diltiazem; Diphenhydramine; Dipyridamole; Divalproex; Doxycycline; Droperidol; Enalapril; Enoxaparin; Epinephrine; Epinephrine in; sesame oil; (Sus-Phrine); Erythromycin; Estrogen,; conjugated; Ethacrynic acid; Ethosuximide; Famciclovir; Famotidine; Felbamate; Fluconazole; Flumazenil; Fluoxetine; Folic acid; Furosemide; Gabapentin; Gentamicin; Glipizide; Glucagon; Glyburide; Griseofulvin; Haloperidol; Heparin; Hydrochlorothiazide; Hydrocortisone; Hydroxyzine; Ibuprofen; Imipramine; Indomethacin; Isosorbide dinitrate; Ketorolac; Labetalol; Lactulose; Levothyroxine; Lidocaine; Lorazepam; Lovastatin; Magnesium oxide; Magnesium sulfate; Mebendazole; Meclizine; Medroxyprogesterone; Mefenamic acid; Meperidine; Methicillin; Methylergonovine; Methylphenidate; Methylprednisolone; Metoclopramide; Metolazone (Diulo; and Zaroxolyn only); Metoprolol; Metronidazole; Midazolam; Morphine sulfate; Nafcillin; Naloxone; Naproxen; Nifedipine; Nitroglycerin; Norfloxacin; Nortriptyline; Nystatin; Ofloxacin; Omeprazole; Ondansetron; Oxybutynin; Oxycodone; Pediazole; Penicillin G; Pentamadine; Perphenazine; Phenobarbital; Phenytoin; Piperacillin; Prednisolone; Prednisone; Procainamide; Prochlorperazine; Promethazine; Propranolol; Pseudoephedrine; Pyrantel pamoate; Ranitidine; Rifampin; Rimantadine; Salsalate; Sertraline; Simvastatin; Spironolactone; Succinylcholine; Sucralfate; Sumatriptan; Terazosin; Terbutaline; Tetracycline; Thiabendazole; Thiamine; Thioridazine; Ticarcillin; Ticlopidine; Tobramycin; Trazodone; Triazolam; Trimethoprim/sulfamethoxaz- ole; Valproic acid; Vecuronium; Venlafaxine; Verapamil; Warfarin; Zidovudine.

As can be readily appreciated, the above-noted compounds are only examples of suitable compounds and screening combinations, and other compounds or similar classes of compounds are also suitable. The activity and efficacy with respect to the megalin-mediated uptake of radiotherapeutics and/or radiodiagnostics into cells of the kidney of such compounds, and various combinations and dosages of such compounds and combinations may be assessed using the methods of the present invention.

In an important embodiment of the invention, the binding of a cofactor protein, like cubilin, and megalin could also be used to monitor the binding of a substance to one of the binding partners. The substance, which can be a small molecule such as a ligand to the megalin receptor, will lead to a change in the allosteric conformation of the binding protein which in consequence leads to a loss of the interaction of the two proteins. Using this effect of ligand-dependent protein-protein interactions one can design assays where the protein-protein interaction serves as a surrogate read-out for the binding of one of the proteins to small molecule ligand. Any assay method which is useful for the measurement of protein-protein interactions can be used for such an indirect assay. Such assay methods are well known in the art and include the methods described in this patent under "Cell free assays" and "Cell based assays". In a preferred embodiment, this assay will measure the binding of substances to megalin receptor, resulting in an effect on the interaction of megalin with the cofactor.

In other embodiments of the invention, assays are included measuring the activity of di- or multimeric complexes of megalin (or parts thereof) and other proteins, such as cofactors, like cubilin. Further included are assays aiming at the identification of compounds which specifically influence only the monomeric, homodimeric or homomultimeric form of megalin, or influencing only multimeric forms of the megalin receptor. Such assays include measuring the effect of a compound on the megalin receptor in the absence of a binding partner, and measuring the effect of a compound on the megalin receptor in the presence of a binding partner, such as a radiotherapeutic and/or radiodiagnostic. One skilled in the art will find numerous more assays which are equally covered by the invention.

Methods and systems of the present invention are well-suited for high-throughput screening of libraries of compounds, and combinations of compounds, to identify candidate compounds and combinations having desired therapeutic or diagnostic properties, i.e. an influence of the megalin mediated uptake of radiotherapeutics and/or radiodiagnostics into cells of the kidney.

In one approach, well established agents, such as therapeutic agents effective in treating various conditions are identified (for example as those mentioned above); modifications to the therapeutic agent(s) are made, for example, analogue and derivative compounds are identified and synthesised; and the modified agents are then screened for efficacy under various conditions using screening techniques of the present invention. In a similar fashion, combinations of therapeutic compounds, combinations of a therapeutic compound with candidate agents, and combinations of candidate agents may be screened for efficacy under certain conditions using screening techniques of the present invention.

According to yet another aspect, the screening methods of the present invention may be accomplished by performing different steps at remote locations. Thus, in vitro and in vivo screening data may be collected at one location, such as in a testing laboratory. The data relating to the uptake properties of cell sample populations at various test stages may be collected and transmitted to a central data processor for data processing and analysis, and screening results may subsequently be transmitted to the testing laboratory or to one or more additional sites. Network and communications technologies are readily available for transmitting the required data and implementation of remote activities, involving data processing and/or analysis, may provide expedited screening results and more economical screening.

In another embodiment of the present invention, the recombinant form of the megalin receptor and/or cubilin is used in screening assays in order to identify chemical compounds which directly influence megalin uptake function and which could in turn be useful in the treatment of proliferative diseases. Such compounds can be derivatives of potential modulators of the megalin-mediated uptake of radiotherapeutics and/or radiodiagnostics into cells of the kidney, which are, for example, selected from the group of compounds comprising modified cubilin, vitamin-binding proteins, carrier proteins, lipoproteins, apolipoproteins (e.g. apo E, and apo B, clusterin), LPO-antagonists (e.g., probucol), RAP and derivatives thereof, Ca²⁺, Ca²⁺- scavengers, lipids, cytochrome C, antimegalin IgG, anti-cubilin IgG, anti-poly alpha2,8 KDN antibody, aminoglycosides (e.g. gentamicin), hormones and precursors thereof, drugs and toxins, enzymes and inhibitors thereof, and immune- and stress-response related proteins, maleate, dextran-based conjugates, aristolochic acid, cadmium chloride, and L-carnithine.

In the case of proteinous candidate compounds those skilled in the art will appreciate that conservative substitutions of amino acids can be made without significantly diminishing a protein's affinity for interacting proteins, DNA binding sites, cofactor modulators, e.g. small molecular hydrophobic compounds, or RNA. Other substitutions may be made that increase the proteins' affinity for megalin. Making and identifying such proteins is a routine matter given the teachings herein, and can be accomplished, for example, by altering the nucleic acid sequence encoding the protein, inserting it into a vector, transforming a cell, expressing the nucleic acid sequence, and measuring the binding affinity of the resulting protein to megalin.

Reporter constructs are used to indirectly monitor the effect of an agent on the effect of an agent on the expression of the megalin gene. A number of reporter constructs in which all or a portion of the upstream regulatory region of the megalin receptor gene or is operably linked to a sequence encoding a detectable protein are useful in the methods of the invention. In this cases, all or part of the upstream regulatory region of a naturally-occurring megalin gene can be operably linked to the sequence encoding a detectable protein in order to provide a suitable reporter construct.

The expression of recombinant polypeptides within the scope of this invention can be performed in vitro. This may occur by a two step procedure, thereby producing first mRNA by in vitro transcription of an apt polynucleotide construct followed by in vitro translation with convenient cellular extracts. These cellular extracts may be reticulocyte lysates but are not limited to this type. In vitro transcription may be performed by T7 or SP6 DNA polymerase or any other RNA polymerase which can recognise per se or with the help of accessory factors the promoter sequence contained in the recombinant DNA construct of choice. Alternatively one of the recently made available one step coupled transcription/translation systems may be used for in vitro translation of DNA coding for the polypeptides of this invention. One illustrative but not limiting example for such a system is the TNT® T7 Quick System by Promega.

The effect of a given agent on the internalisation of radiotherapeutics and/or radiodiagnostics can be measured either directly or indirectly. Direct measurement of binding and/or uptake involves monitoring radioactivity present in cells. Indirect measurement of binding and/or uptake involves monitoring the expression of genes encoding megalin in kidney cells. In general, the expression of these genes is monitored indirectly by monitoring the expression of a reporter construct in which all or part of the upstream regulatory region of the gene of interest is operably linked to a sequence encoding a readily detectable protein.

As noted above, a modulator can also be identified based on its effect on the expression of a megalin gene in particular cell types, in this case kidney cells. The effect of a modulator on the expression of such a naturally-occurring or recombinant megalin gene can be measured directly or indirectly. Direct measurement involves monitoring the expression of the megalin gene itself. One can measure the expression of a genomic copy of the gene or one can measure the expression of a non-endogenous copy of the gene, e.g., a copy introduced by transfection. Generally, expression of the megalin gene is monitored by measuring mRNA production. Alternatively, protein production can be measured. In many cases it is preferable to indirectly monitor the expression of a megalin gene by monitoring the expression of a reporter construct in which all or part of the upstream regulatory region of the megalin gene of interest is operably linked to a sequence encoding a readily detectable protein.

A luciferase encoding reporter construct in which luciferase expression is under the control of a megalin receptor promoter element is an example of such a detectable protein.

Agents which inhibit or reduce the expression of the megalin gene in kidney cells are candidate uptake-modulating agents. Generally, reporter constructs can be used to examine the effect of a given agent on the expression of such gene. Suitable reporter constructs can be created by placing a sequence encoding a readily detectable protein, e.g., luciferase, under the control of all or part the upstream transcription control region of the megalin gene.

To set up an in vitro assay, a selected megalin modulator responsive reporter is introduced into a kidney cell line. The selected megalin modulator responsive reporter may also be introduced into a cell line which does not express the megalin receptor (null cells). Suitable null cell lines include COS cells, HeLa cells, and HEK 293 or other suitable, e.g. human, cells. While the reporter can be introduced into the cells various cell lines by transient transfection, it is preferable that the cells be stably transfected with the reporter construct. Generally, human, murine, and/or rat cells can be used in the methods of the invention. In addition, the reporter constructs can employ the upstream regulatory sequences of human, murine, or rat megalin genes.

Moreover, the cell line and the gene used as the source of upstream regulatory regions for the reporter construct do not have to be of the same species. Thus, a reporter in which the upstream control region of murine megalin gene is operably linked to sequence encoding luciferase can be used in the screening methods of the invention.

The agents (i.e. compounds) of the present invention, may be any composition of matter provided that it has the ability to bind to the megalin receptor. Suitable agents exhibiting these properties include, but are not limited to, peptides, antibodies, carbohydrates, nucleic acids, vitamins, pharmaceutical agents, and the like, including derivatives thereof.

The agents of the present invention may be identified and/or prepared according to any of the methods and techniques known to those skilled in the art. These agents, particularly peptide agents and antibody agents, may occur or be produced as monomer, dimers, trimers, tetrameres or multimers. Such multimers can be prepared using enzymatic or chemical treatment of the native receptor molecules or be prepared using recombinant techniques. Preferably, the agents of the present invention are selected and screened at random or rationally selected or designed using protein modelling techniques.

For random screening, candidate agents are selected at random and assayed for their ability to reduce the amount or rate of binding and uptake of radiopharmaceuticals and/or radiodiagnostics into cells of the kidney. Any of the suitable methods and techniques known to those skilled in the art may be employed to assay candidate agents.

The agent can be selected based on a rational selection or design. Any of the suitable methods and techniques known to those skilled in the art may be employed for rational selection or design. For example, one skilled in the art can readily adapt currently available procedures to generate antibodies, peptides, pharmaceutical agents and the like capable of binding to a specific peptide sequence of the megalin receptor.

Illustrative examples of such available procedures are described, for example, in Hurby et al., (1992); Kaspczak et al., (1989); and Harlow (1990).

As noted above, the modulators of the present invention include those agents which bind directly to the megalin receptor. Additionally, the agents of the present invention bind to the megalin receptor in interfering proximity with the binding site of radiopharmaceuticals and/or radiotherapeutics or bind to the megalin receptor in such a manner so as to conformationally alter the binding site of radiopharmaceuticals and/or radiotherapeutics. Suitable agents can therefore be first identified by their ability to bind the megalin receptor and then by their ability to reduce the amount or rate at which the megalin mediated uptake of radiopharmaceuticals and/or radiotherapeutics into the cells of the kidney takes place. Illustrative examples of agents of the present invention include, but are not limited to: soluble fragments of cubilin, vitamin-binding proteins, carrier proteins, lipoproteins, apolipoproteins (e.g. apo E, and apo B, clusterin), LPO-antagonists (e.g., probucol), RAP and derivatives thereof, Ca²⁺, Ca²⁺-scavengers, lipids, cytochrome C, antimegalin IgG, anti-cubilin IgG, anti-poly alpha2,8 KDN antibody, aminoglycosides (e.g. gentamicin), hormones and precursors thereof, drugs and toxins, enzymes and inhibitors thereof, and immune- and stress-response related proteins, maleate, dextran-based conjugates, aristolochic acid, cadmium chloride, and L-carnithine.

In the following, the use of the receptor associated protein (RAP) and cubilin in the methods of the present invention is described as a non-limiting example for proteinous megalin-binding agents. The preferred agents in this example are based on and derived from the amino acid sequence of the receptor associated protein (RAP) or cubilin. Especially preferred types of agents are isolated RAP or cubilin or fragments thereof, such as a soluble fragment of RAP which contains the megalin binding site. Such agents could act as competitive inhibitors of the binding of radiopharmaceuticals and/or radiodiagnostics to the receptor in vitro as well as in vivo.

The preferred fragments of RAP or cubilin are soluble under physiological conditions and can pass the filtration barrier of the glomerulus. The C-terminus of these polypeptides can be shortened as desired, provided that the binding capacity for the megalin particle remains intact. The preferred amino acid sequence of RAP corresponds to the human protein. Suitable RAP or cubilin sequences can also be derived from the amino acid sequence of RAP or cubilin isolated from other mammals or amphibia.

The proteinous agents, like RAP, cubilin, or a fragment thereof, may be produced using any of the methods and techniques known to those skilled in the art. For example, RAP can be purified from a source which naturally expresses the protein, can be isolated from a recombinant host which has been altered to express RAP or fragment thereof, or can be synthesised using protein synthesis techniques known in the art. The skilled artisan can readily adapt a variety of techniques in order to obtain peptide agents which contain the LRP binding site found on RAP, cubilin or the other compounds mentioned above.

The isolation of native RAP proteins is known, as described, for example, in Ashcom et al., (1990) and Jensen et al., (1989). In order to generate fragments of RAP or cubilin which contain the megalin (LRP) binding site, isolated native protein may be converted by enzymatic and/or chemical cleavage to generate fragments of the whole protein, for example by reacting cell lines which express an RAP with an enzyme such as papain or trypsin or a chemical such as cyanogen bromide. Proteolytically active enzymes or chemicals are preferably selected in order to release the extracellular receptor region. Fragments which contain the megalin LRP binding site, especially fragments which are soluble under physiological conditions, can then be isolated using known methods.

Alternatively, fragments of the above proteinous agents, like RAP or a fragment of RAP may be expressed in a recombinant bacteria, as described, for example, in Williams et al., (1992) and Wurshawsky et al., (1994).

Other agents of the present invention include compositions that bind to megalin in interfering proximity to the binding site of radiopharmaceuticals and/or radiodiagnostics on megalin. Illustrative examples of these agents of the present invention include, but are not limited to, antibodies which bind to the binding site or the proximity of said binding site of radiopharmaceuticals and/or radiodiagnostics on megalin. Although the following description is primarily related to antibodies in the form as candidate agents for a modulation of the megalin-mediated uptake of radiopharmaceutics and/or radiotherapeutics into cells of the kidney, many aspects of the following description will also be relevant for radiopharmaceutics and/or radiotherapeutics in their form of therapeutical and/or diagnostic antibodies which recognise tumours based e.g. on the recognition of tumour-associated antigens. The person of skill in the art will be able to adapt the description below in such a manner to read on antibody-based radiopharmaceutics and/or radiotherapeutics.

Preferred agents include antibodies and antibody fragments which are capable of binding to a residue found on megalin and consequently act as a competitive inhibitor for the binding of radiopharmaceuticals and/or radiodiagnostics. The most preferred antibodies or antibody fragments as agents of the present invention bind to a radiopharmaceutical and/or radiodiagnostic binding-responsible specific epitope in megalin. Antibodies, or other agents such as antisense peptides, which bind to epitopes within this sequence could (also) reduce the amount or rate of the binding of radiopharmaceutical and/or radiodiagnostics to megalin.

The antibodies as agents of the present invention include polyclonal and monoclonal antibodies, as well as antibody fragments and derivatives that contain the relevant antigen binding domain of the antibodies. Such antibodies or antibody fragments are preferably used in the diagnostic and therapeutic embodiments of the present invention.

Suitable monoclonal and polyclonal antibodies may be prepared by any of the methods and techniques well known in the art, such as described in, for example, A. M. Campbell, Monoclonal Antibody Technology: Laboratory Techniques in Biochemistry and Molecular Biology, Elsevier Science Publishers, Amsterdam, The Netherlands (1984) and Harlow; Antibodies, Cold Spring Harbor Press, NY (1989). For example, an antibody capable of binding to a domain of megalin can be generated by immunising an animal with a polypeptide whose sequence is encoded by that megalin domain.

Any animal (mouse, rabbit, etc.) which is known to produce antibodies can be utilised to produce antibodies with the desired specificity and suitable methods for immunisation of these animals are well known in the art, including, for example, subcutaneous or intraperitoneal injection of the polypeptide. One skilled in the art will recognise that the amount of polypeptide used for immunisation will vary based on a number of factors, including the animal which is immunised, the antigenicity of the polypeptide selected, and the site of injection.

The polypeptides used as an immunogen may be modified as appropriate or administered in an adjuvant in order to increase the peptide antigenicity. Suitable methods increasing antigenicity are well known in the art, and include, for example, coupling the antigen with a heterologous protein (such as globulin or beta-galactosidase) or through the inclusion of an adjuvant during immunisation.

A preferred method of generating monoclonal antibodies comprises removing spleen cells from the immunised animals, fusing these cells with myeloma cells, such as SP2/0-Ag14 myeloma cells, and allowing them to become monoclonal antibody-producing hybridoma cells. Any one of a number of methods well known in the art may be used to identify the hybridoma cell which produces an antibody with the desired characteristics. These include screening the hybridomas with an ELISA assay, western blot analysis, or radioimmunoassay (Lutz et al., (1988); Kishimoto et al., (1990)). Hybridomas secreting the desired antibodies are cloned and the class and subclass of the secreted antibodies are determined using procedures known in the art (Campbell, A. M., Monoclonal Antibody Technology: Laboratory Techniques in Biochemistry and Molecular Biology, Elsevier Science Publishers, Amsterdam, The Netherlands (1984)).

For polyclonal antibodies, antibody-containing antisera is preferably isolated from the immunised animal and is screened for the presence of antibodies with the desired specificity using one of the above-described procedures.

The so produced monoclonal antibodies are capable of wholly or partially neutralising the activity of the megalin and/or cubilin polypeptides by cross-recognition or by specifically binding to one of the said polypeptides. These monoclonal antibodies can in addition be used for qualitative and/or quantitative measurement or for purification of the polypeptides in methods according to the invention.

The present invention therefore also includes test systems and/or so-called kits, which contain the (optionally monoclonal) antibodies herein described, together with additional compounds that are required for screening, diagnostic and/or therapeutical purposes. In one embodiment, the kit will comprise recombinant megalin, recombinant cubilin, suitable radiopharmaceutics and/or radiotherapeutics and optionally additional auxiliary compounds, e.g. a megalin antibody, a cubilin antibody, and suitable dyes and reagents.

The antibodies may be used as an isolated whole antibody, or can be used as a source for generating antibody fragments which contain the antigen binding site of the antibody. Examples of such antibody fragments include, but are not limited to the Fv, the F(ab), the F(ab)₂, fragment, as well as single chain antibodies. Various methods known in the art can be used to generate such fragments without undue experimentation. Recombinant techniques are preferred for generating large quantities of antibodies, antibody fragments and single chain antibodies, as described, for example, in Pluckthum, (1992); Carter et al., (1992); and Mullinax et al., (1992). In addition, recombinant techniques may be used to generate heterobifunctional antibodies.

In general, recombinant production of antibodies, antibody fragments or derivatives thereof, uses mRNA encoding an antibody which is isolated from hybridoma cells that produce the desired antibody. This mRNA is then used as a source for generating a cDNA molecule which encodes the antibody, or a fragment thereof. Once obtained, the cDNA may be amplified and expressed according to known methods in a variety of eukaryotic and prokaryotic hosts.

The present invention further employs derivatives of antibodies (antibody derivatives). As used herein, an "antibody derivatives" contain an antibody, or a fragment thereof, as well as an additional moiety which is not normally a part of the antibody. Such moieties may improve the solubility, absorption, biological half-life, etc., of the antibody, decrease the toxicity of the antibody, eliminate or attenuate any undesirable side effect of the antibody, or serve as a detectable marker of the presence of the antibody. Moieties capable of mediating such effects are well known in the art.

Detectably labelled antibodies constitute a special class of the antibody derivatives as agents of the present invention. An antibody is said to be "detectably labelled" if the antibody, or fragment thereof, is attached to a molecule which is capable of identification, visualisation, or localisation using known methods. Suitable detectable labels include radioisotopic labels, enzyme labels, non-radioactive isotopic labels, fluorescent labels, toxin labels, affinity labels, chemiluminescent labels and nuclear magnetic resonance contrast agents.

Illustrative examples of suitable enzyme labels include, but are not limited to, malate dehydrogenase, staphylococcal nuclease, delta-5-steroid isomerase, yeast-alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, and acetylcholine esterase.

Examples of suitable radioisotopic labels include, but are not limited to, ³H, ¹¹¹In, ¹²⁵I, ¹³¹I, ³²P, ³⁵S, ¹⁴C, ⁵¹Cr, ⁵⁷To , ⁵⁸Co, ⁵⁹Fe , ⁷⁵Se, ¹⁵²Eu, ⁹⁰Y, ⁶⁷Cu, ²¹⁷Ci, ²¹¹At, ²¹²Pb, ⁴⁷Sc, ¹⁰⁹Pd, etc. ¹¹¹In is a preferred isotope where *in vivo* imaging is used. In addition, this radionucide has a more favourable gamma emission energy for imaging (Perkins et al., (1985); Carasquillo et al., (1987)). For example, ¹¹¹In coupled to monoclonal antibodies with 1-(p-isothiocyanatobenzyl)-DPTA has shown little uptake in non-tumorous tissues, particularly the liver, and therefore enhances specificity of tumour localisation (Esteban et al., (1987)).

Illustrative examples of suitable non-radioactive isotopic labels include, but are not limited to, ¹⁵⁷Gd, ⁵⁵Mn, ¹⁶²Dy, ⁵²Tr, and ⁵⁶Fe.

Illustrative examples of suitable fluorescent labels include, but are not limited to, an ¹⁵²Eu label, a fluorescein label, an isothiocyanate label, a rhodamine label, a phycoerythrin label, a phycocyanin label, an allophycocyanin label, an o-phthaldehyde label, and a fluorescamine label. Illustrative examples of suitable toxin labels include diphtheria toxin, ricin, and cholera toxin. Illustrative examples of chemiluminescent labels include a luminal label, an isoluminal label, an aromatic acridinium ester label, an imidazole label, an acridinium salt label, an oxalate ester label, a luciferin label, a luciferase label, and an aequorin label.

Illustrative examples of nuclear magnetic resonance contrasting agents include paramagnetic heavy metal nuclei such as Gd, Mn, and Fe.

Typical techniques for binding the above-described labels to antibodies are provided by Kennedy et al. (1976), and Schurs et al. (1977), which are both herein incorporated in their entirety by reference. Coupling techniques mentioned in the latter reference include the glutaraldehyde method, the periodate method, the dimaleimide method, and the m-maleimidobenzyl-N-hydroxy-succinimide ester method.

The present invention additionally includes the use of humanised forms of these antibodies. Humanised forms of the antibodies may be produced, for example, by replacing an immunogenic portion of an antibody with a corresponding, non-immunogenic portion (chimeric antibodies) (Better, et al., Science 240:1041-1043(1988)). Alternatively, suitable "humanised" antibodies can be produced by CDR or CEA grafting/substitution as described, for example, in Jones, et al., (1986); Verhoeyan et al., (1988); and Beidler, et al., (1988).

Another type of the agents of the present invention are peptide agents which are classified as antisense-peptide sequences. Antisense-peptide sequences are short peptides which are specifically designed to bind to a particular amino acid sequence. In general, such antisense peptide agents may be generated using methods known in the art, such as those described, for example, in Hurby et al, "Application of Synthetic Peptides: Antisense Peptides," in Synthetic Peptides, A User's Guide, W. H. Freeman, NY, pp. 289-307 (1992) and Kaspczak et al., Biochemistry 28:9230-8 (1989).

An additional class of the agents of the invention are natural ligands of megalin. As used herein, a natural ligand of megalin is defined as any substance which binds to megalin, such as soluble fragments of cubilin and/or RAP, containing the megalin binding site. Such soluble fragments may be prepared by any suitable method known to those skilled in the art, such as the method of Davis et al., (1987), which involves deletions. Moreover, soluble forms of the receptor may be formed by inserting a stop codon in front of the region of DNA encoding the cytoplasmic or transmembrane domain(s) (Yokade et al., (1992)).

The agents of the present invention may be used in vitro and/or in vivo to study radiopharmaceutic and/or radiodiagnostic attachment and to reduce the rate of uptake of these compounds into the cells of the kidney. In addition, the agents of the present invention may be used in qualitative, quantitative and preparative assays and purification procedures to isolate, identify and facilitate the purification of megalin and/or cubilin.

In one embodiment of the present invention, the recombinant host cells used in the context of the present invention (i.e. for screening and/or expression purposes) are prokaryotic host cells. In addition to prokaryotes, eukaryotic microbes, such as yeast may also be used illustrative examples for suitable cells and organisms for expression of recombinant polypeptides are belonging to but not limited to the following examples: Insect cells, such as *Drosophila* Sf21, SF9 cells or others, Expression strains of Escherichia coli, such as XL1 blue, BRL21, M15, *Saccharomyces cerevisiae, Schizosaccharomyces pombe, Hansenula polymorpha* and *Pichia pastoris* strains, immortalised mammalian cell lines such as AtT-20, VERO and HeLa cells, Chinese hamster ovary (CHO) cell lines, and W138, BHK, COSM6, COS-7, 293 and MDCK cells, BHK-21 cells, Att 20HeLa cells, HeK 294, T47 D cells and others. As discussed above, the methods of the invention identify an agent as a modulator of the megalin receptor mediated uptake by examining the effect of the agent on the uptake of radioactivity into kidney cells and/or the gene expression in kidney cells. Accordingly, the methods of the invention can employ a variety of kidney cell types. Other cell lines useful in the method of the invention include, but are not limited to the following:

| ***Cell line*** | ***ATCC number*** |
|---|---|
| LLC-PK1 | CL-101 |
| LLC-RK1 | CC1-106 |
| A6 | CCL-102 |
| OK | CRL-1840 |
| MDCK | CCL-34 |
| HK-2 | CRL-2190 |
| HaK | CCL-15 |
| LLC-MK2 | CCL-7 |

For *in vivo* use, the agents/modulators of the present invention that have been identified may be provided to a patient as a means of reducing the amount or rate of binding and/or uptake of radiotherapeutics and/or radiodiagnostics to megalin. Furthermore, the compounds can be used in order to reduce the amount of intracellularly transported megalin.

The present invention therefore provides pharmaceutical compositions comprising an agent of the invention. These pharmaceutical compositions may be administered orally, rectally, parenterally, intracistemally, intravaginally, intraperitoneally, topically (as by powders, ointments, drops or transdermal patch), bucally, or as an oral or nasal spray.

As used herein, "pharmaceutically acceptable carrier" is intended to mean a non-toxic solid, semisolid or liquid filler, diluent, encapsulating material or formulation auxiliary of any type. The term "parenteral" as used herein refers to modes of administration which include intravenous, intramuscular, intraperitoneal, intrastemal, subcutaneous and intraarticular injection and infusion. One of ordinary skill will recognise that the choice of a particular mode of administration can be made empirically based upon considerations such as the particular disease state being treated; the type and degree of the response to be achieved; the specific agent or composition employed; the age, body weight, general health, sex and diet of the patient; the time of administration and rate of excretion of the agent or composition; the duration of the treatment; drugs (such as a chemotherapeutic agent as mentioned above) used in combination or coincidental with the specific composition; and like factors well known in the medical arts.

Pharmaceutical compositions of the present invention for parenteral injection may comprise pharmaceutically acceptable sterile aqueous or non-aqueous solutions, dispersions, suspensions or emulsions as well as sterile powders for reconstitution into sterile injectable solutions or dispersions just prior to use. Illustrative examples of suitable aqueous and non-aqueous carriers, diluents, solvents or vehicles include, but are not limited to, water, ethanol, polyols (such as glycerol, propylene glycol, polyethylene glycol, and the like), carboxymethylceuulose and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

The compositions of the present invention may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption such as aluminium monostearate and gelatine.

In some cases, in order to prolong the effect of the therapeutic agent or inhibitor, it is desirable to slow the absorption from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilised, for example, by filtration through a bacterial-retaining filter, or by incorporating sterilising agents in the form of sterile solid compositions which can be dissolved or dispersed in sterile water or other sterile injectable medium just prior to use.

Solid dosage forms for oral administration include, but are not limited to, capsules, tablets, pills, powders, and granules. In such solid dosage forms, the active compounds are preferably mixed with at least one pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatine, polyvinylpyrrolidone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and I) lubricants such as talc, calcium stearate, magnesium stearate, solid polyethylene glycols, sodium lauryl sulfate, and mixtures thereof. In the case of capsules, tablets and pills, the dosage form may also comprise buffering agents as appropriate.

Solid compositions of a similar type may also be employed as fillers in soft and hard filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Illustrative examples of embedding compositions which can be used include polymeric substances and waxes.

The active agents of the invention can also be in microencapsulated form, if appropriate, with one or more of the above-mentioned excipients. Liquid dosage forms for oral administration include, but are not limited to, pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfiryl alcohol, polyethylene glycols and fatty acid esters of sorbitan, and mixtures thereof.

Besides inert diluents, the oral compositions may also contain adjuvants such as wetting agents, emulsifying and suspending agents, sweetening, flavouring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminium metahydroxide, bentonite, agar--agar, and tragacanth, and mixtures thereof.

The agent or inhibitor can also be administered in the form of liposomes. As is known to those skilled in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolisable lipid capable of forming liposomes can be used. The present compositions in liposome form can contain, in addition to the agent or inhibitor, stabilisers, preservatives, excipients, and the like. Preferred lipids are phospholipids and phosphatidyl cholines (lecithins), both natural and synthetic. Methods to form liposomes are known in the art. See, e.g., Prescott, ed., (1976).

The agents of the present invention can be formulated according to known methods to prepare pharmaceutically acceptable compositions, whereby these materials, or their functional derivatives, are combined in a mixture with a pharmaceutically acceptable carrier vehicle. Suitable vehicles and their formulation, inclusive of other human proteins, e.g., human serum albumin, are well known in the art. In order to form a pharmaceutically acceptable composition suitable for effective administration, such compositions will contain an effective amount of one or more agents of the present invention.

Additional pharmaceutical methods may be employed to control the duration of action. Control release preparations may be achieved through the use of polymers to complex or absorb the therapeutic agents of the invention. The controlled delivery may be exercised by selecting appropriate macromolecules (such as polyesters, polyamino acids, polyvinyl, pyrrolidone, ethylenevinylacetate, methylcellulose, carboxymethylcellulose, or protamine sulfate) and methods of incorporation in order to control release. Another possible method to control the duration of action by controlled release preparations is to incorporate antibodies into particles of a polymeric material such as polyesters, polyamino acids, hydrogels, poly(lactic acid) or ethylene vinyl acetate copolymers. Alternatively, instead of incorporating these agents into polymeric particles, it is possible to entrap these materials in microcapsules prepared, for example, by coacervation techniques or by interfacial polymerisation, for example, hydroxymethylcellulose or gelatine-microcapsules and poly(methylmethacylate) microcapsules, respectively, or in colloidal drug delivery systems, for example, liposomes, albumin microspheres, microemulsions, nanoparticles, and nanocapsules or in macroemulsions.

The pharmaceutical formulations of the present invention are prepared, for example, by admixing the active agent with solvents and/or carriers, optionally using emulsifiers and/or dispersants, whilst if water is used as the diluent, organic solvents may be used as solubilising agents or auxiliary solvents. As described above, the excipients used include, for example, water, pharmaceutically acceptable organic solvents such as paraffins, vegetable oils, mono- or polyfunctional alcohols, carriers such as natural mineral powders, synthetic mineral powders, sugars, emulsifiers and lubricants.

One of ordinary skill will appreciate that effective amounts of the inventive therapeutic agents can be determined empirically and may be employed in pure form or, where such forms exist, in pharmaceutically acceptable salt, ester or prodrug form. The agonist or antagonist my be administered in compositions in combination with one or more pharmaceutically acceptable excipients. It will be understood that, when administered to a human patient, the total daily usage of the agents and compositions of the present invention will be decided by the attending physician within the scope of sound medical judgement. The specific therapeutically effective dose level for any particular patient will depend upon a variety of factors including the type and degree of the response to be achieved; the specific agent or composition employed; the age, body weight, general health, sex and diet of the patient; the time of administration, route of administration, and rate of excretion of the agent or composition; the duration of the treatment; drugs (such as a chemotherapeutic agent as described above) used in combination or coincidental with the specific composition; and like factors well known in the medical arts.

Techniques of dosage determination are well known in the art for antibody and peptide agents. In general, it is desirable to provide a patient with a dosage of antibody or peptide agent in the range of from about 1 pg/kg to 10 mg/kg (body weight of patient). The therapeutically effective dose can be lowered if the agent of the present invention is additionally administered with another compound. As used herein, one compound is said to be additionally administered with a second compound when the administration of the two compounds is in such proximity of time that both compounds can be detected at the same time in the patient's serum.

For example, satisfactory results are obtained by oral administration of therapeutic dosages on the order of from 0.05 to 10 mg/kg/day, preferably 0.1 to 7.5 mg/kg/day, more preferably 0.1 to 2 mg/kg/day, administered once or, in divided doses, 2 to 4 times per day. On administration parenterally, for example by i.v. drip or infusion, dosages on the order of from 0.01 to 5 mg/kg/day, preferably 0.05 to 1.0 mg/kg/day and more preferably 0.1 to 1.0 mg/kg/day can be used. Suitable daily dosages for patients are thus on the order of from 2.5 to 500 mg p.o., preferably 5 to 250 mg p.o., more preferably 5 to 100 mg p.o., or on the order of from 0.5 to 250 mg i.v., preferably 2.5 to 125 mg i.v. and more preferably 2.5 to 50 mg i.v.

Dosaging may also be arranged in a patient specific manner to provide a predetermined concentration of an agent in the blood, as determined by the RIA technique. Thus patient dosaging may be adjusted to achieve regular on-going trough blood levels, as measured by RIA, on the order of from 50 to 1000 ng/ml, preferably 150 to 500 ng/ml.

The agents of the present invention are intended to be provided to a patient in an amount sufficient to modulate, and preferably reduce the amount or rate of binding and uptake of radiopharmaceuticals and/or radiodiagnostics to the megalin receptor. An amount is said to be sufficient to "modulate" or "reduce" the amount or rate of binding and uptake of radiopharmaceuticals and/or radiodiagnostics to the megalin receptor" if the dosage, route of administration, etc. of the agent is sufficient to alter and preferably reduce the amount or rate of the attachment to the megalin receptor. Such an effect can be assayed, for example, by examining the amount of radioactivity that was taken up by the kidney cells and/or that is excreted via the faeces of the test animals.

The administration of the agents of the present invention may be for either diagnostic or therapeutic purpose. When provided therapeutically, the agent is provided at (or shortly after) the onset of the appearance of symptoms of a modified excretion of the radiopharmaceutics and/or radiotherapeutics or measurable kidney damages, e.g. renal failure.

The receptor proteins are useful as targets for compounds which turn on, or off, or otherwise regulate binding of radiopharmaceutics and/or radiotherapeutics to the megalin receptor. The assays described in the present description clearly provide routine methodology by which a compound can be tested for an inhibitory effect on binding of a specific compound to megalin, such as a radiotherapeutic and/or radiodiagnostic. The in vitro studies of compounds which appear to inhibit binding selectively to the receptor are then confirmed by animal testing. Since the molecules are so highly evolutionarily conserved, it is possible to conduct studies in laboratory animals such as mice and/or rats to predict the effects in humans.

Assays for testing compounds for useful activity can be based solely on interaction with the receptor protein, preferably expressed on the surface of transfected cells such as those described here, although proteins in solution or immobilised on inert substrates can also be utilised, where the indication is inhibition or increase in binding of radiotherapeutics and/or radiodiagnostics.

Alternatively, the assays can be based on interaction with the gene sequence encoding the receptor protein, preferably the regulatory sequences directing expression of the receptor protein. For example, antisense oligonucleotides or antisense sequences which bind to the regulatory sequences, and/or to the protein encoding sequences can be synthesised using standard oligonucleotide synthetic chemistry. The antisense oligonucleotide can be stabilised for pharmaceutical use using standard methodology (encapsulation in a liposome or microsphere; introduction of modified nucleotides that are resistant to degradation or groups which increase resistance to endonucleases, such as phosphorothiodates and methylation), then screened initially for alteration of receptor activity in transfected or naturally occurring cells which express the megalin receptor, then in vivo in laboratory animals. Typically, the antisense oligonucleotide would inhibit expression of megalin and/or cubilin. However, sequences which block those sequences which "turn off" synthesis can also be targeted.

The megalin receptor protein for study can be isolated from either naturally occurring cells or cells which have been genetically engineered to express the receptor, as described here. In the preferred embodiment, the cells would have been engineered using the intact megalin gene.

Random generation of receptor or receptor encoding sequence binding molecules can be performed as follows. Molecules with a given function, catalytic or ligand-binding, can be selected for from a complex mixture of random molecules in what has been referred to as "in vitro genetics" (Szostak, 1992). One synthesises a large pool of molecules bearing random and defined sequences and subjects that complex mixture, for example, approximately 10¹⁵ individual sequences in 100 µg of a 100 nucleotide RNA, to some selection and enrichment process. For example, by repeated cycles of affinity chromatography and PCR amplification of the molecules bound to the ligand on the column, Ellington and Szostak (1990) estimated that 1 in 10¹⁰ RNA molecules folded in such a way as to bind a given ligand. DNA molecules with such ligand-binding behaviour have been isolated (Ellington and Szostak, 1992; Bock et al, 1992).

Computer modelling technology allows visualisation of the three-dimensional atomic structure of a selected molecule, like megalin and/or the radiopharmaceutics and/or radiotherapeutics binding part of megalin, and the rational design of new compounds that will interact with the molecule. The three-dimensional construct typically depends on data from x-ray crystallographic analyses or NMR imaging of the selected molecule. The molecular dynamics require force field data. The computer graphics systems enable prediction of how a new compound will link to the target molecule and allow experimental manipulation of the structures of the compound and target molecule to perfect binding specificity. Prediction of what the molecule-compound interaction will be when small changes are made in one or both requires molecular mechanics software and computationally intensive computers, usually coupled with user-friendly, menu-driven interfaces between the molecular design program and the user.

Examples of molecular modelling systems are the CHARMm and QUANTA programs, Polygen Corporation, Waltham, Mass. CHARMm performs the energy minimisation and molecular dynamics functions. QUANTA performs the construction, graphic modelling and analysis of molecular structure. QUANTA allows interactive construction, modification, visualisation, and analysis of the behaviour of molecules with each other.

A number of articles review computer modelling of drugs interactive with specific proteins, such as Rotivinen, et al., 1988 Acta Pharmaceutica Fennica 97, 159-166; Ripka, New Scientist 54-57 (Jun. 16, 1988); McKinaly and Rossmann, 1989 Annu. Rev. Pharmacol. Toxiciol. 29, 111-122; Perry and Davies, OSAR: Quantitative Structure-Activity Relationships in Drug Design pp. 189-193 (Alan R. Liss, Inc. 1989); Lewis and Dean, 1989 Proc. R. Soc. Lond. 236, 125-140 and 141-162; and, with respect to a model receptor for nucleic acid components, Askew, et al., 1989 J. Am. Chem. Soc. 111, 1082-1090. Other computer programs that screen and graphically depict chemicals are available from companies such as BioDesign, Inc., Pasadena, Calif., Allelix, Inc, Mississauga, Ontario, Canada, and Hypercube, Inc., Cambridge, Ontario. Although these are primarily designed for application to drugs specific to particular proteins, they can also be adapted to design of drugs specific to regions of DNA or RNA, once that region is identified. In the present invention, such regions would comprise regulatory regions of either megalin and/or cubilin.

Although described above with reference to design and generation of compounds which could alter binding, one could also screen libraries of known compounds, including natural products or synthetic chemicals, and biologically active materials, including proteins, for compounds which are inhibitors or activators, as already mentioned above.

Nucleic acid molecules containing the 5' regulatory sequences of the receptor genes can be used to regulate or inhibit gene expression *in vivo.* Vectors, including both plasmid and eukaryotic viral vectors, may be used to express a particular recombinant 5' flanking region-gene construct in cells depending on the preference and judgement of the skilled practitioner (see, e.g., Sambrook et al., Chapter 16). Furthermore, a number of viral and nonviral vectors are being developed that enable the introduction of nucleic acid sequences *in vivo* (see, e.g., Mulligan, (1993); U.S. Pat. No. 4,980,286; U.S. Pat. No. 4,868,116; incorporated herein by reference). Recently, a delivery system was developed in which nucleic acid is encapsulated in cationic liposomes which can be injected intravenously into a mammal. This system has been used to introduce DNA into the cells of multiple tissues of adult mice, including endothelium and bone marrow (see, e.g., Zhu et al., 1993; incorporated herein by reference).

The 5' flanking sequences of the receptor gene can also be used to inhibit the expression of the receptor. For example, an antisense RNA of all or a portion of the 5' flanking region of the receptor gene can be used to inhibit expression of the receptor *in vivo.* Expression vectors (e.g., retroviral expression vectors) are already available in the art which can be used to generate an antisense RNA of a selected DNA sequence which is expressed in a cell (see, e.g., U.S. Pat. No. 4,868,116; U.S. Pat. No. 4,980,286). Accordingly, DNA containing all or a portion of the sequence of the 5' flanking region of the megalin gene can be inserted into an appropriate expression vector so that upon passage into the cell, the transcription of the inserted DNA yields an antisense RNA that is complementary to the mRNA transcript of the megalin protein gene normally found in the cell. This antisense RNA transcript of the inserted DNA can then base-pair with the normal mRNA transcript found in the cell and thereby prevent the mRNA from being translated. It is of course necessary to select sequences of the 5' flanking region that are downstream from the transcriptional start sites for the receptor protein gene to ensure that the antisense RNA contains complementary sequences present on the mRNA.

Antisense RNA can be generated *in vitro* also, and then inserted into cells. Oligonucleotides can be synthesised on an automated synthesiser (e.g., Model 8700 automated synthesiser of Milligen-Biosearch, Burlington, Mass. or ABI Model 380B). In addition, antisense deoxyoligonucleotides have been shown to be effective in inhibiting gene transcription and viral replication (see e.g., Zamecnik et al., 1978; Zamecnik et al., 1986; Wickstrom et al., 1988; Crooke, 1993. Furthermore, recent work has shown that improved inhibition of expression of a gene by antisense oligonucleotides is possible if the antisense oligonucleotides contain modified nucleotides (see, e.g., Offensperger et. al., 1993 (*in vivo* inhibition of duck hepatitis B viral replication and gene expression by antisense phosphorothioate oligodeoxynucleotides); Rosenberg et al., WO 93/01286 (synthesis of sulfurthioate oligonucleotides); Agrawal et al., 1988 (synthesis of antisense oligonucleoside phosphoramidates and phosphorothioates to inhibit replication of human immunodeficiency virus-1); Sarin et al., 1989 (synthesis of antisense methylphosphonate oligonucleotides); Shaw et al., 1991 (synthesis of 3' exonuclease-resistant oligonucleotides containing 3' terminal phosphoroamidate modifications); incorporated herein by reference).

The sequences of the 5' flanking region of receptor protein gene can also be used in triple helix (triplex) gene regulation. Oligonucleotides complementary to gene promoter sequences on one of the strands of the DNA have been shown to bind promoter and regulatory sequences to form local triple nucleic acid helices which block transcription of the gene (see, e.g., Maher et al., 1989; Orson et al., 1991; Postal et al., 1991; Cooney et al., 1988; Young et al., 1991; Duval-Valentin et al., 1992; Blume et al., 1992 Nucl. Acids Res. 20, 1777-1784; Grigoriev et al., 1992, J. Biol. Chem. 267, 3389-3395.

Recently, both theoretical calculations and empirical findings have been reported which provide guidance for the design of oligonucleotides for use in oligonucleotide-directed triple helix formation to inhibit gene expression. For example, oligonucleotides should generally be greater than 14 nucleotides in length to ensure target sequence specificity (see, e.g., Maher et al., (1989); Grigoriev et al., (1992)). Also, many cells avidly take up oligonucleotides that are less than 50 nucleotides in length (see e.g., Orson et al., (1991); Holt et al., 1988; Wickstrom et al., 1988). To reduce susceptibility to intracellular degradation, for example by 3' exonucleases, a free amine can be introduced to a 3' terminal hydroxyl group of oligonucleotides without loss of sequence binding specificity (Orson et al., 1991). Furthermore, more stable triplexes are formed if any cytosines that may be present in the oligonucleotide are methylated, and also if an intercalating agent, such as an acridine derivative, is covalently attached to a 5' terminal phosphate (e.g., via a pentamethylene bridge); again without loss of sequence specificity (Maher et al., (1989); Grigoriev et al., (1992).

Methods to produce or synthesise oligonucleotides are well known in the art. Such methods can range from standard enzymatic digestion followed by nucleotide fragment isolation (see e.g., Sambrook et al., Chapters 5, 6) to purely synthetic methods, for example, by the cyanoethyl phosphoramidite method using a Milligen or Beckman System 1Plus DNA synthesiser (see also, Ikuta et al., 1984 (phosphotriester and phosphite-triester methods); Narang et al., (1980) (phosphotriester method). Accordingly, DNA sequences of the 5' flanking region of the receptor protein gene described herein can be used to design and construct oligonucleotides including a DNA sequence consisting essentially of at least 15 consecutive nucleotides, with or without base modifications or intercalating agent derivatives, for use in forming triple helices specifically within the 5' flanking region of a receptor protein gene in order to inhibit expression of the gene.

In some cases it may be advantageous to insert enhancers or multiple copies of the regulatory sequences into an expression system to facilitate screening of methods and reagents for manipulation of expression.

Compounds which are effective for blocking binding of the receptor can also consist of fragments of the receptor proteins, expressed recombinantly and cleaved by enzymatic digest or expressed from a sequence encoding a peptide of less than the full length receptor protein. These will typically be soluble proteins, i.e., not including the transmembrane and cytoplasmic regions, although smaller portions determined in the assays described above to inhibit or compete for binding to the receptor proteins can also be utilised. It is a routine matter to make appropriate receptor protein fragments, test for binding, and then utilise. The preferred fragments are of human origin, in order to minimise potential immunological response. The peptides can be as short as five to eight amino acids in length and are easily prepared by standard techniques. They can also be modified to increase in vivo half-life, by chemical modification of the amino acids or by attachment to a carrier molecule or inert substrate.

Based on studies with other peptide fragments blocking receptor binding, the IC₅₀, the dose of peptide required to inhibit binding by 50%, ranges from about 50 µM to about 300 µM, depending on the peptides. These ranges are well within the effective concentrations for the in vivo administration of peptides, based on comparison with, for example, the RGD-containing peptides, described, for example, in U.S. Pat. No. 4,792,525 to Ruoslaghti, et al., used in vivo to alter cell attachment and phagocytosis. The peptides can also be conjugated to a carrier protein such as keyhole limpet hemocyanin by its N-terminal cysteine by standard procedures such as the commercial Imject kit from Pierce Chemicals or expressed as a fusion protein, which may have increased efficacy. As noted above, the peptides can be prepared by proteolytic cleavage of the receptor proteins, or, preferably, by synthetic means. These methods are known to those skilled in the art. An example is the solid phase synthesis described by J. Merrifield, 1964, used in U.S. Pat. No. 4,792,525, and described in U.S. Pat. No. 4,244,946, wherein a protected alpha-amino acid is coupled to a suitable resin, to initiate synthesis of a peptide starting from the C-terminus of the peptide. Other methods of synthesis are described in U.S. Pat. Nos. 4,305,872 and 4,316,891. These methods can be used to synthesise peptides having identical sequence to the megalin receptor protein described herein, or substitutions or additions of amino acids, which can be screened for activity as described above.

The peptide can also be administered as a pharmaceutically acceptable acid- or base-addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

Peptides containing cyclopropyl amino acids, or amino acids derivatised in a similar fashion, can also be used. These peptides retain their original activity but have increased half-lives in vivo. Methods known for modifying amino acids, and their use, are known to those skilled in the art, for example, as described in U.S. Pat. No. 4,629,784 to Stammer.

The peptides are generally active when administered parenterally in amounts above about 1 ug/kg of body weight. Based on extrapolation from other proteins, for treatment of most inflammatory disorders, the dosage range will be between 0.1 to 70 mg/kg of body weight. This dosage will be dependent, in part, on whether one or more peptides are administered.

The present invention further includes the use of the agents of the present invention in diagnostic and therapeutic applications.

Conditions for incubating an test agent with a test sample, consisting of megalin and/or cubilin and/or radiopharmaceutics and/or radiotherapeutics vary. Incubation conditions will depend on factors such as the type of candidate agent, format, and detection system employed for the assay, as well as the nature of the test sample used in the assay. For example, condition will vary slightly when a whole antibody, a single chain antibody, a F(ab) fragment, or a peptide candidate agent is used. One skilled in the art will recognise that any one of the commonly available immunological assay formats (such as radioimmunoassays, enzyme-linked immunosorbent assays, diffusion based ouchterlony, or rocket immunofluorescent assays) can readily be adapted to employ the antibodies as agents of the present invention. Examples of such assays can be found in T. Chard, An Introduction to Radioimmunoassay and Related Techniques, Elsevier Science Publishers, Amsterdam, The Netherlands (1986); G. R. Bullock et al., Techniques in Immunocytochemistry, Academic Press, Orlando, Fla. Vol. 1 (1982), Vol. 2 (1983), Vol. 3 (1985); and P. Tijssen, P., Practice and Theory of Enzyme Immunoassays: Laboratory Techniques in Biochemistry and Molecular Biology, Elsevier Science Publishers, Amsterdam, The Netherlands (1985).

In one embodiment of the above-described method, the candidate compound of the present invention is immobilised on a solid support for use in the diagnostic and/or screening assay. Illustrative examples of such solid supports include, but are not limited to, plastics such as polycarbonate, complex carbohydrates such as agarose and sepharose, and acrylic resins, such as polyacrylamide and latex beads. Techniques for coupling candidate compounds such as antibodies, peptides and the like to such solid supports are well known in the art, as described, for example, in D. M. Weir et al., Handbook of Experimental Immunology, 4th Ed., Black-well Scientific Publications, Oxford, England, Chapter 10 (1986) and W. D. Jacoby et al., (1974). In another format of the assay, the megalin and/or cubilin is coupled to said solid support.

Additionally, one or more of the candidate compound of the present invention which is used in one of the above-described methods can be detectably labelled prior to use, for example, through the use of radioisotopes, affinity labels (such as biotin, avidin, etc.), enzymatic labels (such as horse radish peroxidase, alkaline phosphatase, etc.) fluorescent labels (such as FITC or rhodamine, etc.), paramagnetic atoms, etc. Procedures for accomplishing such labelling are well-known in the art (L. A. Sternberger et al., (1970); E. A. Bayer et al., (1979); E. Engval et al., (1972); and J. W. Goding, (1976)).

The materials used in the inventive assays are ideally suited for the preparation of a kit. For example, the present invention provides a compartmentalised kit to receive in close confinement, one or more containers which comprises: a) a first container comprising an modulator for the binding and/or uptake of radiopharmaceutics and/or radiodiagnostics to the megalin receptor; and b) one or more other containers comprising one or more of the following: wash reagents and reagents capable of detecting the presence of radiopharmaceuticals and/or radiodiagnostics bound or taken up into the cells of the kidney.

As used herein, a compartmentalised kit includes any kit in which modulators, radiopharmaceutics and/or radiotherapeutics and/or reagents are contained in separate containers. Illustrative examples of such containers include, but are not limited to, small glass containers, plastic containers or strips of plastic or paper. Particularly preferred types of containers allow the skilled worker to efficiently transfer reagents from one compartment to another compartment such that the samples and reagents are not cross-contaminated and the agents or solutions of each container can be added in a quantitative fashion from one compartment to another. Such containers include, but are not be limited to, a container which will accept the test sample, a container which contains one or more of the agents of the present invention used in the assay, containers which contain wash reagents (such as phosphate buffered saline, Tris-buffers, etc.), and containers which contain the reagents used to detect the bound agent.

The types of detection reagents which can be used in the above described kits include, but are not limited to, labelled secondary agents, or in the alternative, if the primary agent is labelled, enzymatic or agent binding reagents which are capable of reacting with the labelled agent. One skilled in the art will readily recognise that the agents of the present invention can be readily incorporated into one of the established kit formats which are well known in the art.

The present invention further includes DNA vectors which contain the DNA sequences described above and below. In particular, these may be vectors in which the DNA molecules described are functionally linked to control sequences which allows expression of the corresponding polypeptides. These are preferably plasmids which can be replicated and/or expressed in prokaryotes such as E. coli and/or in eukaryotic systems such as yeasts or mammalian cell lines. These vectors may also be mammalian viral vectors which can be replicated and/or expressed in eukaryotes such as mammalian cell lines and in the human patient, as "host," for integration into the cellular genome of the patient and expression as genetic therapy systems.

The invention also includes host organisms transformed with the above vectors. Expression in prokaryotes and eukaryotes may be carried out using techniques known in the art. The DNA sequences according to the invention may be expressed as fusion polypeptides or as intact, native polypeptides. Fusion proteins may advantageously be produced in large quantities. They are generally more stable than the native polypeptide and are easy to purify. The expression of these fusion proteins can be controlled by normal host DNA sequences.

For example, the DNA sequences according to the invention can be cloned and expressed as lacZ fusion genes in E. coli. A person skilled in the art has a variety of vector systems available for this purpose, e.g. the pUR-vector series (U. Ruther and B. Muller-Hill, (1983)). The bacteriophage promoter .lambda.P_{R} may also be used, in the form of the vectors pEX-1 to -3, for expressing large amounts of Cro-β-galactosidase fusion protein (K. K. Stanley and J. P. Luzio. (1984)). Analogously, the tac promoter which can be induced with IPTG can also be used, for example in the form of the pROK-vector series (CLONTECH Laboratories).

The prerequisite for producing intact native polypeptides using E. coli is the use of a strong, regulatable promoter and an effective ribosome binding site. Promoters which may be used for this purpose include the temperature sensitive bacteriophage .lambda.P_{L} -promoter, the tac-promoter inducible with IPTG or the T7-promoter. Numerous plasmids with suitable promoter structures and efficient ribosome binding sites have been described, such as for example pKC30 (.lambdaP_{L} ; Shimatake and Rosenberg, (1981), pKK173-3 (tac, Amann and Brosius, (1985)) or pET-3 (T7-promoter (Studier and Moffat, J (1986)).

A number of other suitable vector systems for expressing the DNA according to the invention in E. coli are known from the prior art and are described, for example, in Sambrook et al., Molecular Cloning: A Laboratory Manual, Cold Spring Harbor Laboratory Press (1989)).

Suitable E. coli strains which are specifically tailored to a particular expression vector are known to those skilled in the art (Sambrook et al., Molecular Cloning: A Laboratory Manual, Cold Spring Harbor Laboratory Press (1989)). The experimental performance of the cloning experiments, the expression of the polypeptides in E. coli and the working up and purification of the polypeptides are known and are described, for example, in Sambrook et al., Molecular Cloning: A Laboratory Manual, Cold Spring Harbor Laboratory Press (1989). In addition to prokaryotes, eukaryotic microorganisms such as yeast may also be used.

For expression in yeast, the plasmid YRp7 (Stinchcomb et al. (1979); Kingsman et al., (1979); Tschumper et al., (1980)) and the plasmid YEp13 (Bwach et al., (1979)) are used, for example. The plasmid YRp7 contains the TRP 1-gene which provides a selection marker for a yeast mutant (e.g. ATCC No. 44076) which is incapable of growing in tryptophan-free medium. The presence of the TRP1 defect as a characteristic of the yeast strain used then constitutes an effective aid to detecting transformation when cultivation is carried out without tryptophan. The same is true with the plasmid YEp13, which contains the yeast gene LEU-2, which can be used to complete a LEU-2-minus mutant.

Other suitable marker genes for yeast include, for example, the URA3- and HIS3-gene. Preferably, yeast hybrid vectors also contain a replication start and a marker gene for a bacterial host, particularly E. coli, so that the construction and cloning of the hybrid vectors and their precursors can be carried out in a bacterial host. Other expression control sequences suitable for expression in yeast include, for example, those of PHO3- or PHO5-gene.

Other suitable promoter sequences for yeast vectors contain the 5'-flanking region of the genes of ADH I (Ammerer, (1983)), 3-phosphoglycerate kinase (Hitzeman et al., (1980)) or other glycolytic enzymes (Kawaski and Fraenkel, (1982)) such as enolase, glycerinaldehyde-3-phosphate-dehydrogenase, hexokinase, pyruvate-decarboxylase, phosphofructokinase, glucose-6-phosphate isomerase, phosphoglucose-isomerase and glucokinase. When constructing suitable expression plasmids, the termination sequences associated with these genes may also be inserted in the expression vector at the 3'-end of the sequence to be expressed, in order to enable polyadenylation and termination of the mRNA.

Other promoters are the promoter regions of the genes for alcohol dehydrogenase-2, isocytochrome C, acid phosphatase and enzymes which are responsible for the metabolism of maltose and galactose. Promoters which are regulated by the yeast mating type locus, such as promoters of the genes BARI, MF.alpha. 1, STE2, STE3, STE5 can be inserted in temperature regulated systems by the use of temperature-dependent sir mutations. (Rhine, Ph.D. Thesis, University of Oregon, Bugene, Oreg. (1979); Herskowitz and Oshima, The Molecular Biology of the Yeast Saccharomyces, Cold Spring Harbor Laboratory Press, part I, pp. 181-209 (1981)). Generally, however, any vector which contains a yeast-compatible promoter and origin replication and termination sequences is suitable. Thus, hybrid vectors which contain sequences homologous to the yeast plasmid DNA may also be used. Such hybrid vectors are incorporated by recombination within the cells of existing plasmids or replicate autonomously.

In addition to yeasts, other eukaryotic systems may, of course, be used to express the polypeptides according to the invention. Since post-translational modifications such as disulphide bridge formation, glycosylation, phosphorylation and/or oligomerisation are frequently necessary for the expression of biologically active eukaryotic proteins by means of recombinant DNA, it may be desirable to express the DNA according to the invention not only in mammalian cell lines but also insect cell lines.

Functional prerequisites of the corresponding vector systems comprise, in particular, suitable promoter, termination and polyadenylation signals as well as elements which make it possible to carry out replication and selection in mammalian cell lines. For expression of the DNA molecules in the context of the invention it is particularly desirable to use vectors which are replicable both in mammalian cells and also in prokaryotes such as *E. coli.*

Vectors derived from viral systems such as SV40, Epstein-Barr-virus, etc., include, for example, pTK2, pSV2-dhfv, pRSV-neo, pKO-neo, pHyg, p205, pHEBo, etc. (Sambrook et al., Molecular Cloning: A Laboratory Manual, Cold Spring Harbor Press, N.Y. (1989)).

After transformation in suitable host cells, e.g. kidney or CHO-cells, corresponding transformed cells may be obtained with the aid of selectable markers (thymidine-kinase, dihydrofolate-reductase etc.) and the corresponding polypeptides are isolated after expression. The host cells suitable for the vectors are known, as are the techniques for transformation (microinjection, electroporation, calcium phosphate method, etc.) as described, for example, in Sambrook et al., Molecular Cloning: A Laboratory Manual, Cold Spring Harbor Press, N.Y. (1989).

For cloning corresponding DNA fragments in prokaryotic or eukaryotic systems, the selected vector may be cut, for example, with a restriction endonuclease and, optionally after modification of the linearised vector thus formed, an expression control sequence equipped with corresponding restriction ends is inserted. At the 3'-end (in the direction of translation) the expression control sequence contains the recognition sequence of a restriction endonuclease, so that the vector already containing the expression control sequence is digested with the said restriction enzyme and the DNA molecule according to the invention, provided with ends which fit, can be inserted. It is advantageous to cleave the vector which already contains the expression control sequence with a second restriction endonuclease inside the vector DNA and to insert the DNA molecule provided with the correct ends into the vector fragment produced. The techniques required are described, for example, by Sambrook et al. Molecular Cloning: A Laboratory Manual Cold Spring Harbor Press. N.Y. (1989).

Apart from the DNA molecules specified, the invention also relates to processes for preparing the vectors described herein, particularly expression vectors. These vectors are characterised in that a DNA provided with corresponding ends and coding for a functional derivative or a fragment of the megalin receptor is inserted into a vector DNA cut with restriction endonucleases and containing the expression control sequences described by way of example, in such a way that the expression control sequences regulate the expression of the DNA inserted. The peptides and antibody agents of the present invention which are obtained by the expression of recombinant DNA or from the native receptor molecule may, of course, also be derivatised by chemical or enzymatic processes.

The present invention also encompasses pharmaceutical compositions which include a modulator agent identified using the above-described methods. These compositions include a pharmaceutically effective amount of the modulator of the megalin mediated uptake of radiopharmaceutics and/or radiotherapeutics in a pharmaceutically acceptable carrier or diluent. Acceptable carriers or diluents for therapeutic use are well known in the pharmaceutical art, and are described, for example, in Remington's Pharmaceutical Sciences, Mack Publishing Co. (Gennaro ed., 1985). Preservatives, stabilisers, dyes and even flavouring agents may be provided in the pharmaceutical composition. For example, sodium benzoate, sorbic acid and esters of p-hydroxybenzoic acid may be added as preservatives. In addition, antioxidants and suspending agents may be used.

The compositions of the present invention may be formulated and used as tablets, capsules or elixirs for oral administration, suppositories for rectal administration, sterile solutions, suspensions for injectable administration, and the like. Injectables can be prepared in conventional forms, either as liquid solutions or suspensions, solid forms suitable for solution or suspension in liquid prior to injection, or as emulsions. Suitable excipients are, for example, water, saline, dextrose, mannitol, lactose, lecithin, albumin, sodium glutamate, cysteine hydrochloride, and the like. In addition, if desired, the injectable pharmaceutical compositions may contain minor amounts of nontoxic auxiliary substances, such as wetting agents, pH buffering agents, and the like. If desired, absorption enhancing preparations (e.g., liposomes) may be utilised.

The pharmaceutically effective amount of the composition required as a dose will depend on the route of administration, the type of animal being treated, and the physical characteristics of the specific animal under consideration. The dose can be tailored to achieve optimal efficacy but will depend on such factors as weight, diet, concurrent medication and other factors which those skilled in the medical arts will recognise. Typically, human clinical applications of products are commenced at lower dosage levels, with dosage level being increased until the desired effect is achieved. The determination of effective dosage levels, that is the dosage levels necessary to achieve the desired result, will be within the ambit of one skilled in the art based on generally accepted protocols for clinical studies.

In practising the methods of the invention, the modulator agents are used alone or in combination with one another, or in combination with other therapeutic or diagnostic agents, e.g. radiotherapeutics and/or radiodiagnostics. A modulating agent can be administered parenterally, intravenously, subcutaneously, intramuscularly, colonically, rectally, nasally or intraperitoneally, employing a variety of dosage forms.

In the methods of the present invention, polypeptides consisting of the megalin and/or cubilin parental sequences or fragments thereof are isolated, i.e. unaccompanied by at least some of the material with which it is associated in its natural state, preferably constituting at least about 0.5%, preferably at least about 5%, more preferably at least about 50% by weight of total polypeptide present in a given sample, and a pure polypeptide constitutes at least about 90%, and preferably at least about 99% by weight of the total polypeptide in a given sample, as are preferred subject polypeptides comprising other than parental sequence.

In addition the subject polypeptides consisting of the megalin and/or cubilin parental sequences or fragments thereof have corresponding-polypeptide-specific antibody binding, elicitation or binding or elicitation inhibitory activity, e.g. elicit specific antibody in a heterologous host, etc. In a particular embodiment, the above polypeptide fragments provide specific antigens and/or immunogens, especially when coupled to carrier proteins. For example, peptides are covalently coupled to keyhole limpet antigen (KLH) and the conjugate is emulsified in Freunds complete adjuvant. Laboratory rabbits are immunised according to conventional protocol and bled. The presence of specific antibodies is assayed by solid phase inmmunosorbant assays using immobilised corresponding polypeptide.

### EXAMPLES

The following examples are meant to be illustrative, not limiting.

In order to investigate, whether megalin and cubilin play a role in the renal uptake of radiopharmaceuticals into cells of the proximal tubule, in-vivo experiments with RAP-deficient mice (Jax®Mice, strain B6.129S7-*Lrpap1*^{*tm1Her*}) were carried out.

RAP (receptor-associated-protein) is a 39 kDa chaperone-like protein that binds tightly and with a high affinity in the nanomolar range to megalin. RAP is mainly located in the endo-plasmatic reticulum where it is responsible for the proper folding and escort of megalin to the plasma membrane (Willnow, 1995). RAP also binds to other members of the LDL-receptor family, such as the receptor LRP (LDL-receptor related protein) which is not expressed in the kidneys but mostly in the liver and the brain. In RAP-deficient mice the megalin expression in the kidneys is reduced by about 80% (Birn, 2000) and also the cubilin expression is reduced by some extend. These mice are therefore a representative model to study the megalin dependence of the uptake of radiopharmaceuticals. There exist also megalin knock-out mice which have the disadvantage that only 2% of the bred animals survive to adulthood due to several malformations especially of the forebrain (Leheste, 1999).

RAP-deficient mice (stock #002987) and mice of the control strain C57BL/ 6J (Jax® Mice, stock #000664) were treated with a single intravenous injection (1-2 µCi/ animal) of the following radiopharmaceuticals:
- In-111 MX-DTPA labelled single-chain Fragment (scFv) against ED-B (see table 2, "FRA1")
- Tc-99m labelled scFv against ED-B (see table 2, "FRA2"), and
- Re-188 labelled somatostatin-analogue peptide (Neotide ®)

After 24 hours post injection the animals were killed and the organs as well as the collected urine and faeces counted for radioactivity. All organs including the blood showed no significant difference between controls and RAP-deficient mice with the exception of the kidneys. The kidney uptake of the radiolabeled compounds was markedly reduced in RAP-deficient mice. The results are depicted in tables 2 and 3, below.

**Table 2**

| (n = 3) | **In-111-mx-DTPA-FRA1** | | **Tc-99m-FRA2** | | **Re-188-Neotide®** | | **Tc-99m-NeoTect®** | |
|---|---|---|---|---|---|---|---|---|
| | **Control** | **RAP**^{**-/-**} | **Control** | **RAP**^{**-/-**} | **Control** | **RAP**^{**-/-**} | **Control** | **RAP**^{**-/-**} |
| **Kidneys** (% ID/g) | 67.02 | 8.49 | 4.03 | 2.17 | 3.27 | 1.07 | 261.44 | 99.32 |
| *SD* | *5.27* | *2.36* | *0.98* | *0.24* | *0.59* | *0.06* | *79.44* | *23.97* |
| **Urine** (% ID) | 48.01 | 58.87 | 49.41 | 61.86 | 36.69 | 57.85 | 23.60 | 40.44 |
| *SD* | *6.34* | *12.65* | *4.47* | *2.74* | *8.71* | *5.81* | *2.80* | *5.00* |

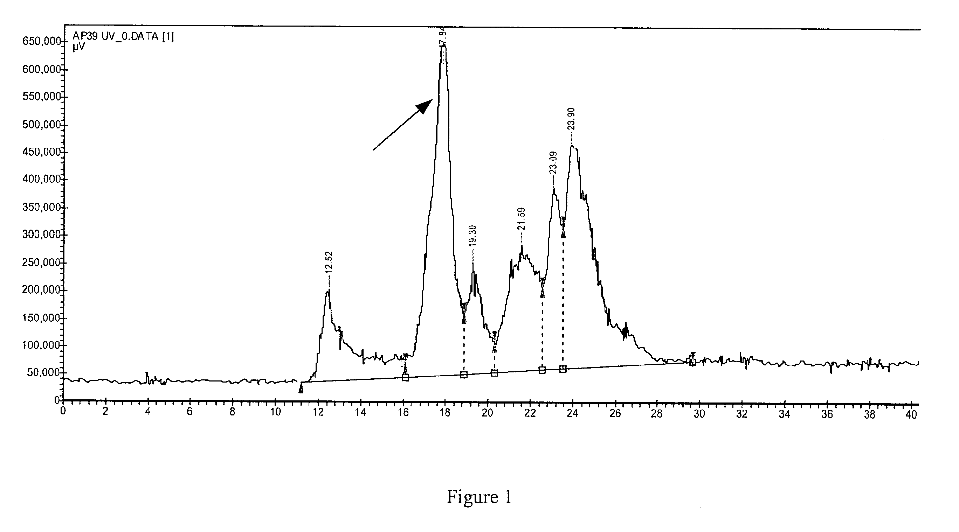

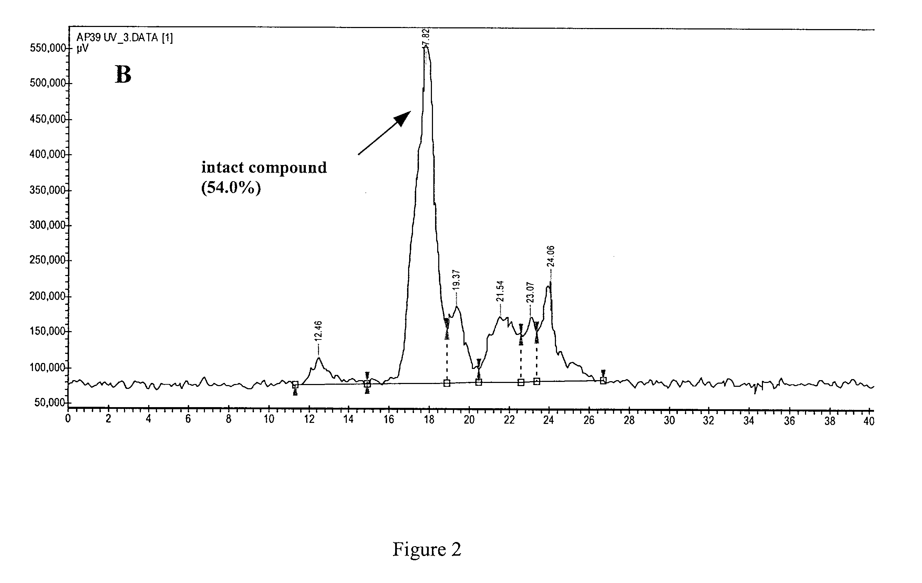

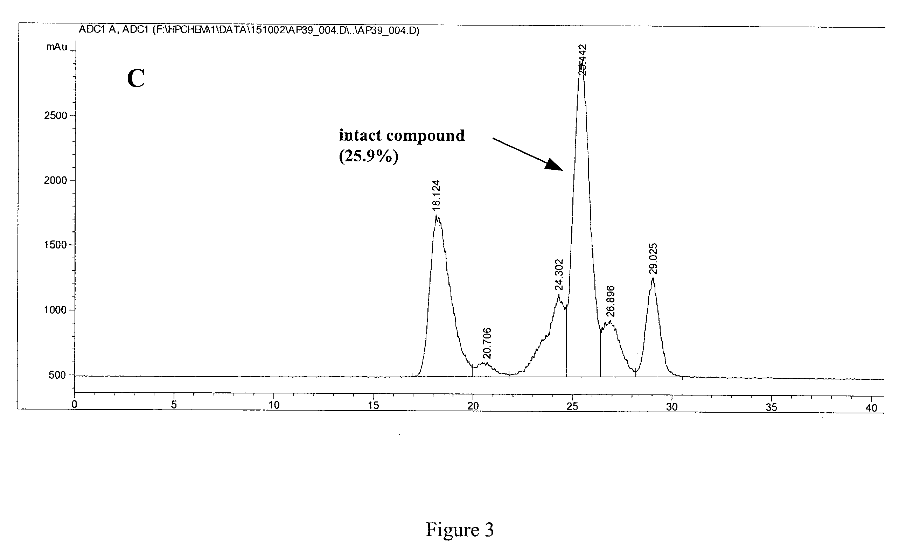

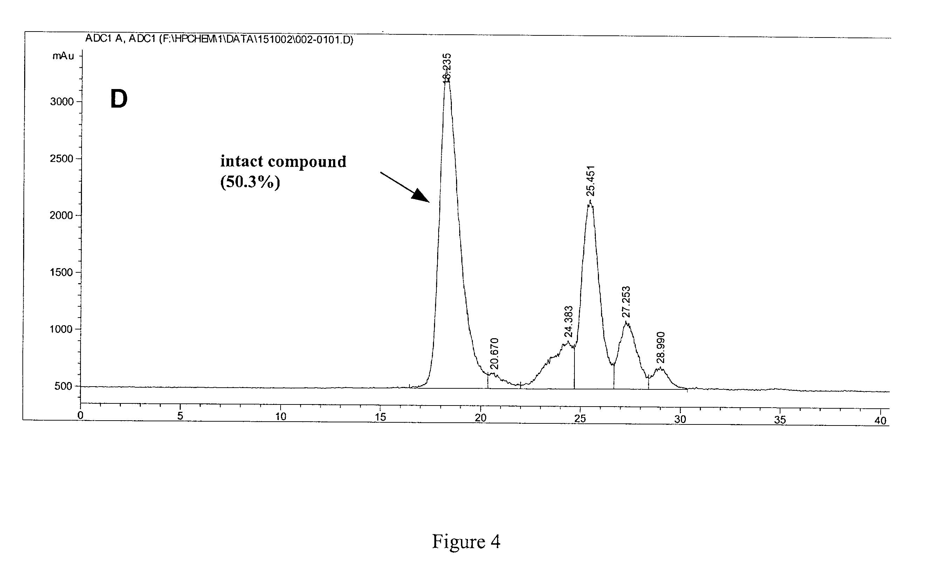

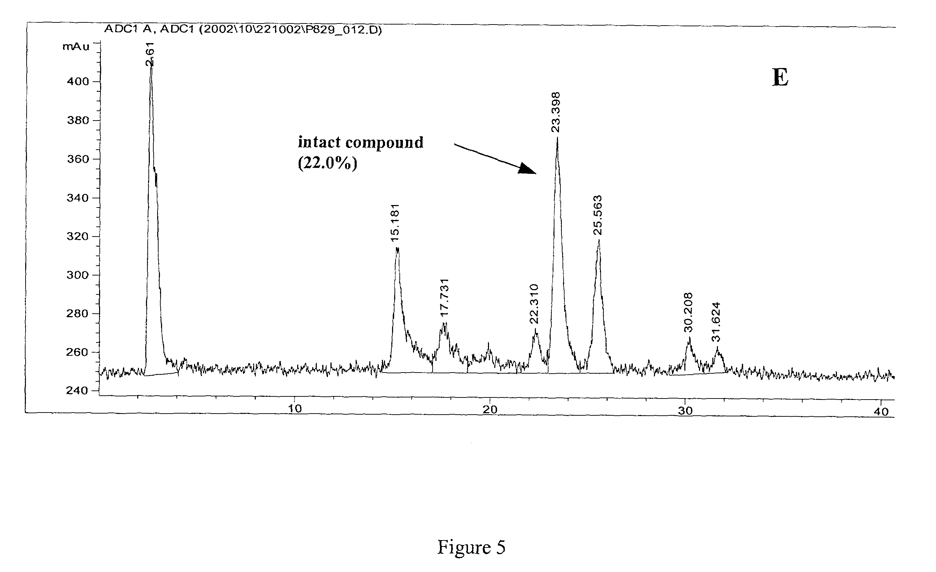

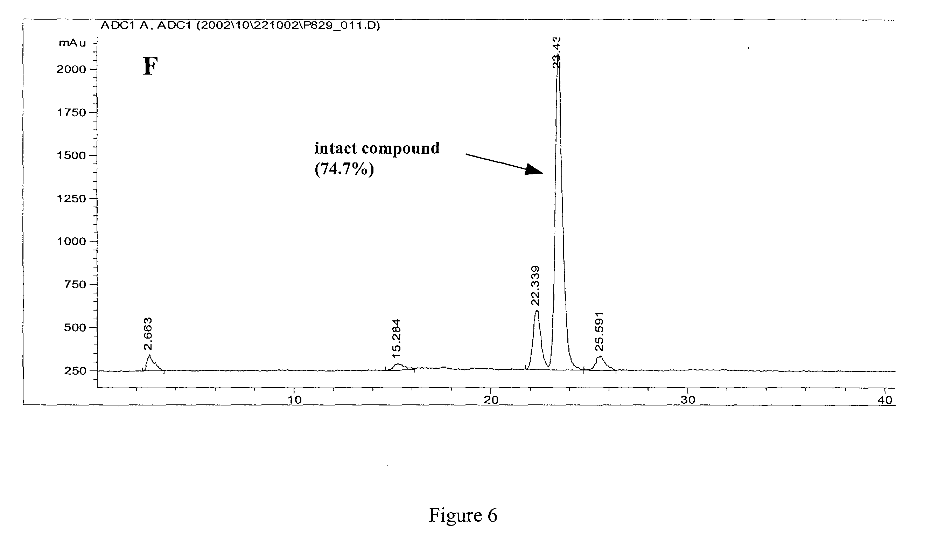

The results of these assays are further illustrated by the HPLC diagrams that are depicted in Figures 1 to 6, wherein a comparison of HPLC analyses of urine samples from controls (Figures 1, 3, and 5) and Rap-deficient mice (Figures 2, 4, and 6) after injection of radiopharmaceuticals: In-111-mx-DTPA-FRA1 (Figures 1 and 2), Tc-99m-FRA2 (Figures 3 and 4), and Tc-99m-NeoTect® (Figures 5 and 6). The HPLC analyses of urine samples show that in comparison with the control strain most of the radiolabeled compounds in the RAP deficient mice is excreted into the urine in an intact form due to the reduced megalin receptor expression in the kidneys and the subsequent reduced renal reabsorption.

It could therefore be concluded that the megalin receptor is mainly responsible for the uptake of radiopharmaceuticals in the proximal tubular cells of the kidney. It can also be assumed that binding to cubilin might play a role in renal endocytosis of radiopharmaceuticals.

The following assays can exemplarily be used to screen for compounds which are effective in methods for alter megalin expression, concentration, or uptake of radiopharmaceutics and/or radiodiagnostics.

**SPA (Scintillation Proximity Assay) -** wheat term agglutinin coupled SPA-beads (PVT-PEI WGA Type A beads, AMERSHAM) bind to prepared membranes (e.g. from OK cells). Only when the radiolabeled ligand such as I-125-anti-megalin IgG or I-125-RAP binds to the megalin receptor a signal is induced in the SPA-bead which can be measured by a Top Count NXT (PERKIN ELMER LIFE SCIENCE). In a 96-well plate each well contains 20 µl assay buffer (50 mM Hepes pH 8.0, % mM MgCl₂, 1 mM EGTA, 0.3% Polyethylenimine, 1 tablet Complete EDTA free), 20 µl SPA-beads dissloved in assay buffer (40 mg/ml), 20 µl purified megalin or membrane preparation in assay buffer and 40 µl labeled ligand (eg. I-125-anti-megalin IgG or I-125-RAP). The plate is covered and incubated on a shaker for 1-6 h. Again 100 µl assay buffer is added to each well and the plate centrifuged at 1500 rpm at RT and counted.

**SPR (Surface Plasmon Resonance) analysis** - interactions and binding kinetics of the megalin receptor and possible ligands can be assessed by a BIAcore 2000 instrument with the accompanying software (BIACORE, Sweden). The BIAcore sensor chips (type CM5) are activated with a 1:1 mixture of 0.2 M *N*-ethyl-*N*'-(3-dimethylaminopropyl) carbodiimide and 0.05 M *N*-hydroxysuccimide in water. Purified megalin from rabbit or mouse kidneys is immobilized on the chips at a density of ~ 40 fmol/ mm². The SPR signal from immobilized rabbit megalin generates 15-21x10³ BIAcore response units equivalent to 25-35 fmol/ mm². The flow cells are generated with 20 µl 1.5 M glycine-HCl pH 3.0. The flow buffer is 10 mM Hepes, 150 mM NaCl, 1.5 mM CaCl₂ and 1mM EGTA pH 7.4. The number of ligands bound to per immobilized receptor is estimated by divding the ratio Response Units (RU)_{ligand}/mass_{ligand} with RU_{receptor/}mass_{receptor}.

For the in vitro assays, the following (non-limiting) kidney cell lines can be used:

**Table 1**

| ***Cell line*** | ***ATCC number*** |
|---|---|
| LLC-PK1 | CL-101 |
| LLC-RK1 | CC1-106 |
| A6 | CCL-102 |
| OK | CRL-1840 |
| MDCK | CCL-34 |
| HK-2 | CRL-2190 |
| HaK | CCL-15 |
| LLC-MK2 | CCL-7 |

In-vitro binding assay without cofactor - Kidney cell membrane extracts are prepared as described previously (Taylor J.E., et al. 1994). Lysates are incubated with 1-2 µCi of the radiopharmaceutical and 1 µg of the respective candidate compound for 6 h at 4°C. The lysates are washed rapidly three times in 150 mM NaCl, 10 mM Tris-HCl pH 7.5, 2 mM each MgCl₂, CaCl₂, and MnCl₂ for 10 min. SDS sample buffer is added to the supernatant or lysates. Proteins are separated by electrophoresis on 4% SDS-polyacrylamide gel electrophoresis under nonreducing conditions and counted for radioactivity.

In vitro binding assay with cofactor - The same assay as above was repeated, except that an amount of cofactor in a range of between 100 ng and 1 µg was added together with the candidate compound.

Cell Lines expressing the receptor and Tissue Culture - Both Cell lines containing the wild type megalin receptor and the megalin receptor containing a stop codon in the cytoplasmic domain as well as cells that are additionally expressing cubilin (for cofactor analysis) are prepared. As a control, cells lacking the megalin receptor and/or the expression thereof are used. The plasmid pcDNA3.1/ZEO DAB555 contains the mouse megalin cDNA under control of the CMV promoter pcDNA3.1/ZEO(+) vector (Invitrogen) and is used to create megalin overexpressing cell lines. Multiple clones are generated after transfection and Zeocin selection and analysed for levels of megalin expression by immunoblotting using specific antibody detection. All cell lines are maintained in Dulbecco's minimal essential medium (DMEM) with 100 units/ml penicillin, and 100 µg/ml streptomycin sulfate, supplemented with 5% (v/v) fetal calf serum (Life Technologies, Inc., Grand Island, N.Y.).

Northern blot analysis of murine and/or human tissues shows that megalin is most abundantly expressed in kidney and is present at lower levels in some other tissues. The Northern blot technique can therefore be employed to show differences in the expression of megalin in these cells.

Northern blot analysis - 0.5 micrograms of poly(A)+ RNA prepared from different murine tissues or from 3T3-L1 cells or kidney cells on zero, two, four, six or eight days after initiation of differentiation into adipocytes as described by Baldini et al., 1992 Proc. Natl. Acad. Sci. U.S.A. 89, 5049-5052, is fractionated on a formaldehyde/agarose gel (1.0%) and then blotted and fixed onto a Biotrans.TM. nylon membrane.

The blots are hybridised with probes that are ³²P-labelled (2 x 10⁶ cpm/ml, random-primed labelling system). The hybridisation and washing conditions, at 42°C and 50°C, respectively, are performed as described by Charron et al., 1989. The probe for SR-BI mRNA analysis was a 0.6 kb BamHI fragment from the cDNAs coding region. The coding region of murine cytosolic hsp70 gene (Hunt and Calderwood, 1990) is used as a control probe for equal mRNA loading.

The cell surface 4°C binding is assayed using either method A or method B as indicated. In method A, cells are pre-chilled on ice for 15 min, re-fed with radioactivity of radiopharmaceutic in ice-cold medium B supplemented with 10% (v/v) fetal bovine serum, with or without 75-200 µg/ml unlabeled M-BSA, and incubated 2 hr at 4°C. on a shaker. Cells are then washed rapidly three times with Tris wash buffer (50 mM Tris-HCl, 0.15 M NaCl, pH 7.4) containing 2 mg/ml BSA, followed by two 5 min washes, and two rapid washes with Tris wash buffer without BSA. The cells are solubilised in 1 ml of 0.1 N NaOH for 20 min at room temperature on a shaker, 30 µl are removed for protein determination, and the radioactivity in the remainder is determined using a LKB gamma counter.

Method B differs from method A in that the cells are pre-chilled for 45 minutes, the medium contains 10 mM HEPES and 5% (v/v) human lipoprotein-deficient serum rather than fetal bovine serum, and the cell-associated radioactivity released by treatment with dextran sulfate is measured.

Cellular Binding and uptake Assays - For uptake and binding assays, the cells are plated in 6-well plates at a cell density of 60,000 cells/well in Medium A (for example minimal essential medium) supplemented with 100 units/ml penicillin, 100 µg/ml streptomycin sulfate, and with 5% (v/v) fetal calf serum. On day 3 the cells are washed twice with PBS (pH 6.0) and switched to serum-free medium B (Dulbecco's modified Eagle's medium (minus glutamine) with 10-30 kBq/ml of the following radiopharmaceuticals: In-111 MX-DTPA labelled single-chain Fragment (scFv) against ED-B; Tc-99m labelled scFv against ED-B; or Re-188 labelled somatostatin-analogue peptide. The cells are the incubated with the respectice candidate agent. After 1, 3, and 5 h, at 37°C or at 0-4°C for binding, the medium is discarded and the cells are washed several times with ice-cold PBS. Surface binding, uptake, and degradation are measured by counting for radioactivity.

Megalin receptor activities at 37°C are measured by ligand binding, uptake and degradation assays. The specific binding of the radiotherapeutic(s) and/or radiodiagnostic(s) is determined by adding an 100 fold excess of the non-labelled pharmacophore to the incubation medium. Then a subtraction calculation is performed. The values for binding and uptake can be combined and are presented as binding plus uptake observed after a 5 hour incubation and are expressed as cpm of radioactivity of radiopharmaceutic per 5 hr per mg cell protein. Degradation activity is expressed as cpm of radioactivity of radiopharmaceutic degraded in 5 hours per mg of cell protein. The values represent the differences between the results obtained in the presence (single determinations) and absence (duplicate determinations) of excess unlabeled candidate agent.

Determination of megalin expression in cells The following example is meant to be illustrative, not limiting. For illustrative purposes, this example employs cells which save been stably transfected with a megalin-luciferase reporter construct. Other reporters may be used, e.g. an additional cubilin-reporter construct. The assay, as described, is configured for cells grown in 96 well plates. The assay is not in any way confined to these characteristics and can equally well employ transiently transfected cells, reporter proteins other than luciferase, or cells grown in tissue culture wells of other sizes. These parameters were chosen as examples because they afford the considerable opportunity for automation and high throughput.

Cells are plated in 96 well plates in complete medium at 70% confluence. Each assay, which can accommodate 8 different cell lines, includes cells of kidney (for example, as described above) and non-kidney origin. After plating, the cells are allowed to rest undisturbed for 6 hours to allow attachment, and then each well is rinsed twice with 200 µl of serum free medium and then re-fed with 200 µl of serum-free medium that contains a candidate agent of interest. Each cell type is exposed to three different concentrations of the compound of interest and to control solution without the candidate agent. Each treatment is performed in triplicate. Thus, as configured here, each assay includes triplicate measurements of 8 cell lines under four different experimental conditions for a total of 96 samples. After 48 hours of incubation, the cells are lysed in situ in 50 µl of a solution compatible with the luciferase assay (Karas et al., 1994) and luciferase activity is determined spectrophotometrically (either manually by removal of the cell lysate from the tissue culture plate, or automatically in a computerised plate reader). Agents that decrease the luciferase activity over control conditions in the kidney cells are identified as modulating agents.

The general assay described above is amenable to variation in a number of ways. As noted above, the assay can employ transiently transfected cells or stably transfected cells. The use of transiently transfected cells is particularly well suited to rapid testing of new cell lines to determine their suitability for further study. In some cell types, the level of activation of the megalin reporter construct may be low, and this may be augmented by co-transfection (again in either a transient or a stable fashion) of a cubilin (or an isoform or mutant thereof) and/or additional megalin expression construct. In addition, the level of expression of the megalin receptor expression construct can be controlled by use of a regulatable promoter such as the tetracycline-responsive system that has been described. Other means of regulating the megalin receptor expression construct are also compatible with this screening assay.

The foregoing descriptions of particular embodiments and examples are offered by way of illustration and not by way of limitation. All publications and patent applications cited in this specification and all references cited therein are herein incorporated by reference as if each individual publication or patent application or reference were specifically and individually indicated to be incorporated by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

### Literature reference list

[1] Apocada, G.: Endocytic traffic in polarized epithelial cells: role of the actin and microtubule cytoskeleton. Traffic 2; 149-159 (2001).
[2] Brown, D.: Targeting of membrane transporters in renal epithelia: when cell biology meets physiology. Am J Physiol Renal Physiol 278; F192-F201 (2000).
[3] Mukherjee, S., Ghosh R.N., Maxfield F.R.: Endocytosis. Physiol Reviews 77 (3); 759-803 (1997).
[4] Nielsen, S.: Endocytosis in renal proximal tubules. Dan Med Bull 41 (3); 243-263 (1994).
[5] Gekle, M., Mildenberger, S., Freudinger, R. et al: Albumin endocytosis in OK cells: dependence on actin and microtubules and regulation by protein kinases. Am J Physiol 272 (Renal Physiol, 41); F668-F677 (1997).
[6] Adibi, S.A.: Renal assimilation of oligopeptides: physiological mechanisms and metabolic importance. Am J Physiol 272 (Endocrinol Metab 35); E723-736 (1997).
[7] Inui, K.I., Masuda, S., Saito, H.: Cellular and molecular aspects of drug transport in the kidney. Kid Intern 58; 944-958 (2000).
[8] Behr, T.M., Goldenberg, D.M., Becker, W.: Reducing the renal uptake of raidolabeled antibody fragments and peptides for diognosis and therapy: present status, future prospects and limitations. Eur J Nucl Med 25; 201-212 (1998).
[9] Tsai, S.W., Wiiliams, L.E., Anderson, A.L. et al.: Metabolism and renal clearance of ¹¹¹In-labeled DOTA conjugated antibody fragments. Bioconjugate Chem 12; 264-270 (2001).
[10] Kobayahsi, H., Yoo, T.M., Kim, I.S. et al.: L-Lysine effectively blocks renal uptake of ¹²⁵I- or ⁹⁹Tc-labeled anti-Tac disulfide-stabilized Fv fragment. Cancer Res 56; 3788-3795 (1996).
[11] De Palatis, L.R., Frazeir, K.A., Cheng, R.C. et al.: Lysine reduces renal accumulation of radioactivity associated with injection of [¹⁷⁷Lu]α-[2-(4-aminophenyl)ethyl]-1,4,7,10-tetraaza-cyclodecane-1,4,7,10-tetraacetic acid-CC49 Fab radioimmuno conjugate. Cancer Res 55; 5288-5295 (1995).
[12] Behr, T.M., Becker, W., Sharkey, R.M. et al.: Reduction of the renal uptake of monoclonal antibody fragments by amino acid infusion. J Nucl Med 37; 829-833 (1996).
[13]Bernard, B.F., Krenning, E.P., Wout, A.P. et al.: L-lysine reduction of Indium-111 octreotide and Yttrium-90 octrotide renal uptake. J Nucl Med 38; 1929-1933 (1997).
[14] Barone, R., De Champs, J., Smith, C. et al.: Metabolic effects of amino acids solutions infused for renal protection. J Nucl Med 41; 94P (2000).
[15] Weber, R.W., Boutin, R.H., Nedelman, M.A. et al.: Enhanced kidney clearance with an ester-linekd 99mTc-radiolabeled antibody Fab'-chelator conjugate. Bioconjugate Chem 1; 431-437 (1990).
[16] Lewis, M.R., Shively J.E.: Maleimidocysteineamido-DOTA derivatives: new reagents for radiometal chelate conjugation to antibody sulfhydryl groups undergo pH-dependent cleavage reactions. Bioconjugate Chem 9; 72-86 (1998).
[17] Novak-Hofer, I., Zimmermann, K., Schubiger, P.A.: Peptide linkers lead to modification of liver metabolism and improved tumor targeting of copper-67-labeled antibody fragments. Cancer Biother Radiopharm 16 (6); 469-481 (2001).
[18] Arano, Y., Fujioka, Y., Akizawa, H. et al.: Chemical design of radiolabeled antibody fragments for low renal radioactivity levels. Cancer Res 59; 128-134 (1999).
[19]Christensen, E.I., Birn, H.: megalin and cubilin: multifunctional endocytic receptors. Nature Rev 3; 258-268 (2002).
[20] Bim, H., Vorum, H., Verroust P.J. et al.: Receptor associated protein is important for normal processing of megalin in the kidney proximal tubules. J Am Soc Nephol 11; 191-202 (2000).
[21] Willnow, T.E., Armstrong, S.A., Hammer, R.E., Herz, J.: Functional expression of low density lipoprotein receptor-related proteine is controlled by receptor-associated protein in vivo. Proc Natl Acad Sci 92; 4537-4541 (1995).
[22] Leheste, J.R., Rolinski, B., Vorum, H. et al.: megalin knockout mice as an animal model of low molecular weight proteinuria. Am J Pathol 155 (4); 1361-1370 (1999).
[23] Förster GJ, Engelbach M, Brockmann J, Reber H et al.: Preliminary data on biodistribution and dosimetry for therapy planning of somatostatin receptor positive tumors: comparison of ⁸⁶Y-DOTATOC and ¹¹¹In-DTPA-octreotide. Eur J Nucl Med 28 (12); 1743-1750,2001.
[24] Kwekkeboom DJ, Bakker W, Kooij PPM, Konijenberg MW et al.: [¹⁷⁷Lu-DOTA⁰,Tyr³]octreotate: comparison with [111In-DTPA⁰]octreotide in patients. Eur J Nucl Med 28 (9); 1319-1325, 2001.
[25] Paganelli G, Zoboli S, Cremonesi M, Bodei L et al.: Receptor mediated radiotherapy with ⁹⁰Y-DOTA-D-Phe¹-Tyr³-octreotide. Eur J Nucl Med 28 (4); 426-434, 2001.
[26] Wiseman GA, White CA, Sparks RB, Erwin WD et al.: Biodistribution and dosimetry results from a phase III prospectively randomized controlled trial of ZevalinTM radioimmunotherapy for low grade, follicular, or transformed B-cell non-Hodgkin's lymphoma. Crit Rev Oncol Hematol 39; 181-194, 2001.
[27] Behr TM, Liersch T, Greiner-Bechert L, Griesinger F et al.: Radioimmunotherapy of small-volume diseases of metastatic colorectal cancer. Cancer 94; 1373-1381, 2002.
[28] Taylor JE, Thevenian MA, Bashirzadek R, et al.: Detection of somatostatin receptor subtype 2 (SSTR2) in established tumours and tumour cell lines: Evidence for SSTR heterogeneity. Peptides 15(7); 1229-1236, 1994.
[29] Karas et al., Circulation 89:1943, 1994.
[30] Hammad SM, Barth JL, Knaak C, Argraves WS. megalin acts in concert with cubilin to mediate endocytosis of high density lipoproteins. J Biol Chem. 2000 Apr 21;275(16):12003-8.
[31] Prescott, ed., METHODS IN CELL BIOLOGY, Volume XIV, Academic Press, New York, N.Y. (1976), p. 33 et seq.
[32] Lenna C.A. and Hannink, M. 1996, Nucl. Acids Res. 24: 3341-3347.
[33] Koivunen E. et al. (1993) J. Biol. Chem. 268(27):20205.
[34] Zervos et al. (1993) Cell 72:223-232.
[35] Madura et al. (1993) J. Biol. Chem. 268:12046-12054.
[36] Bartel et al. (1993) Biotechniques 14:920-924.
[37] Iwabuchi et al. (1993) Oncogene 8:1693-1696.
[38] Vidal M, Legrain P, Nucleic Acids Res 1999 Feb 15;27(4):919-29.
[39] Maleka et al., Environ. Health Perspectives 102:572, 1994.
[40] Knight et al., Maturitas 22:167, 1995.
[41] Stern, Maturitas 4:33, 1982.
[42] Jayalilaka et al., J. Organic Chrom. 617:19, 1993.
[43] Hurby et al., "Application of Synthetic Peptides: Antisense Peptides," in Synthetic Peptides, A User's Guide, W. H. Freeman, NY, pp. 289-307 (1992).
[44] Kaspczak et al., Biochemistry 28:9230 (1989).
[45] Harlow, Antibodies, Cold Spring Harbor Press, NY (1990).
[46] Ashcom et al., J. Cell. Biol. 110:1041-1048 (1990).
[47] Jensen et al., FEBS Lett. 255:275-280 (1989).
[48] Williams et al., J. Biol. Chem. 267:9035-9040 (1992).
[49] Wurshawsky et al., J. Biol. Chem. 269:3325-3330 (1994).
[50] Lutz et al., Exp. Cell Res. 175:109-124 (1988).
[51] Kishimoto et al., Proc. Natl. Acad. Sci. USA 87:2244-2248 (1990).
[52] Pluckthum, Bio/Technology 10:163-167 (1992).
[53] Carter et al., Bio/Technology 10:167-170 (1992).
[54] Mullinax et al., Biotechniques 12:864-869 (1992).
[55] Perkins et al., Eur. J. Nucl. Med. 10:296-301 (1985).
[56] Carasquillo et al., J. Nucl. Med. 28:281-287 (1987).
[57] Esteban et al., J. Nucl. Med. 28:861-870 (1987).
[58] Kennedy et al. Clin. Chim. Acta 70:1-31 (1976).
[59] Schurs et al. Clin. Chim. Acta 81:1-40 (1977).
[60] Better, et al., Science 240:1041-1043(1988).
[61] Jones, et al., Nature 321:552-525 (1986).
[62] Verhoeyan et al., Science 239:1534 (1988).
[63] Beidler, et al., J. Immunol. 141:4053-4060 (1988).
[64] Davis et al., Nature 326:760-765 (1987).
[65] Yokade et al., J. Cell. Biol. 117:39 (1992).
[66] Szostak, TIBS 19:89, (1992).
[67] Mulligan, Science, 260, 926-932 (1993).
[68] Zhu et al., Science 261, 209-211 (1993).
[69] Zamecnik et al., Proc. Natl. Acad. Sci. USA 75, 280-284 (1978).
[70] Zamecnik et al., Proc. Natl. Acad. Sci., 83, 4143-4146 (1986).
[71] Wickstrom et al., Proc. Natl. Acad. Sci. USA 85, 1028-1032 (1988).
[72] Crooke, FASEB J. 7, 533-539 (1993).
[73] Offensperger et. al., EMBO J. 12, 1257-1262 (1993).
[74] Agrawal et al., Proc. Natl. Acad. Sci. USA 85, 7079-7083 (1988).
[75] Sarin et al., Proc. Natl. Acad. Sci. USA 85, 7448-7794 (1989).
[76] Shaw et al., Nucleic Acids Res 19, 747-750 (1991).
[77] Maher et al., Science 245, 725-730 (1989).
[78] Orson et al., Nucl. Acids Res. 19, 3435-3441 (1991).
[79] Postal et al., Proc. Natl. Acad. Sci. USA 88, 8227-8231 (1991).
[80] Cooney et al., Science 241, 456-459 (1988).
[81] Young et al., Proc. Natl. Acad. Sci. USA 88, 10023-10026 (1991).
[82] Duval-Valentin et al., Proc. Natl. Acad. Sci. USA 89, 504-508 (1992).
[83] Blume et al., Nucl. Acids Res. 20, 1777-1784 (1992).
[84] Grigoriev et al., J. Biol. Chem. 267, 3389-3395 (1992).
[85] Holt et al., Mol. Cell. Biol. 8, 963-973 (1988).
[86] Wickstrom et al., Proc. Natl. Acad. Sci. USA 85, 1028-1032 (1988).
[87] Ikuta et al., in Ann. Rev. Biochem. 53, 323-356 (1984).
[88] Narang et al., in Methods Enzymol., 65, 610-620 (1980).
[89] J. Merrifield, J. Am. Chem. Soc. 85, 2149 (1964).
[90] W. D. Jacoby et al., Meth. Enzym. 34, Academic Press, N.Y. (1974).
[91] L. A. Sternberger et al., J. Histochem. Cytochem. 18:315 (1970).
[92] E. A. Bayer et al., Meth. Enzym. 62:308 (1979).
[93] E. Engval et al., Immunol. 109:129 (1972).
[94]J. W. Goding, J. Immunol. Meth. 13:215 (1976).
[95]U. Ruther and B. Muller-Hill, EMBO J. 2:1791 (1983).
[96]K. K. Stanley and J. P. Luzio. EMBO J. 3:1429 (1984).
[97]Shimatake and Rosenberg, Nature 292:128 (1981).
[98]Amann and Brosius, Gene 40:183 (1985).
[99]Studier and Moffat, J. Mol. Biol. 189:113 (1986).
[100]Stinchcomb et al. Nature 282:39 (1979).
[101]Kingsman et al., Gene 7:141 (1979).
[102]Tschumper et al., Gene 10:157 (1980).
[103]Bwach et al., Gene 8:121-133 (1979).
[104]Ammerer, Methods of Enzymology 101:192-210 (1983).
[105]Hitzeman et al., J. Biol. Chem. 255:2073 (1980).
[106]Kawaski and Fraenkel, BBRC 108:1107-1112 (1982).
[107]Charron et al., Proc. Natl. Acad. Sci. U.S.A. 86, 2535-2539 (1989).
[108]Hunt and Calderwood, Gene 87, 199-204 (1990).

## Claims

1. A method for producing a pharmaceutical for modulating the megalin-receptor mediated binding and/or uptake of a radiodiagnostic and/or radiotherapeutic or a pharmaceutically acceptable salt thereof into cells of the kidney in a patient in need thereof, comprising
an effective amount of a radiodiagnostic and/or radiotherapeutic or a pharmaceutically acceptable salt thereof, and
an effective amount of a modulator of the megalin-receptor mediated uptake of said radiodiagnostic and/or radiotherapeutic into cells of the kidney, or a pharmaceutical salt thereof; a prodrug thereof; or a metabolite thereof.

2. A method according to claim 1 wherein said modulator can be administered before, simultaneously together with and/or after said radiodiagnostic and/or radiotherapeutic.

3. A method according to claim 1 or 2 wherein said modulator is a modulator of the expression of the megalin receptor in kidney cells, in particular an inhibitor of the megalin-receptor mediated uptake of said radiodiagnostic and/or radiotherapeutic into cells of the kidney.

4. A method according to any of claims 1 to 3 wherein said modulator is administered as an injection (intraperitoneally and/or intravenously), an infusion, a bolus dose and/or as an oral dosage.

5. A method according to any of claims 1 to 4 wherein said modulator is a compound selected from the group of compounds comprising modified cubilin, vitamin-binding proteins, carrier proteins, lipoproteins, apolipoproteins (such as apo E, and apo B, clusterin), LPO-antagonists (such as probucol), RAP and derivatives thereof, Ca²⁺, Ca²⁺-scavengers, lipids, cytochrome C, antimegalin IgG, anti-cubilin IgG, anti-poly alpha2,8 KDN antibody, aminoglycosides (such as gentamicin), hormones and precursors thereof, drugs and toxins, enzymes and inhibitors thereof, and immune- and stress-response related proteins, maleate, dextran-based conjugates, aristolochic acid, cadmium chloride, and L-carnithine.

6. A method according to any of claims 1 to 5 wherein said modulator is further **characterised by** at least one of the following properties,
- an affinity to megalin and/or cubilin of 50-500 nM, preferably of less than 50 nM,
- the ability to pass through the filtration barrier of the glomerulus,
- a reduction of the uptake of said radiodiagnostic and/or radiotherapeutic into cells of the kidney by more than about 60%, and
- no significant (p < 0.05) reduction of the uptake (%ID/g) of said radiodiagnostic and/or radiotherapeutic into tumour or other diseased cells.

7. A method according to any of claims 1 to 6 wherein the modulator is added during the prevention, diagnosis, monitoring and/or treatment of cancer, such as prostate cancer, breast cancer, leukaemia, pancreatic cancer, lung cancer, colon cancer, sarcoma, glioblastoma, melanoma, ovarian cancer, head and neck carcinomas, and lymphoma; and other diseases, such as Parkinson disease, Alzheimer syndrome, coronary diseases, and restenosis, or during the prevention of damages of the kidney of a patient during a radiotherapy and/or radiodiagnosis.

8. A method according to any of claims 1 to 7 wherein said radiodiagnostic and/or radiotherapeutic is selected from the group of compounds comprising radiolabeled antibodies, radiolabeled enzymes, radiolabeled peptides, and radiolabeled nucleic acids, comprising radiometals selected from the group of Fluorine-18, Chromium-51, Cobalt-58, Gallium-67, Copper-67, Molybdenum-99, Technetium-99, Indium-111, Iodine-123, Iodine-125, Iodine-131, Yttrium-90, Gold Au-198 colloid, Xenon-133, Thallium-201, Rhenium-188, Strontium-189, Actinium-225, Astatine-211, Bismuth-212/213, Rhenium-186, Holmium-166, Samarium-153, and Lutetium-177.

9. A method according to any of claims 1 to 8 wherein said modulator is administered together with a cofactor selected from the group of compounds comprising modified cubilin, vitamin-binding proteins, carrier proteins, lipoproteins, apolipoproteins (such as apo E, and apo B, clusterin), LPO-antagonists (such as probucol), RAP and derivatives thereof, Ca²⁺, Ca²⁺-scavengers, lipids, cytochrome C, antimegalin IgG, anti-cubilin IgG, anti-poly alpha2,8 KDN antibody, aminoglycosides (such as gentamicin), hormones and precursors thereof, drugs and toxins, enzymes and inhibitors thereof, and immune- and stress-response related proteins, maleate, dextran-based conjugates, aristolochic acid, cadmium chloride, and L-camithine.

10. A method of identifying a modulator compound of the megalin-receptor mediated binding and/or uptake of radiotherapeutics and/or radiodiagnostics into cells of the kidney, comprising:
providing an assay for measuring the megalin-receptor mediated binding and/or uptake of radiotherapeutics and/or radiodiagnostics into cells or tissues of the kidney,
adding at least one radiotherapeutic and/or radiodiagnostic and/or a suitably labelled precursor thereof to said assay;
adding at least one candidate modulator compound to be tested to the assay; and
determining if the amount of radiotherapeutics and/or radiodiagnostics bound to or taken up by the megalin-receptor-mediated binding and/or uptake is altered as compared to binding or uptake in the absence of the at least one candidate modulator compound to be tested,
wherein a difference in binding or uptake mediated by the megalin-receptor identifies a compound which is a modulator of the megalin-receptor mediated binding and/or uptake of radiotherapeutics and/or radiodiagnostics into cells of the kidney.

11. A method according to claim 10 wherein said at least one candidate modulator compound is added before, simultaneously together with and/or after said radiotherapeutic and/or radiodiagnostic.

12. A method according to claim 10 or 11, wherein said determining if the amount of the radiotherapeutic and/or radiodiagnostics bound to or taken up by the megalin-receptor-mediated binding and/or uptake is altered as compared to binding or uptake in the absence of the candidate compound to be tested comprises measuring the expression of the megalin receptor and, optionally of cubilin, in cells of the kidney.

13. A method according to any of claims 10 to 12, wherein said candidate compound is added together with a cofactor selected from the group of compounds comprising modified cubilin, vitamin-binding proteins, carrier proteins, lipoproteins, apolipoproteins (such as apo E, and apo B, clusterin), LPO-antagonists (such as probucol), RAP and derivatives thereof, Ca²⁺, Ca²⁺-scavengers, lipids, cytochrome C, antimegalin IgG, anti-cubilin IgG, anti-poly alpha2,8 KDN antibody, aminoglycosides (such as gentamicin), hormones and precursors thereof, drugs and toxins, enzymes and inhibitors thereof, and immune- and stress-response related proteins, maleate, dextran-based conjugates, aristolochic acid, cadmium chloride, and L-carnithine.

14. The method according to any of claims 10 to 13, wherein the assay is performed in vitro or in vivo.

15. The method according to any of claims 10 to 14, wherein the assay is selected from the group comprising a two-hybrid assay, a biochemical pull-down assay, a high-throughput screening, in vitro fluorescent polarisation assay, an in vitro fluorescent binding assay, a conformational sensor-solid-phase chemiluminescence assay, a surface plasmon resonance assay, a filter binding assay, a sepharose chromatography, and a scintillation proximity assay.

16. The method according to any of claims 10 to 15, wherein the assay is a an in vitro, cell-free mixture comprising a pre-determined amount of the megalin receptor and the cofactor.

17. The method of claim 14 wherein the assay is performed in a mammalian cell culture, said cell culture preferably comprising kidney cells, selected from the group of LLC-PK1 (ATCC CL-101); LLC-RK1 (ATCC CCl-106); A6 (ATCC CCL-102); OK (ATCC CRL-1840); MDCK (ATCC CCL-34); HK-2 (ATCC CRL-2190); HaK (ATCC CCL-15); and LLC-MK2 (ATCC CCL-7).

18. The method of claim 17 wherein said mammalian cell is transformed with a plasmid containing a DNA sequence encoding the megalin-receptor and, optionally, with a plasmid containing a DNA sequence encoding cubilin.

19. The method according to claims 17 or 18, wherein the assay includes a cell expressing the megalin-receptor protein and the compound is a nucleic acid sequence which potentially alters expression of the megalin-receptor protein.

20. The method according to any of claims 10 to 19, wherein said radiotherapeutic is selected from the group of compounds comprising radiolabeled antibodies, radiolabeled peptides, radiolabeled enzymes, radiolabeled nucleic acids, comprising radiometals selected from the group of Fluorine-18, Chromium-51, Cobalt-58, Gallium-67, Copper-67, Molybdenum-99, Technetium-99, Indium-111, Iodine-123, Iodine-125, Iodine-131, Yttrium-90, Gold Au-198 colloid, Xenon-133, Thallium-201, Rhenium-188, Strontium-189, Actinium-225, Astatine-211, Bismuth-212/213, Rhenium-186, Holmium-166, Samarium-153, and Lutetium-177.

21. The method according to any of claims 10 to 20,wherein the candidate compound is selected from a library of naturally occurring or synthetic compounds which are randomly tested for alteration of binding.

22. The method according to any of claims 10 to 21, wherein said candidate compound is selected from the group of megalin-binding compounds comprising modified cubilin, vitamin-binding proteins, carrier proteins, lipoproteins, apolipoproteins, like apo E and apo B, clusterin, LPO-antagonists, like probucol, RAP and derivatives thereof, Ca²⁺, Ca²⁺- scavengers, lipids, cytochrome C, antimegalin IgG, anti-cubilin IgG, anti-poly alpha2,8 KDN antibody, aminoglycosides, like gentamicin, hormones and precursors thereof, drugs and toxins, enzymes and inhibitors thereof, and immune- and stress-response related proteins, maleate, dextran-based conjugates, aristolochic acid, cadmium chloride, and L-carnithine.

23. Modulator, preferably inhibitor, of the of the megalin-receptor mediated binding and/or uptake of radiotherapeutics into cells of the kidney, identified according to a method according to any of claims 10 to 22.

24. Modulator of the megalin-receptor mediated binding and/or uptake of radiotherapeutics into cells of the kidney according to claim 23, wherein said modulator is further **characterised by** at least one of the following properties,
- an affinity to megalin and/or cubilin of 50-500 nM, preferably of less than 50 nM,
- the ability to pass through the filtration barrier of the glomerulus,
- a reduction of the uptake of said radiodiagnostic and/or radiotherapeutic into cells of the kidney by more than about 60%, and
- no significant (p < 0.05) reduction of the uptake (%ID/g) of said radiodiagnostic and/or radiotherapeutic into tumour or other diseased cells.

25. Modulator of the megalin-receptor mediated binding and/or uptake of a radiodiagnostic and/or radiotherapeutic into cells of the kidney according to claim 23 or 24, wherein the compound competitively and/or sterically inhibits binding of said radiodiagnostic and/or radiotherapeutic to the megalin-receptor protein.

26. Modulator of the megalin-receptor mediated binding and/or uptake of radiotherapeutics into cells of the kidney according to any of claims 23 to 25, wherein said compound is selected from the group of megalin-binding compounds comprising modified cubilin, vitamin-binding proteins, carrier proteins, lipoproteins, apolipoproteins (such as apo E, and apo B, clusterin), LPO-antagonists (such as probucol), RAP and derivatives thereof, Ca²⁺, Ca²⁺-scavengers, lipids, cytochrome C, antimegalin IgG, anti-cubilin IgG, anti-poly alpha2,8 KDN antibody, aminoglycosides (such as gentamicin), hormones and precursors thereof, drugs and toxins, enzymes and inhibitors thereof, and immune- and stress-response related proteins, maleate, dextran-based conjugates, aristolochic acid, cadmium chloride, and L-carnithine.

27. A method for producing a pharmaceutical composition for the treatment of cancer, comprising
identifying a modulator compound of the megalin-receptor mediated binding and/or uptake of radiotherapeutics into cells of the kidney according to any of claims 10 to 22, and
mixing of said modulator compound thus identified with a pharmaceutically suitable carrier and/or diluent.

28. A pharmaceutical composition, produced according to claim 27.

29. A pharmaceutical composition according to claim 28, further comprising a an effective amount of a chemotherapeutic agent, preferably selected from the group consisting of a DNA-interactive agent, alkylating agent, antimetabolite, tubulin-interactive agent, and a hormonal agent, Methotrexate, Fluorouracil, Fluorodeoxyuridin, CB3717, Azacitidine, Floxuridine, Mercapyopurine, 6-Thioguanine, Pentostatin, Cytarabine, and Fludarabine.

30. Use of a modulator of the of the megalin-receptor mediated binding and/or uptake of radiotherapeutics and/or radiodiagnostics into cells of the kidney according to any of claims 23 to 26 for the prevention, diagnosis, monitoring and/or treatment of cancer.
